# EUROPÄISCHE PATENTSCHRIFT

(11) **EP 2 370 101 B1**
(45) Veröffentlichungstag und Bekanntmachung des Hinweises auf die Patenterteilung: **27.03.2013**
(21) Anmeldenummer: 09796949.7
(22) Anmeldetag: 09.12.2009
(51) Int. Cl.: A61K 39/39, C07K 14/475

(54) **VERWENDUNG VON FLT3-LIGAND ZUR VERSTÄRKUNG VON IMMUNREAKTIONEN BEI RNA-IMMUNISIERUNG**
USE OF FLT3 LIGAND FOR STRENGTHENING IMMUNE RESPONSES IN RNA IMMUNIZATION
UTILISATION D'UN LIGAND DE FLT3 POUR LE RENFORCEMENT DE RÉACTIONS IMMUNITAIRES LORS DE L'IMMUNISATION ARN

(30) Priorität: 10.12.2008 DE 102008061522
(43) Veröffentlichungstag der Anmeldung: 05.10.2011
(73) Patentinhaber: BioNTech AG, 55131 Mainz (DE); Johannes Gutenberg-Universität Mainz, 55122 Mainz (DE)
(72) Erfinder: SAHIN, Ugur, 55116 Mainz (DE); TÜRECI, Özlem, 55116 Mainz (DE); KREITER, Sebastian, 55131 Mainz (DE); SELMI, Abderraouf, 55118 Mainz (DE)
(74) Vertreter: Schnappauf, Georg
(86) Internationale Anmeldenummer: PCT/EP2009/008811
(87) Internationale Veröffentlichungsnummer: WO 2010/066418

(56) Entgegenhaltungen:
- WO-A2-02/061113
- WO-A2-2005/052119
- US-B1- 7 005 131
- XU JUAN ET AL: "Recombinant DNA vaccine of the early secreted antigen ESAT-6 by Mycobacterium tuberculosis and Flt3 ligand enhanced the cell-mediated immunity in mice" VACCINE, Bd. 26, Nr. 35, August 2008 (2008-08), Seiten 4519-4525, XP002571844 ISSN: 0264-410X
- CARRALOT J-P ET AL: "Polarization of immunity induced by direct injection of naked sequence-stabilized mRNA vaccines" CMLS CELLULAR AND MOLECULAR LIFE SCIENCES, BIRKHAUSER VERLAG, HEIDELBERG, DE, Bd. 61, Nr. 18, 1. September 2004 (2004-09-01), Seiten 2418-2424, XP002355208 ISSN: 1420-682X
- CHAVAN R ET AL: "Expression of CCL20 and granulocyte-macrophage colony-stimulating factor, but not Flt3-L, from modified vaccinia virus Ankara enhances antiviral cellular and humoral immune responses" JOURNAL OF VIROLOGY, Bd. 80, Nr. 15, August 2006 (2006-08), Seiten 7676-7687, XP002571845 ISSN: 0022-538X

## Beschreibung

Die Erfindung betrifft das Gebiet der Vakzinierung und Immunstimulierung durch die Verwendung von RNA, insbesondere mRNA, die für ein oder mehrere Antigene, die beispielsweise mit Infektionserkrankungen oder malignen Erkrankungen wie Krebs in Beziehung stehen, kodiert.

Das Immunsystem kann sowohl spezifische, als auch nicht-spezifische Immunität aufweisen. Im Allgemeinen wird spezifische Immunität durch B- und T-Lymphozyten erzeugt, die auf ihrer Zelloberfläche spezifische Rezeptoren für ein bestimmtes Antigen aufweisen. Das Immunsystem kann auf unterschiedliche Antigene in zweierlei Weise reagieren: (i) Humorale Immunität, die B-Zell-Stimulierung und Produktion von Antikörpern oder Immunglobulinen einbezieht, und (ii) Zell-vermittelte Immunität, die im Allgemeinen T-Zellen, einschließlich cytotoxischer T-Lymphozyten (CTL), einbezieht.

Antigen-spezifische T-Zell-Reaktionen werden durch antigene Peptide, die an die Bindungsgrube von Glykoproteinen des Haupt-Histokompatibilitätskomplexes (MHC) gebunden sind, als Teil des Mechanismus des Immunsystems hervorgerufen, bei dem fremde Antigene identifiziert und eine Reaktion gegen sie ausgelöst wird. Die gebundenen antigenen Peptide interagieren mit T-Zell-Rezeptoren und modulieren so eine Immunreaktion. Die antigenen Peptide sind nicht-kovalent an bestimmte "Bindetaschen" gebunden, die von polymorphen Resten der Bindungsgrube des MHC-Proteins gebildet werden.

MHC Klasse II-Moleküle sind heterodimere Glykoproteine, die aus α- und β-Ketten bestehen. Die α1- und β1-Domänen dieser Moleküle falten sich zusammen und bilden eine Peptid-Bindungsgrube. Antigene Peptide binden an das MHC-Molekül durch Interaktion zwischen Anker-Aminosäuren auf dem Peptid und den α1- und β1-Domänen. MHC Klasse 1-Moleküle besitzen andere Domänen-Organisationen als MHC Klasse II-Moleküle, jedoch im Allgemeinen eine ähnliche Struktur mit einer Peptid-Bindestelle oder -grube, die zu den Membrandomänen entfernt liegt.

Der anfängliche Schritt bei der Präsentation eines fremden Protein-Antigens ist die Bindung des nativen Antigens an eine Antigen-präsentierende Zelle (APC). Nach Bindung an APCs dringen Antigene in die Zellen ein, entweder durch Phagozytose, Rezeptor-vermittelte Endozytose oder Pinozytose. Derartige internalisierte Antigene sind in intrazellulären Membran-gebundenen Vesikeln, die Endosome genannt werden, lokalisiert. Nach einer Endosomen-Lysosomen-Fusion werden die Antigene in kleine Peptide durch in den Lysosomen gelegene zelluläre Proteasen prozessiert. Die Peptide assoziieren mit den α- und β-Ketten von MHC Klasse II-Molekülen innerhalb dieser Lysosomen. Diese MHC Klasse II-Moleküle, die zuvor im rauen endoplasmatischen Retikulum synthetisiert worden waren, werden sequentiell an die Golgi-Komplexe und sodann an das lysosomale Kompartiment transportiert. Der Peptid-MHC-Komplex wird auf der Oberfläche von APCs für eine T- und B-Zell-Aktivierung präsentiert.

Nicht-spezifische Immunität umfasst verschiedene Zellen und Mechanismen wie Phagozytose durch Makrophagen oder Granulozyten und Aktivität von natürlichen Killerzellen (NK). Nicht-spezifische Immunität beruht auf Mechanismen, die evolutionär weniger fortgeschritten sind, und weist nicht die Eigenschaften hinsichtlich Spezifität und Erinnerungsvermögen, die wichtige Merkmale einer spezifischen Immunreaktion sind, auf.

Rekombinante Impfstoffe haben in der Human- und Veterinärmedizin eine besondere Bedeutung als Wirkstoffe und Arzneimittel für die Prophylaxe und Therapie von Infektions-und Krebserkrankungen. Ziel einer Impfung mit einem rekombinanten Impfstoff ist gegen ein definiertes Antigen eine spezifische Immunreaktion zu induzieren, die präventiv oder therapeutisch gegen definierte Krankheiten wirksam ist.

Nachdem gezeigt werden konnte, dass die direkte intramuskuläre Injektion von Plasmid-DNA zur langanhaltenden Expression der kodierten Gene auf der Zelloberfläche führt (Wolff, J.A. et al. (1990) Science 247:1465-1468), erschienen DNA-basierte Impfstoffe als neue, vielversprechende Immunisierungsstrategie. Diese Beobachtungen waren ein bedeutender Ansporn um Nukleinsäuren-basierte Impfstoffe zu entwickeln. Zunächst wurden DNA-basierte Impfstoffe gegen infektiöse Pathogene erprobt (Cox, G.J. et al. (1993) J. Virol. 67:5664-5667, Davis, H.L. et al. (1993) Hum. Mol. Genet. 2:1847-1851, Ulmer, J.B. (1993) Science 259:1745-1749, Wang, B. et al. (1993) Proc. Natl. Acad. Sci. U.S.A. 90:4156-4160), bald aber auch in der Gentherapie gegen Tumoren weiter erforscht, um eine spezifische Antitumor-Immunität zu induzieren (Conry, R. M. et al. (1994) Cancer Res. 54:1164-1168, Conry, R.M. et al. (1995) Gene Ther. 2:59-65, Spooner, R.A. et al. (1995) Gene Ther. 2:173-180, Wang, B. et al. (1995) Hum. Gene Ther. 6:407-418). Diese Strategie der Tumorimmunisierung hat eine Reihe von entscheidenden Vorteilen. Nukleinsäure-basierte Impfstoffe sind einfach in der Herstellung und relativ kostengünstig. Sie können außerdem aus einer geringen Zellzahl amplifiziert werden.

DNA ist stabiler als RNA birgt aber einige potentielle Sicherheitsrisiken wie die Induktion von anti-DNA-Antikörpern (Gilkeson, G.S. et al. (1995) J. Clin. Invest. 95:1398-1402) und die Integration des Transgens ins Wirtsgenom. Das kann zur Inaktivierung zellulärer Gene, einer unkontrollierbaren Langzeitexpression des Transgens oder Onkogenese führen und ist daher für Tumor-assoziierte Antigene mit onkogenem Potential wie z.B. erb-B2 (Bargmann, C.I. et al. (1986) Nature 319:226-230) und p53 (Greenblatt, M.S. et al. (1994) Cancer Res. 54:4855-4878) generell nicht anwendbar. Um diese potentiellen Gefahren zu umgehen, bietet der Gebrauch von RNA eine attraktive Alternative.

Zu den Vorteilen der RNA als eine Art reversible Gentherapie zählt die vorübergehende Expression und der nicht transformierende Charakter. Die RNA muss nicht in den Kern gelangen, um transgen exprimiert zu werden und kann außerdem nicht ins Wirtsgenom integriert werden, wodurch das Risiko der Onkogenese eliminiert ist. Wie mit DNA (Condon, C. et al. (1996) Nat. Med. 2:1122-1128, Tang, D.C. et al. (1992) Nature 356:152-154) können auch durch die Injektion von RNA sowohl die zelluläre als auch die humorale Immunantwort in vivo induziert werden (Hoerr, I. et al. (2000) Eur. J. Immunol. 30:1-7, Ying, H. et al. (1999) Nat. Med. 5:823-827).

Für die Immuntherapie mit in vitro transkribierter (IVT-RNA) oder in vitro amplifizierter RNA werden zwei unterschiedliche Strategien verfolgt, die beide in verschiedenen Tiermodellen erfolgreich getestet werden konnten und im Menschen erste Anwendung fanden.

Entweder werden Dendritische Zellen (DCs) durch Lipofektion oder Elektroporation mit der in vitro transkribierten RNA transfiziert und dann appliziert (Heiser, A. (2000) J. Immunol. 164:5508-5514) oder die RNA wird über unterschiedliche Immunisierungsrouten direkt injiziert (Hoerr, I. et al. (2000) Eur. J. Immunol. 30:1-7, Granstein, R.D. et al. (2000) Journal of Investigative Dermatology 114:632-636, Conry, R.M. (1995) Cancer Research 55:1397-1400). Es konnte gezeigt werden, dass die Immunisierung mit RNA-transfizierten DCs antigenspezifische CTLs in vitro und in vivo induziert (Su, Z. (2003) Cancer Res. 63:2127-2133, Heiser, A. et al. (2002) J. Clin. Invest. 109:409-417). Erste klinische Daten zur Nutzung von RNA-transfizierten Dendritischen Zellen als Tumorvakzine stammen aus den Jahren 2001 und 2002 und konnten zeigen, dass Antigen-spezifische T-Zellen in Tumorpatienten induziert werden können (Heiser, A. et al. (2002) J. Clin. Invest. 109:409-417, Rains, N. (2001) Hepato-Gastroenterology 48:347-351). Für die direkte intradermale Injektion von RNA in Patienten liegen mittlerweile erste Daten einer klinischen Phase I/II Studie bei Melanompatienten vor (Weide, B. (2008) Journal of Immunotherapy 31:180-188). Hier wurde die Sicherheit und geringe Toxizität der Injektion von nackter RNA gezeigt. Auf präklinischen Daten basierend, welche eine verbesserte TH-1 Immunität nach GM-CSF Gabe gezeigt hatten, wurde GM-CSF als Adjuvanz benutzt (Carralot, J.P. et al. (2004) Cell Mol. Life Sci. 61:2418-2424). Es konnten allerdings keine klinischen Effekte bei den behandelten Malanompatienten beobachtet werden.

RNA-Vakzinen können daher verwendet werden, um transient Zellen mit RNA zu transfizieren, die für Proteinantigene kodieren, deren Expression eine Immunreaktion stimuliert. Aufgrund der intrazellulären Produktion dieser Antigene und ihrer Prozessierung durch den endogenen Weg lösen RNA-Vakzinen humorale Immunität, sowie T-Zell-Immunität unter Produktion von cytotoxischen T-Lymphozyten (CTL) aus.

Aufgrund der vorstehend beschriebenen Eigenschaften scheint RNA besonders gut für klinische Anwendungen geeignet zu sein. Die Nutzung von RNA wird aber vor allem durch die kurze Halbwertszeit der RNA im Zytoplasma stark eingeschränkt, da das Molekül durch Enzyme rasch abgebaut wird, was eine geringe Proteinexpression zur Folge hat. Daher ist es von großem Interesse, die immunogene Potenz von RNA als Wirkstoff zu amplifizieren.

Adjuvantien werden seit langem zur Potenzierung der Wirkung von Impfungen genutzt (Aguilar, J.C. et al. (2007) Vaccine 25:3752-3762, Chiarella, P. et al. (2007) Expert Opinion on Biological Therapy 7:1551-1562). Verschiedenste Agentien wie CpG, Poly I:C, GM-CSF, Flt3-Ligand oder Monophosphoryl Lipid A wurden schon in präklinischen und frühen klinischen Untersuchungen hinsichtlich ihrer Potenz im Rahmen von Tumorvakzinationsstrategien untersucht (Speiser, D.E. et al. (2005) Journal of Clinical Investigation 115:739-746, Cui, Z.R. et al. (2006) Cancer Immunology Immunotherapy 55:1267-1279, Jaffee, E.M. (2001) Journal of Clinical Oncology 19:145-156, Shackleton M. et al. (2004) Cancer Immunity 4:9-20, Neidhart, J. et al. (2004) Vaccine 22:773-780). Zur Verstärkung von Immunreaktionen nach Vakzination mit RNA-transfizierten Dendritischen Zellen wurden in präklinischen Studien verschiedene Adjuvantien (IL-12, CD40-L, OX40-L, 4-1BBL) kotransfiziert (Dannull, J. et al. (2005) Blood 105:3206-3213, Bontkes, H.J. et al. (2007) Gene Therapy 14:366-375, Grunebach, F. (2005) Cancer Gene Therapy 12:749-756). Alternativ wurde auch doppelsträngige RNA (Poly I:C) mit der Antigen-kodierenden RNA kotransfiziert (Michiels, A. (2006) Gene Therapy 13:1027-1036).

Im Rahmen von Untersuchungen zur Nutzung von Adjuvantien im Kontext der Vakzination mit nackter IVT-RNA wurde bisher nur die s.c. Gabe von GM-CSF getestet, welche in präklinischen Untersuchungen zu einer leicht verstärkten Induktion der TH-1 Immunität führte (Carralot, J.P. (2004) Cell Mol. Life Sci. 61:2418-2424). Die Erfordernisse an Adjuvantien zur Verwendung im Rahmen der direkten Applikation nackter RNA unterscheiden sich fundamental von denen für Adjuvantien, welche im Rahmen von Peptid-, DNA- oder Zell-basierten Vakzinen verwendet werden. Dies erklärt sich aus dem für die Aufnahme von RNA aus dem Extrazellulärraum in Zellen verantwortlichen Mechanismus.

Es besteht daher ein Bedarf für Mittel, die den Grad einer Immunstimulierung bei einer Verabreichung von RNA-Vakzinen verstärken.

Diese Aufgabe wird erfindungsgemäß durch den Gegenstand der Patentansprüche gelöst.

Die Erfindung geht auf diese Bedürfnisse dadurch ein, dass Verbindungen beschrieben werden, die eine Aufnahme von RNA in das Cytosol von Antigen-präsentierenden Zellen unterstützen und/oder eine wirksamere Immunreaktion bei einer Verabreichung einer Vakzine-RNA erzeugen können.

Die Erfinder haben festgestellt, dass eine Verabreichung von RNA-Molekülen, die für Antigene kodieren, die für eine Vakzinierung und Therapie einsetzbar sind, in Verbindung mit einer Verabreichung von Flt3-Ligand (Flt3-L) wirksam zu einer Immunreaktion führen kann, die für diese Antigene spezifisch ist.

Erfindungsgemäß konnte festgestellt werden, dass verschiedene bekannte Adjuvantien nicht nur zu keiner Steigerung der T-Zell-Primingeffizienz nach direkter Immunisierung mit nackter IVT-RNA führen, sondern tendenziell die T-Zell-Antwort vermindern. Dieser Befund war überraschend und ist nur durch den Einfluß der Adjuvantien auf die RNA-Aufnahme in Antigen-präsentierenden Zellen zu erklären. Es handelt sich dabei um einen Mechanismus, der in einer von den Erfindern erstmals beschriebenen Weise für die Aufnahme von langkettigen Ribonukleinsäuren verantwortlich ist. Dieser Aufnahmemechanismus wird durch verschiedene Adjuvantien in seiner Effizienz inhibiert. Einzig Flt3-Ligand konnte einen signifikanten Adjuvanzeffekt bei RNA-Immmunisierung zeigen. Die hier dargestellten Untersuchungen zeigen insbesondere, dass bei Verabreichung von Flt3-Ligand zusammen mit für ein Antigen kodierender RNA eine starke Zunahme von Antigen-spezifischen CD8⁺ T-Zellen festgestellt werden konnte.

Die Erfindung betrifft allgemein ein Zuführen von Vakzine-RNA an Zellen. Insbesondere betrifft die Erfindung eine gemeinsame Verwendung von Vakzine-RNA und Flt3-Ligand zur Induktion, Erzeugung oder Verstärkung einer Immunreaktion bei einer Verabreichung an Tiere (einschließlich des Menschen).

Flt3-Ligand verstärkt erfindungsgemäß vorzugsweise bei einer Verwendung mit einer RNA-Vakzine eine Immunreaktion eines Tiers gegen spezifische Antigene, die durch die Verwendung der RNA-Vakzine erzeugt werden. Typische Vakzinen, die in diesem Ansatz verwendet werden, sind virale Vakzinen wie Influenza-, Herpes-, Cytomegalovirus-, HIV-1-, HTLV-1- und FIV-Vakzinen, bakterielle Vakzinen, Krebsvakzinen und Parasitenvakzinen.

Vorzugsweise wird ein Tier erfindungsgemäß immunisiert, indem FIt3-Ligand und RNA, die für ein Antigen kodiert, in ein Tier eingebracht wird. Die RNA wird in Antigen-präsentierende Zellen des Tiers (Monozyten, Makrophagen, Dendritische Zellen oder andere Zellen) aufgenommen. Ein antigenisches Translationsprodukt der RNA wird gebildet und das Produkt wird gegebenenfalls prozessiert und durch die Zellen in dem Kontext von Haupthistokompatibilitätskomplexen präsentiert, wodurch eine Immunreaktion gegen das Antigen erzeugt wird. Die RNA erzeugt somit bei einer Translation das Antigen.

In bestimmten Ausführungsformen wird der FIt3-Ligand vor, gleichzeitig mit und/oder nach einer Verabreichung einer RNA-Vakzine verabreicht. Bevorzugt wird der FIt3-Ligand vor einer Verabreichung einer RNA-Vakzine verabreicht.

In einem Aspekt betrifft die Erfindung ein immunogenes Präparat, das RNA, die für mindestens ein Antigen kodiert, und FIt3-Ligand umfasst. Die RNA und der FIt3-Ligand können in dem erfindungsgemäßen immunogenen Präparat in einer gemeinsamen Zusammensetzung, d.h. im Gemisch, vorliegen. Ferner sind erfindungsgemäß auch Ausführungsformen vorgesehen, bei denen die RNA und der FIt3-Ligand gemeinsam, jedoch nicht in derselben Zusammensetzung vorliegen. Solche Ausführungsformen betreffen insbesondere Kits mit mindestens zwei Behältnissen, wobei ein Behältnis eine Zusammensetzung enthält, die die RNA umfasst, und ein anderes Behältnis eine Zusammensetzung enthält, die den FIt3-Ligand umfasst.

In dem erfindungsgemäßen immunogenen Präparat ist die RNA vorzugsweise mRNA. Die RNA wird vorzugsweise durch in vitro-Transkription erhalten.

Das erfindungsgemäße immunogene Präparat kann ferner mindestens einen RNA-stabilisierenden Faktor wie einen RNase-Inhibitor für eine Stabilisierung der RNA umfassen.

Das erfindungsgemäße immunogene Präparat ist vorzugsweise ein Präparat, das für eine therapeutische Anwendung formuliert ist. Erfindungsgemäß umfasst der Begriff "therapeutische Anwendung" eine Behandlung oder Vermeidung einer Erkrankung. In diesem Aspekt betrifft die Erfindung eine pharmazeutische Zusammensetzung, die ein erfindungsgemäßes immunogenes Präparat umfasst.

Typischerweise kann das erfindungsgemäße immunogene Präparat bzw. die erfindungsgemäße pharmazeutische Zusammensetung weiterhin ein Lösungsmittel wie ein wässriges Lösungsmittel oder ein jegliches Lösungsmittel, das es ermöglicht, die Integrität der RNA aufrechtzuerhalten, ein Adjuvanz wie Aluminiumhydroxid, Freundsches Adjuvanz, Oligonukleotide mit einem CpG-Motiv oder ein jegliches anderes Adjuvanz, das dem Fachmann bekannt ist, und ein jegliches Stabilisierungsmittel wie Protamin umfassen. Eine erfindungsgemäße pharmazeutische Zusammensetzung umfasst vorzugsweise ein pharmazeutisch verträgliches Verdünnungsmittel und/oder einen pharmazeutisch verträglichen Träger.

Es ist ferner möglich, die Immunogenizität der erfindungsgemäßen Präparate durch Zugabe eines oder mehrerer weiterer Adjuvanzien zu steigern. Es ist ferner möglich, die RNA der erfindungsgemäßen immunogenen Zubereitung durch Komplexierung mit kationischen Verbindungen, vorzugsweise polykationischen Verbindungen wie beispielsweise einem kationischen oder polykationischen Peptid oder Protein zu stabilisieren. Gemäß einer bevorzugten Ausführungsform der erfindungsgemäßen immunogenen Zubereitung ist das RNA-komplexierende Peptid oder Protein ein Protamin, ein Poly-L-Lysin, ein Poly-L-Arginin oder ein Histon.

Eine erfindungsgemäße pharmazeutische Zusammensetzung liegt vorzugsweise in einer Form vor, die es für eine Vakzinierung eines Lebewesens geeignet macht.

Vorzugsweise liegt ein erfindungsgemäßes immunogenes Präparat oder eine erfindungsgemäße pharmazeutische Zusammensetung oder zumindest die RNA umfassende Komponente davon in Form einer Formulierung für eine intranodale Verabreichung vor.

Die vorstehend beschriebenen Präparate und Zusammensetzungen sind in den hierin beschriebenen Verfahren, insbesondere Immunisierungsverfahren, einsetzbar.

In einem weiteren Aspekt betrifft die Erfindung Fl+3-Ligand zur Verwendung in einem Verfahren zum Erzeugen oder Verstärken einer Immunreaktion bei einem Individuum, das eine Verabreichung von RNA, die für ein Antigen kodiert, gegen das die Immunreaktion gerichtet sein soll, und eine Verabreichung von FIt3-Ligand umfasst. Die Immunreaktion hat vorzugsweise eine protektive und/oder therapeutische Wirkung auf das Individuum und umfasst vorzugsweise eine Antigen-spezifische T-Zell-Immunreaktion.

In einem weiteren Aspekt betrifft die Erfindung Fl+3-Ligand zur Verwendung in einem Verfahren zur Erhöhung der Menge von Antigen-spezifischen Effektorzellen, insbesondere CD8⁺ cytotoxischen T-Zellen und/oder CD4⁺ Helfer-T-Zellen in einem Individuum, das eine Verabreichung von RNA, die für das Antigen kodiert, und eine Verabreichung von Flt3-Ligand umfasst.

Ein weiterer Aspekt betrifft eine Vermeidung und/oder Behandlung von Krebs unter Verwendung eines Immunisierungsprotokolls, das eine Verwendung von Flt3-Ligand einbezieht. In diesem Aspekt betrifft die Erfindung insbesondere Fl+3-Ligand zur Verwendung in einem Verfahren zur Vermeidung und/oder Behandlung von Krebs in einem Individuum, das eine Verabreichung von RNA, die für ein Tumorantigen kodiert, gegen das die Immunreaktion gerichtet sein soll, und eine Verabreichung von Flt3-Ligand umfasst.

Ein weiterer Aspekt betrifft eine Vermeidung und/oder Behandlung viraler Infektionen unter Verwendung eines Immunisierungsprotokolls, das eine Verwendung von Flt3-Ligand einbezieht. In diesem Aspekt betrifft die Erfindung insbesondere Flt3-Ligand zur Verwendung in einem Verfahren zur Vermeidung und/oder Behandlung einer viralen Infektion in einem Individuum, das eine Verabreichung von RNA, die für ein virales Antigen kodiert, gegen das die Immunreaktion gerichtet sein soll, und eine Verabreichung von Flt3-Ligand umfasst.

Ein weiterer Aspekt betrifft eine Vermeidung und/oder Behandlung von bakteriellen Infektionen unter Verwendung eines Immunisierungsprotokolls, das eine Verwendung von Flt3-Ligand einbezieht. In diesem Aspekt betrifft die Erfindung insbesondere Flt3-Ligand zur Verwendung in einem Verfahren zur Vermeidung und/oder Behandlung einer bakteriellen Infektion in einem Individuum, das eine Verabreichung von RNA, die für ein bakterielles Antigen kodiert, gegen das die Immunreaktion gerichtet sein soll, und eine Verabreichung von Flt3-Ligand umfasst.

Ein erfindungsgemäβes Immuniserrungsprotokoll, das eine Verwendung von Flt3-Ligand einbezieht, ist und für eine Vermeidung und/oder Behandlung einer Infektion durch einzellige Organismen geeignet. Dabei wird Flt3-Ligand in einen Verfahren zur Vermeidung und/oder Behandlung einer Infektion durch einen einzelligen Organismus in einem Individuum verwendet das eine Verabreichung von RNA, die für ein Antigen des einzelligen Organismus kodiert, gegen das die Immunreaktion gerichtet sein soll, und eine Verabreichung von Flt3-Ligand umfasst.

Ein weiterer Aspekt betrifft eine Vermeidung und/oder Behandlung von Allergie bei einem Patienten, die eine Verabreichung von Flt3-Ligand zusammen mit einer Allergen-spezifischen Immuntherapie einbezieht. In diesem Aspekt betrifft die Erfindung insbesondere Flt3-Ligand zur Verwendung in einem Verfahren zur Vermeidung und/oder Behandlung einer Allergie in einem Individuum, das eine Verabreichung von RNA, die für ein für die Allergie relevantes Allergen kodiert, und eine Verabreichung von Flt3-Ligand umfasst.

Vorteile einer Behandlung und/oder Vermeidung von Erkrankungen oder Infektionen unter Verwendung der hierin beschriebenen Strategie sind unter anderem, dass die Immunogenizität von schwach immunogenen Antigenen wie rekombinanten Antigenen gesteigert werden kann, die Menge an verwendetem Antigen bzw. dafür kodierender RNA verringert werden kann, das Erfordernis für Booster-Immunisierungen verringert wird, und die Effizienz einer Immunisierung gesteigert wird.

Die Verwendung von Flt3-Ligand mit RNA-Vakzinen kann die Immunogenizität bestimmter viraler Proteine und Krebs-spezifischer Antigene, die normalerweise eine schwache Immunreaktion auslösen, verstärken. Die Vakzinierungstechnik ist beispielsweise auf die Induktion einer Immunreaktion gegen schwach immunogene virale Proteine anwendbar. Bei den erfindungsgemäßen RNA-Vakzinen ist das Proteinantigen niemals gegenüber Serum-Antikörpern ausgesetzt, sondern wird durch transfizierte Zellen selbst nach Translation der mRNA erzeugt. Somit sollte Anaphylaxis kein Problem darstellen. Die Erfindung ermöglicht daher die wiederholte Immunisierung eines Patienten ohne Gefahr für allergische Reaktionen.

Die erfindungsgemäße Immunisierungsstrategie ermöglicht auch die quantitative Steigerung der Frequenz Antigen-spezifischer T-Lymphozyten nach RNA basierter Immunisierung. Diese Effizienzsteigerung kann für die Immuntherapie von Patienten im Sinne einer besseren klinischen Wirksamkeit oder im Sinne der Reduktion der Vakzinedosis respektive Applikationshäufigkeit bei gleicher Wirksamkeit nutzbar gemacht werden.

In HLA-transgenen Mäusen können durch eine erfindungsgemäße Immunisierung mit einer RNA-Vakzine, die für humane Tumor-assozierte Antigene kodiert, T-Zell-Klone oder T-Zell-Rezeptoren isoliert werden, welche natürlich prozessierte Epitope im Kontext eines humanen HLA-Moleküls erkennen. Durch die erfindungsgemäße Immuniserungsstrategie können mit einer höheren Wahrscheinlichkeit Antigen-spezifische T-Zellen generiert werden. Weiterhin ist durch die erfindungsgemäße Immunisierungsstrategie die Möglichkeit gegeben, auch Antigen-spezifische T-Zellen, welche in einer niedrigen Vorläuferfrequenz vorliegen, stark zu amplifizieren. Diese Effizienzsteigerung ermöglicht die umfassendere Isolation der im naiven Repertoire vorhandenen Antigen-spezifischen T-Zellen. Des Weiteren ist die Erhöhung der Effizienz durch das beschriebene Immunisierungsverfahren mit einer Kostenreduktion verbunden.

Es ist auch vorgesehen, Zellen aus einem Tier zu entfernen und die Zellen in vitro mit Flt3-Ligand/RNA zu transfizieren. Die RNA wird in die Zellen eingebaut und ein antigenisches Translationsprodukt des Polynukleotids wird ausgebildet. Nach Transfektion werden die Zellen, die das Antigen exprimieren, in das Tier vorzugsweise durch Injektion eingebracht, wo das Immunsystem nun auf das nunmehr endogene Antigen reagieren kann und eine Immunreaktion gegen das Immunogen ausgelöst wird. In dieser Ausführungsform sind die zu transfizierenden Zellen vorzugsweise lymphoide Zellen, insbesondere Antigen-präsentierende Zellen, die von dem Tier entnommen wurden.

Falls Zellen aus einem Tier in vitro transfiziert werden sollen, kann die Quelle von Zellen periphere Blutzellen sein, die rasch aus Gesamtblut isoliert werden können, um eine Quelle für Zellen bereitzustellen, die sowohl Klasse I- als auch Klasse II-MHC-Moleküle enthalten. Diese Zellen können ferner in B-Zellen, Helfer-T-Zellen, cytotoxische T-Zellen oder Makrophagen/Monozyten fraktioniert werden. Knochenmarkszellen können eine Quelle weniger differenzierter lymphoider Zellen bereitstellen.

T-Zellen, insbesondere CD4⁺- und CD8⁺-Lymphozyten, können in vitro oder in einem Lebewesen in einem Verfahren stimuliert oder aktiviert werden, das die Bereitstellung für die T-Zellen bzw. Verabreichung an das Lebewesen von RNA, die für mindestens ein Antigen kodiert, für das die T-Zellen spezifisch sein sollen, und Flt3-Ligand umfasst. Eine derartige Stimulierung oder Aktivierung äußert sich vorzugsweise in einer Expansion, cytotoxischen Reaktivität und/oder Zytokinausschüttung der T-Zellen.

Die vorstehend beschriebenen Ansiehe eignen sich insbesondere für eine Behandlung oder Prophylaxe von infektiösen Erkrankungen, die beispielsweise von Bakterien oder Viren verursacht werden. In bestimmten Ausführungsformen ist das erfindungsgemäß verwendete Antigen von einem infektiösen Erreger wie Hepatitis A, B, C, HIV, Mykobakterien, Malariaerreger, Erreger von SARS, Herpesvirus, Influenzavirus, Poliovirus bzw. von bakteriellen Erregern wie Chlamydien und Mykobakterien abgeleitet. Eine besonders nützliche Anwendung der vorliegenden Erfindung liegt in der Krebs-Immuntherapie oder - Vakzinierung, wo insbesondere eine Aktivierung von Tumorantigen-reaktiven T-Zellen verstärkt wird, wodurch die Aussicht für eine T-Zell-Immuntherapie oder -Vakzinierung gegen Tumorzellen verbessert wird.

In spezifischen Ausführungsformen ist das erfindungsgemäß verwendete Antigen ausgewählt aus der Gruppe bestehend aus den folgenden Antigenen: p53 ART-4, BAGE, ss-Catenin/m, Bcr-abL CAMEL, CAP-1, CASP-8, CDC27/m, CDK4/m, CEA, CLAUDIN-6, CLAUDIN-12, c-MYC, CT, Cyp-B, DAM, ELF2M, ETV6-AML1, G250, GAGE, GnT-V, Gap100, HAGE, HER-2/neu, HPV-E7, HPV-E6, HAST-2, hTERT (oder hTRT), LAGE, LDLR/FUT, MAGE-A, vorzugsweise MAGE-A1, MAGE-A2, MAGE-A3, MAGE-A4, MAGE-A5, MAGE-A6, MAGE-A7, MAGE-A8, MAGE-A9, MAGE-A10, MAGE-A11 oder MAGE-A12, MAGE-B, MAGE-C, MART- 1/Melan-A, MC1R, Myosin/m, MUC1, MUM-1, -2, -3, NA88-A, NF1, NY-ESO-1, NY-BR-1, p190 minor bcr-abL Pml/RARa, PRAME, Proteinase-3, PSA, PSM, RAGE, RU1 oder RU2, SAGE, SART-1 oder SART-3, SCGB3A2, SCP1, SCP2, SCP3, SSX, SURVIVIN, TEL/AML1, TPI/m, TRP-1, TRP-2, TRP-2/INT2, TPTE und WT, vorzugsweise WT-1.

### Detaillierte Beschreibung der Erfindung

Erfindungsgemäß können Standardverfahren für eine Herstellung von rekombinanten Nukleinsäuren, Kultivierung von Zellen und Einbringen von Nukleinsäuren in Zellen verwendet werden. Enzymatische Reaktionen erfolgen gemäß Herstellerangaben oder in an sich bekannter Weise.

Der Begriff "Flt3-Ligand" oder "Flt3-L" betrifft "Fms-like tyrosine kinase 3 ligand". Flt3 ist eine Rezeptor-Tyrosin-Kinase (RTK), die durch unreife hämatopoetische Vorläuferzellen exprimiert wird. Der Ligand für Flt3 (Flt3-L) ist ein Transmembranprotein oder lösliches Protein und wird durch eine Vielzahl von Zellen, einschließlich hämatopoetischer Zellen und Stromazellen im Knochenmark exprimiert. In Kombination mit anderen Wachstumsfaktoren stimuliert der Ligand Proliferation und Entwicklung von verschiedenen Zelltypen, einschließlich Stammzellen, myeloider und lymphoider Vorläuferzellen, Dendritischer Zellen und NK-Zellen. Aktivierung des Rezeptors führt zu einer Tyrosin-Phosphorylierung von verschiedenen Schlüssel-Adaptor-Proteinen, die bekanntlich an verschiedenen Signaltransduktionswegen, die Proliferation, Überleben und andere Prozesse in hämatopoetischen Zellen kontrollieren, beteiligt sind.

Der Begriff "Flt3-Ligand" umfasst jegliche Moleküle, insbesondere Peptide und Proteine, die an Flt3-Rezeptor binden und vorzugsweise die biologische Aktivität aufweisen, ein stimulatorisches Signal an die Zelle über den gebundenen Flt3-Rezeptor zu transduzieren.

Der Begriff "Flt3-Ligand" umfasst alle Varianten, insbesondere Splicevarianten und posttranslationell modifizierte Varianten, Konformationen, Isoformen und Species-Homologe von Flt3-Ligand, die natürlicherweise von Zellen exprimiert werden oder die von Zellen exprimiert werden, die mit einer Nukleinsäure, die für Flt3-Ligand kodiert, transfiziert wurden. Des Weiteren umfasst der Begriff "Flt3-Ligand" alle rekombinant hergestellten und herstellbaren Formen von Flt3-Ligand.

Der Begriff "Nukleinsäure, die für Flt3-Ligand kodiert" betrifft vorzugsweise eine Nukleinsäure, die eine Nukleinsäuresequenz umfasst, die aus der Gruppe ausgewählt ist, bestehend aus (i) SEQ ID NOs: 3 und 4 des Sequenzprotokolls, (ii) einer von der Nukleinsäuresequenz gemäß (i) abgeleiteten Sequenz, und (iii) einem Teil der Nukleinsäuresequenz gemäß (i) oder (ii).

Flt3-Ligand umfasst in einer bevorzugten Ausführungsform eine Aminosäuresequenz, die von einer Nukleinsäure kodiert wird, die eine Nukleinsäuresequenz umfasst, die aus der Gruppe ausgewählt ist, bestehend aus (i) SEQ ID NOs: 3 und 4 des Sequenzprotokolls, (ii) einer von der Nukleinsäuresequenz gemäß (i) abgeleiteten Sequenz, und (iii) einem Teil der Nukleinsäuresequenz gemäß (i) oder (ii). In einer weiteren bevorzugten Ausführungsform umfasst Flt3-Ligand eine Aminosäuresequenz, die aus der Gruppe ausgewählt ist, bestehend aus SEQ ID NOs: 1 und 2 des Sequenzprotokolls, einer davon abgeleiteten Sequenz, oder einem Teil davon.

Formen an Flt3-Ligand, die erfindungsgemäß verwendet werden können, umfassen in nicht begrenzender Weise Flt3-Ligand aus Maus und Mensch wie gezeigt in SEQ ID NOs: 1 und 2 des Sequenzprotokolls und Polypeptide mit davon abgeleiteten Sequenzen.

Vorzugsweise betrifft der Begriff "davon abgeleitete Sequenz" in Bezug auf SEQ ID NOs: 1 und 2 Sequenzen, die bezüglich SEQ ID NOs: 1 und 2 verkürzt sind und hauptsächlich den extrazellulären Anteil der Proteine umfassen. Solche Sequenzen umfassen vorzugsweise nicht den Transmembrananteil und intrazellulären Anteil. Der Begriff "Flt3-Ligand" umfasst Polypeptide wie sie in der US-PS 5,554,512 und in der US-PS 6,291,661 beschrieben sind, die hierin durch Bezugnahme eingeschlossen sind.

Besonders bevorzugte Formen von Flt3-Ligand sind biologisch aktive, lösliche Formen und insbesondere diejenigen Formen, die die extrazelluläre Domäne oder ein oder mehrere Fragmente der extrazellulären Domäne umfassen. Solche Formen umfassen vorzugsweise nicht den Transmembrananteil und intrazellulären, d.h. cytoplasmatischen, Anteil von Flt3-Ligand. Lösliche Formen von Flt3-Ligand sind Polypeptide, die aus den Zellen, in denen sie exprimiert werden, sekretiert werden können. In solchen Formen wird die intrazelluläre Domäne und die Transmembran-Domäne des Polypeptids oder ein Teil davon deletiert, so dass das Polypeptid vollständig aus der Zelle, in der es exprimiert wird, sekretiert wird. Die intrazelluläre Domäne und Transmembran-Domäne der Polypeptide können erfindungsgemäß in an sich bekannter Weise durch Verfahren bestimmt werden, die für eine Bestimmung solcher Domänen anhand von Sequenzinformation bekannt sind. Bezüglich SEQ ID NO: 1 kann die intrazelluläre Domäne als die Aminosäuren 206-235 und die Transmembran-Domäne als die Aminosäuren 185-205 bzw. 183-205 definiert werden.

Humaner Flt3-Ligand kann eine Aminosäuresequenz umfassen, die aus der Gruppe ausgewählt ist, bestehend aus Aminosäuren 1-X, 27-X oder 28-X von SEQ ID NO: 1 oder einer davon abgeleiteten Sequenz, worin X eine Aminosäure von 160-235, vorzugsweise 160, 161, 162, 163, 164, 165, 166, 167, 168, 169, 170, 171, 172, 173, 174, 175, 176, 177, 178, 179, 180, 181, 182, 183, 184, oder 185 darstellt.

Muriner Flt3-Ligand kann eine Aminosäuresequenz umfassen, die aus der Gruppe ausgewählt ist, bestehend aus Aminosäuren 1-Y, 27-Y oder 28-Y von SEQ ID NO: 2 oder einer davon abgeleiteten Sequenz, worin Y eine Aminosäure von 163-232 darstellt.

Ausführungsformen von löslichem humanen Flt3-Ligand umfassen die Aminosäuresequenz der Reste 1-160 von SEQ ID NO: 1 (einschließlich), 27-160 von SEQ ID NO: 1 (einschließlich), 28-160 von SEQ ID NO: 1 (einschließlich), 1-179 von SEQ ID NO: 1 (einschließlich), 27-179 von SEQ ID NO: 1 (einschließlich), 28-179 von SEQ ID NO: 1 (einschließlich), 1-182 von SEQ ID NO: 1 (einschließlich), 27-182 von SEQ ID NO: 1 (einschließlich), 28-182 von SEQ ID NO: 1 (einschließlich), 1-185 von SEQ ID NO: 1 (einschließlich), 27-185 von SEQ ID NO: 1 (einschließlich), 28-185 von SEQ ID NO: 1 (einschließlich), 1-235 von SEQ ID NO: 1 (einschließlich), 27-235 von SEQ ID NO: 1 (einschließlich) und 28-235 von SEQ ID NO: 1 (einschließlich).

Ausführungsformen von löslichem murinen Flt3-Ligand umfassen die Aminosäuresequenz der Reste 1-163 von SEQ ID NO: 2 (einschließlich), die Aminosäuresequenz der Reste 28-163 von SEQ ID NO: 2 (einschließlich), die Aminosäuresequenz der Reste 1-188 von SEQ ID NO: 2 (einschließlich), die Aminosäuresequenz der Reste 28-188 von SEQ ID NO: 2 (einschließlich), die Aminosäuresequenz der Reste 1-232 von SEQ ID NO: 2 (einschließlich) und die Aminosäuresequenz der Reste 28-232 von SEQ ID NO: 2 (einschließlich).

Der Begriff "Flt3-Ligand" umfasst erfindungsgemäß auch Moleküle, die die vorstehend genannten Sequenzen in Verbindung, vorzugsweise in Form einer kovalenten Fusion, mit einem oder mehreren heterologen Peptiden oder Proteinen, gegebenenfalls getrennt durch einen Linker, umfassen. In diesem Zusammenhang ist ein Peptid oder Protein zu einer Sequenz, mit der es in Verbindung vorliegt, heterolog, falls das Peptid oder Protein nicht natürlicherweise in Verbindung mit der Sequenz vorliegt. Beispielsweise sind Sequenzen, die von einem natürlichen Flt3-Ligand abgeleitet sind, und Sequenzen, die von Antikörpern abgeleitet sind, heterologe Sequenzen. Diese heterologen Peptide oder Proteine können beispielsweise eine Sekretion der vorstehend genannten Sequenzen aus einer Wirtszelle steuern, eine Kompartimentalisierung der vorstehend genannten Sequenzen in bestimmte Organellen einer Zelle herbeiführen, die Stabilität der vorstehend genannten Sequenzen erhöhen und/oder eine Aufreinigung ermöglichen oder erleichtern. In einer Ausführungsform ist das heterologe Peptid oder Protein von einem Antikörper, vorzugsweise der schweren Kette eines Antikörpers, insbesondere einem Antikörper der Klasse IgG1, IgG2, vorzugsweise IgG2a, IgG2b, IgG3, IgG4, IgM, IgA, vorzugsweise IgA1, IgA2, sekretorischer IgA, IgD oder IgE abgeleitet. Vorzugsweise ist das heterologe Peptid oder Protein von der konstanten Region eines Antikörpers abgeleitet und umfasst vorzugsweise diese Region oder einen Teil davon. Vorzugsweise umfasst das heterologe Peptid oder Protein die in SEQ ID NO: 5 dargestellte Sequenz oder eine davon abgeleitete Sequenz. In einer Ausführungsform umfasst der Flt3-Ligand erfindungsgemäß die in SEQ ID NO: 6 dargestellte Sequenz oder eine davon abgeleitete Sequenz.

Weiterhin sind von dem Begriff "Flt3-Ligand" erfindungsgemäß alle Polypeptide umfasst, die eine Aminosäuresequenz umfassen, die von den hierin spezifisch beschriebenen Sequenzen abgeleitet ist.

Der Begriff "Immunreaktion" wird hierin in seiner herkömmlichen Bedeutung verwendet und umfasst humorale und zelluläre Immunität. Eine Immunreaktion manifestiert sich dadurch, dass eine oder mehrere Reaktionen stattfinden, die ausgewählt sind aus Entwicklung von Antikörpern gegen ein Antigen und Expansion von Antigen-spezifischen T-Lymphozyten, vorzugsweise CD4⁺ T-Lymphozyten und CD8⁺ T-Lymphozyten, mehr bevorzugt CD8⁺ T-Lymphozyten, die in verschiedenen Proliferations- oder Cytokin-Produktionstests in vitro nachgewiesen werden können.

Der Begriff "Immuntherapie" betrifft eine Behandlung auf der Basis einer Aktivierung einer spezifischen Immunreaktion.

Begriffe wie "schützen", "prophylaktisch" oder "protektiv" bedeuten hierin Verhindern oder Behandeln oder beides des Auftretens und/oder des Vermehrens eines Tumors oder Pathogens in einem Lebewesen. Eine prophylaktische Verabreichung einer Vakzine kann den Empfänger vor einer Entstehung von Tumorwachstum oder vor einer Infektion durch ein Pathogen schützen. Eine therapeutische Verabreichung einer Vakzine oder Immuntherapie kann den Empfänger beispielsweise vor einer Verbreitung oder Metastasierung bestehender Tumoren schützen oder die Reduzierung der Tumormasse von bestehenden Tumoren bewirken.

Antigen-präsentierende Zellen oder APC wie hierin verwendet sind Zellen, die Peptidfragmente von Proteinantigenen in Assoziierung mit MHC-Molekülen auf ihrer Zelloberfläche aufweisen. Manche APC können Antigen-spezifische T-Zellen aktivieren. Beispiele von APC umfassen in nicht begrenzender Weise Dendritische Zellen, Makrophagen, Monozyten, B-Zellen und dergleichen.

Der Begriff "MHC/Peptid-Komplex" betrifft einen nicht-kovalenten Komplex der Bindedomäne eines MHC-Klasse I- bzw. MHC-Klasse II-Moleküls und eines MHC-Klasse I-bzw. MHC-Klasse II-Bindepeptids.

Der Begriff "MHC-Bindepeptid" oder "bindendes Peptid" betrifft ein Peptid, das an ein MHC-Klasse I- und/oder ein MHC-Klasse II-Molekül bindet. Im Fall von Klasse I-MHC/Peptid-Komplexen sind die Bindepeptide typischerweise 8-10 Aminosäuren lang, obwohl längere oder kürzere Peptide wirksam sein können. Im Fall von Klasse II-MHC/Peptid-Komplexen sind die Bindepeptide typischerweise 10-25 Aminosäuren lang und insbesondere 13-18 Aminosäuren lang, obwohl längere und kürzere Peptide wirksam sein können.

Der Begriff "Haupt-Histokompatibilitätskomplex" und die Abkürzung "MHC" betreffen einen Komplex von Genen, der in allen Vertebraten auftritt. MHC-Proteine oder -Moleküle fungieren bei einer Signalgebung zwischen Lymphozyten und Antigen-präsentierenden Zellen in normalen Immunreaktionen dadurch, dass sie Peptide binden und sie für eine mögliche Erkennung durch T-Zell-Rezeptoren (TCR) präsentieren. MHC-Moleküle binden Peptide in einem intrazellulären Prozessierungskompartiment und präsentieren diese Peptide auf der Oberfläche von Antigen-präsentierenden Zellen gegenüber T-Zellen. Die menschliche MHC-Region, auch als HLA bezeichnet, findet sich auf Chromosom 6 und beinhaltet die Klasse I-Region und die Klasse II-Region.

Der Begriff "MHC-Klasse I" oder "Klasse I" betrifft die Haupt-Histokompatibilitätskomplex-Klasse I-Proteine oder -Gene. Innerhalb der MHC-Klasse I-Region finden sich beim Menschen die HLA-A-, HLA-B-, HLA-C-, HLA-E-, HLA-F-, CD1a-, CD1b- und CD1c-Unterregionen.

Die α-Ketten der Klasse I sind Glykoproteine mit einem Molekulargewicht von etwa 44 kDa. Die Polypeptidkette ist etwas über 350 Aminosäurereste lang. Sie kann in drei funktionelle Regionen unterteilt werden: eine externe, eine transmembranöse und eine cytoplasmatische Region. Die externe Region ist 283 Aminosäurereste lang und in drei Domänen, α1, α2 und α3, unterteilt. Die Domänen und Regionen werden gewöhnlich von separaten Exons des Klasse I-Gens kodiert. Die transmembrane Region umspannt die Lipid-Doppelschicht der Plasmamembran. Sie besteht aus 23 zumeist hydrophoben Aminosäureresten, die in einer α-Helix angeordnet sind. Die cytoplasmatische Region, d.h. der dem Cytoplasma zugewandte Teil, der sich an die Transmembranregion anschließt, ist typischerweise 32 Aminosäurereste lang und hat die Fähigkeit, mit den Elementen des Cytoskeletts zu interagieren. Die α-Kette interagiert mit β2-Mikroglobulin und bildet so α-β2-Dimere auf der Zelloberfläche.

Der Begriff "MHC-Klasse II" oder "Klasse II" betrifft die Haupt-Histokompatibilitätskomplex-Klasse II-Proteine oder -Gene. Innerhalb der MHC-Klasse II-Region finden sich im Menschen die DP-, DQ- und DR-Subregionen für Klasse II-α-Ketten-und -β-Ketten-Gene (d.h. DPα, DPβ, DQα, DQβ, DRα und DRβ).

Klasse II-Moleküle sind Heterodimere, die aus je einer α- und einer β-Kette bestehen. Beide Ketten sind Glykoproteine mit einem Molekulargewicht von 31-34 kDa (α) oder 26-29 kDa (β). Die Gesamtlänge der α-Ketten variiert von 229 bis 233 Aminosäureresten, die der β-Ketten von 225 bis 238 Resten. Sowohl α- als auch β-Ketten bestehen aus einer externen Region, einem verbindenden Peptid, einer transmembranösen Region und einem cytoplasmatischen Schwanz. Die externe Region besteht aus zwei Domänen, α1 und α2 oder β1 und β2. Das verbindende Peptid ist in α- und β-Ketten 13 bzw. 9 Reste lang. Es verbindet die zweite Domäne mit der transmembranösen Region, die sowohl in α- als auch in β-Ketten aus 23 Aminosäureresten besteht. Die Länge der cytoplasmatischen Region, d.h. der dem Cytoplasma zugewandte Teil, der sich an die Transmembranregion anschließt, variiert von 3 bis 16 Resten in α-Ketten und von 8 bis 20 Resten in β-Ketten.

Der Begriff, "MHC-Bindedomäne" betrifft die "MHC-Klasse I-Bindedomäne" und "MHC-Klasse II-Bindedomäne".

Der Begriff "MHC-Klasse I-Bindedomäne" betrifft die Region eines MHC-Klasse 1-Moleküls oder einer MHC-Klasse I-Kette, die für eine Bindung an ein antigenes Peptid notwendig ist. Eine MHC-Klasse I-Bindedomäne wird vorwiegend durch die α1- und α2-Domänen der MHC-Klasse I-α-Kette gebildet. Obwohl die α3-Domäne der α-Kette und β2-Mikroglobulin keine essentiellen Teile der Bindedomäne darstellen, sind sie vermutlich für eine Stabilisierung der Gesamtstruktur des MHC-Klasse I-Moleküls wichtig und daher schließt der Begriff "MHC-Klasse I-Bindedomäne" diese Regionen vorzugsweise ein. Eine MHC-Klasse I-Bindedomäne kann auch im Wesentlichen als die extrazelluläre Domäne eines MHC-Klasse I-Moleküls definiert werden, was sie von den Transmembran- und cytoplasmatischen Regionen unterscheidet.

Der Begriff "MHC-Klasse II-Bindedomäne" betrifft die Region eines MHC-Klasse II-Moleküls oder einer MHC-Klasse II-Kette, die für die Bindung an ein antigenes Peptid notwendig ist. Eine MHC-Klasse II-Bindedomäne wird vorwiegend durch die α1- und β1-Domänen der MHC-Klasse II-α- und -β-Ketten gebildet. Die α2- und β2-Domänen dieser Proteine sind vermutlich jedoch auch für eine Stabilisierung der Gesamtstruktur der MHC-Bindegrube wichtig und daher schließt der Begriff "MHC-Klasse II-Bindedomäne" erfindungsgemäß vorzugsweise diese Regionen ein. Eine MHC-Klasse II-Bindedomäne kann auch im Wesentlichen als die extrazelluläre Domäne eines MHC-Klasse II-Moleküls definiert werden, was sie von der Transmembran- und cytoplasmatischen Domäne unterscheidet.

Erfindungsgemäß umfasst der Begriff "Antigen" ein jegliches Molekül, das mindestens ein Epitop umfasst. Vorzugsweise ist ein Antigen erfindungsgemäß ein Molekül, das, gegebenenfalls nach Prozessierung, eine Immunreaktion auslösen kann, die vorzugsweise für das Antigen spezifisch ist. Erfindungsgemäß kann ein jegliches geeignetes Antigen, das ein Kandidat für eine Immunreaktion ist, wobei die Immunreaktion sowohl eine humorale als auch zelluläre Immunreaktion sein kann, verwendet werden. In den erfindungsgemäßen Ausführungsformen wird das Antigen oder eine prozessierte Form davon vorzugsweise durch eine Zelle in dem Zusammenhang mit MHC-Molekülen präsentiert werden, wodurch eine Immunreaktion gegen das Antigen oder die prozessierte Form davon ausgelöst wird.

Der Begriff "Antigen" umfasst insbesondere Proteine, Peptide, Nukleinsäuren, insbesondere RNA, sowie Nukleotide. Ein Antigen ist vorzugsweise ein Produkt, das von Allergenen, Viren, Bakterien, Pilzen, Parasiten und anderen infektiösen Agenzien und Pathogenen, oder Tumorantigenen abgeleitet ist. Ein Antigen kann erfindungsgemäß einem natürlich auftretenden Produkt, z.B. einem viralen Protein, entsprechen oder es kann davon abgeleitet sein, indem insbesondere die Abfolge und/oder Länge der Sequenz verändert wurde, weitere Sequenzen angefügt oder eingefügt wurden etc., insbesondere, um die Immunogenizität zu erhöhen. Das verwendete Antigen wird jedoch vorzugweise eine Immunreaktion erzeugen, die auch gegen das natürliche Produkt, aus dem es abgeleitet ist, gerichtet ist. Der Begriff "Antigen" umfasst erfindungsgemäß somit auch immunogene Teile oder Epitope von Gesamtproteinen oder Gesamtpeptiden, die in der Form von Proteinen, Peptiden, multimerischen Proteinen oder Peptiden, synthetischen Peptiden und dergleichen vorliegen können. Der Begriff "Immunogenizität" betrifft die relative Wirksamkeit eines Antigens, eine Immunreaktion zu erzeugen.

Der Begriff "Antigen" umfasst auch derivatisierte Antigene, d.h. erst durch Umwandlung (z.B. intermediär im Molekül, durch Komplettierung mit Körpereiweiss) antigen werdende - und sensibilisierende - Sekundärsubstanzen.

In einer bevorzugten Ausführungsform ist das Antigen ein Tumorantigen, d.h. ein Bestandteil von Krebszellen, die dem Cytoplasma, der Zelloberfläche und dem Zellkern entstammen können, insbesondere diejenigen, die intrazellulär oder als Oberflächenantigene an Tumorzellen vorzugsweise vermehrt entstehenden Antigene. Beispiele sind das karzinoembryonale Antigen, α1-Fetoprotein, Isoferritin und fetales Sulfoglykoprotein, α2-H-Ferroprotein und γ-Fetoprotein und verschiedene Virustumorantigene. In einer weiteren Ausführungsform ist das Antigen ein Virusantigen wie virale Ribonukleoproteine oder Hüllproteine. Insbesondere sollte das Antigen oder Peptide davon von MHC-Molekülen präsentiert werden und dadurch zur Modulation, insbesondere Aktivierung von Zellen des Immunsystems, vorzugsweise CD4⁺- und CD8⁺-Lymphozyten, insbesondere über die Modulation der Aktivität eines T-Zell-Rezeptors fähig sein und somit vorzugsweise die T-Zell-Vermehrung induzieren.

Erfindungsgemäß umfasst ein Tumorantigen vorzugsweise ein jegliches Antigen, das für Tumor oder Krebs bzw. Tumor- oder Krebszellen bezüglich Art und/oder Menge charakteristisch ist.

Flt3-Ligand kann auch im Zusammenhang mit einer Behandlung von Allergien verwendet werden. Die hierin beschriebenen Immunisierungsprotokolle unter Verwendung von Flt3-Ligand weisen eine Anwendbarkeit bei der Allergen-spezifischen Immuntherapie von Allergien auf. Allergen-spezifische Immuntherapie ist als die Verabreichung von vorzugsweise zunehmenden Dosen einer Allergen-Vakzine an ein Lebewesen mit einer oder mehreren Allergien definiert, um einen Zustand zu erreichen, bei dem die Symptome, die mit einer darauf folgenden Exposition gegenüber dem verursachenden Allergen assoziiert sind, gelindert werden. Die Wirksamkeit einer Allergen-spezifischen Immuntherapie unter Verwendung von Flt3-Ligand kann durch bekannte Standardverfahren bewertet werden wie durch Messung von Allergen-spezifischen IgG- und IgE-Antikörpern aus dem Patienten.

Immunogene sind Antigene, die eine Immunreaktion in einem Lebewesen induzieren.

Die erfindungsgemäß zu verwendenden Zusammensetzungen sind bezüglich der Art und Anzahl der Antigene, die durch die RNA-Moleküle kodiert werden, nicht begrenzt.

Erfindungsgemäß kann eine einzelne RNA-Spezies mit einer definierten Sequenz verabreicht werden, jedoch können auch mehrere unterschiedliche RNAs mit unterschiedlichen Sequenzen verabreicht werden. In einer Ausführungsform wird erfindungsgemäß ein Pool von RNA-Molekülen verabreicht. In dem Fall, dass die RNA erfindungsgemäß RNA-Moleküle mit unterschiedlichen Sequenzen umfasst, können die kodierenden Sequenzen dieser RNAs von dem gleichen oder verschiedenen Antigenen abgeleitet sein.

Der Begriff "Pathogen" betrifft pathogene Mikroorganismen und umfasst Viren, Bakterien, einzellige Organismen und Parasiten. Humanes Immundefizienzvirus (HIV), Cytomegalovirus (CMV), Herpesvirus (HSV), Hepatitis A-Virus (HAV), HBV, HCV, Papillomvirus und humanes T-lymphotrophisches Virus (HTLV) sind Beispiele für pathogene Viren. Einzellige Organismen umfassen Plasmodien, Trypanosomen, Amöben und dergleichen.

Der Begriff "Vakzine" wie hierin verwendet betrifft eine Zusammensetzung, die ein oder mehrere Antigene bzw. die dafür kodierende(n) Nukleinsäure(n) umfasst. Eine Vakzine kann weiterhin ein oder mehrere Adjuvanzien, Verdünnungsmittel, Trägerstoffe und dergleichen umfassen und wird an ein Lebewesen durch einen jeglichen geeigneten Weg verabreicht, um eine schützende und/oder therapeutische Immunreaktion gegenüber einem Antigen zu erzeugen. Eine Vakzine kann daher einer Vermeidung einer Erkrankung dienen und vor beispielsweise einer Infektion verabreicht werden oder sie kann nach Ausbruch einer Erkrankung verabreicht werden. Eine Vakzine kann natürliche, derivatisierte, synthetische, rekombinante oder nicht-rekombinante Antigene bzw. die dafür kodierende(n) Nukleinsäure(n) umfassen. Erfindungsgemäß enthält eine Vakzine RNA, die Polynukleotid-Sequenzen aufweist, die für ein oder mehrere Antigene kodieren. Die RNA kann nackte RNA sein oder kann in Liposomen oder andere Partikel für einen Gentransfer eingebaut sein. Weitere Mittel, die in die Vakzine eingebaut werden können, um eine Verabreichung zu erleichtern, umfassen Polypeptide, Peptide, Polysaccharid-Konjugate, Lipide und dergleichen.

Der Fachmann wird erkennen, dass ein Prinzip der Immunbiologie und Vakzinierung darin beruht, dass eine immunprotektive Reaktion gegen eine Erkrankung dadurch erzeugt wird, dass ein Lebewesen mit einem Antigen immunisiert wird, das bezüglich der zu behandelnden Erkrankung immunologisch relevant ist. Daher wird verstanden werden, dass in den erfindungsgemäßen Verfahren einer Behandlung von Krebs, Infektionserkrankungen und dergleichen Vakzinen einbezogen werden sollen, die Antigene umfassen, die für die zu vermeidende oder zu behandelnde Erkrankung immunologisch relevant sind. Beispielsweise würden Krebsvakzinen ein oder mehrere Krebsantigene umfassen.

Bei einer RNA-Vakzine wird eine RNA, die operabel für ein immunogenes Peptid oder Protein kodiert und sich vorzugsweise in einem pharmazeutisch verträglichen Träger befindet, an die Zellen eines Tiers verabreicht, das beispielsweise an Krebs oder an einer pathogenen Infektion leidet, wobei die RNA in die Zellen eingebaut wird und eine Menge eines immunogenen Peptids oder Proteins erzeugt wird, das gegebenenfalls nach Prozessierung fähig ist, eine schützende oder therapeutisch wirksame Immunreaktion zu erzeugen.

Das den Zellen zugeführte RNA-Material kann die gesamte Sequenz oder nur einen Teil eines immunogenen Peptids oder Proteins enthalten. Es kann auch Sequenzen enthalten, die für andere Polypeptidsequenzen kodieren. Weiterhin kann es Elemente enthalten, die bei einer Regulation der Genexpression beteiligt sind (z.B. Promotor-, Enhancer-, 5'- oder 3'-UTR-Sequenzen, und dergleichen). Die RNA kann auch eine immunstimulierende Sequenz umfassen, die die Immunogenizität eines bestimmten Genprodukts verstärkt und/oder sie kann Sequenzen umfassen, die die Aufnahme des Polynukleotids verstärken.

In diesem Zusammenhang soll angemerkt werden, dass für eine Wirksamkeit eine erfindungsgemäße Vakzine Immunität nur in einem Teil der Bevölkerung auslösen kann, da manche Individuen keine Fähigkeit aufweisen könnten, eine robuste oder schützende Immunreaktion zu erzeugen oder in manchen Fällen eine jegliche Immunreaktion gegenüber der Vakzine zu erzeugen. Die Unfähigkeit könnte Ursache in dem genetischen Hintergrund des Individuums oder in einem Immundefizienzzustand (entweder erworben oder kongenital) oder in einer Immunsuppression (beispielsweise durch Behandlung mit Immunsuppressiva, um Organabstoßung zu verhindern oder einen Autoimmunzustand zu unterdrücken) haben.

Effektorzellen wie hierin beschrieben sind Zellen, die Effektorfunktionen während einer Immunreaktion durchführen. Solche Zellen sekretieren beispielsweise Cytokine und/oder Chemokine, töten Mikroben ab, erkennen infizierte oder entartete Zellen und töten sie gegebenenfalls ab und sekretieren Antikörper. Beispiele umfassen in nicht begrenzender Weise T-Zellen (cytotoxische T-Zellen, Helfer-T-Zellen, Tumor-infiltrierende T-Zellen), B-Zellen, NK-Zellen, Neutrophile, Makrophagen und Dendritische Zellen.

Dendritische Zellen umfassen eine heterogene Zellpopulation mit bestimmter Morphologie und einer weit verbreiteten Gewebsverteilung. Das dendritische Zellsystem und dessen Rolle in dem Immunsystem wurde von Steinman, R.M., Annu. Rev. Immunol. 9:271-296 (1991) erörtert, wobei die Offenbarung durch Bezugnahme eingeschlossen ist. Dendritische Zellen besitzen eine Kapazität für eine Sensibilisierung MHC-restringierter T-Zellen und sind sehr wirksam bei einer Präsentierung von Antigenen gegenüber T-Zellen. Der Begriff "Dendritische Zellen" oder "DC" betrifft Mitglieder einer diversen Population von morphologisch ähnlichen Zelltypen, die sich in lymphoiden oder nicht-lymphoiden Geweben finden. Dendritische Zellen sind eine Klasse von "professionellen" Antigen-präsentierenden Zellen und weisen eine Fähigkeit zur Sensibilisierung MHC-restringierter T-Zellen auf. Abhängig von der jeweiligen Linie und dem jeweiligen Reifegrad können Dendritische Zellen durch Funktion oder Phänotyp, insbesondere durch den Zelloberflächen-Phänotyp erkannt werden. Diese Zellen zeichnen sich durch eine bestimmte Morphologie, phagozytische/endozytische Fähigkeit, einen hohen Grad an Oberflächen-MHC-Klasse II-Expression und die Fähigkeit, Antigene gegenüber T-Zellen, insbesondere naiven T-Zellen, zu präsentieren, aus. Funktionell können dendritische Zellen durch einen Test identifiziert werden, bei dem die Fähigkeit zur Antigenpräsentation festgestellt wird. Ein solcher Test kann eine Bewertung der Fähigkeit, T-Zellen durch Präsentation eines Test-Antigens zu stimulieren, und gegebenenfalls eine Bestimmung der T-Zell-Proliferation, Freisetzung von IL-2 und dergleichen umfassen.

Erfindungsgemäß können lymphoide dendritische Zellen, die in vivo oder in vitro gegenüber RNA exponiert wurden, als Antigen-präsentierende Zellen für die Induktion einer Immunreaktion gegenüber Antigenen, die durch die RNA kodiert werden, verwendet werden.

Immunadjuvantien oder Adjuvantien sind Verbindungen, die bei einer Verabreichung an ein Individuum die Immunreaktion gegen ein Antigen gegenüber einem Test-Individuum, an das nur das Antigen verabreicht wird, erhöhen oder bestimmte Aktivitäten von Zellen des Immunsystems verstärken.

Erfindungsgemäß kann für ein oder mehrere Antigene kodierende RNA mit einem jeglichen Adjuvanz verabreicht werden. Der Begriff "Adjuvanz" betrifft dabei eine jegliche Substanz, die von dem Antigen und Flt3-Ligand verschieden ist, und bei einem Einbau in eine Vakzine die Immunreaktion eines Wirts gegenüber einem Antigen beschleunigt, verlängert oder verstärkt. Obwohl Flt3-Ligand erfindungsgemäß nicht als Adjuvanz wie hierin definiert betrachtet wird, kann er dennoch aufgrund seiner beschriebenen Wirkung, Immunreaktionen zu verstärken, als Adjuvanz betrachtet werden. Jedoch wird für die Zwecke der Klarheit Flt3-Ligand hier nicht als Adjuvanz bezeichnet. Man vermutet, dass Adjuvanzien ihre biologischen Wirkungen durch eine oder mehrere Mechanismen ausüben, einschließlich einer Erhöhung der Oberfläche eines Antigens, einer Verlängerung der Retention des Antigens in dem Körper, einer Verlangsamung der Freisetzung des Antigens, einer Zielsteuerung eines Antigens zu Makrophagen, einer Erhöhung der Aufnahme von Antigen, einer Steigerung von Antigen-Prozessierung, einer Stimulierung von Cytokinfreisetzung, einer Stimulierung und Aktivierung von Immunzellen wie B-Zellen, Makrophagen, Dendritischen Zellen, T-Zellen und einer anderweitigen Auslösung einer nicht-spezifischen Aktivierung der Zellen des Immunsystems. Adjuvanzien umfassen eine heterogene Gruppe von Verbindungen wie Öl-Emulsionen (beispielsweise Freundsches Adjuvanz), mineralische Verbindungen (wie Alaun), bakterielle Produkte (wie Bordetella pertussis-Toxin), Liposomen und immunstimulierende Komplexe.

Ein "Hilfsmolekül" wie hierin definiert ist ein Molekül, das gegebenenfalls an ein Lebewesen verabreicht wird, um die Immunreaktion des Lebewesens gegenüber einem Antigen zu beschleunigen, verlängern oder zu verstärken. Beispielsweise können Cytokine, Wachstumsfaktoren und dergleichen bei einer Verstärkung oder Modulierung einer Immunreaktion verwendet werden. Cytokine umfassen in nicht begrenzender Weise Interleukine wie Interleukin-1, 2, 3, 4, 5, 6, 7, 10, 12, 15, 18 und 23, Chemokine, GM-CSF, G-CSF, Interferon-α und -γ, Mitglieder der TNF-Familie wie TNF-α, TGF-β, CpG-Sequenzen und dergleichen.

Die an die Zellen zugeführte RNA kann auch Antisense-RNA oder siRNA sein. Somit kann erfindungsgemäß der hierin beschriebene Flt3-Ligand verwendet werden, um Antisense-RNA oder siRNA in Zielzellen zuzuführen.

Die Fähigkeit einer Zusammensetzung, die Aktivität von T-Zell-Rezeptoren zu modulieren, kann einfach durch einen in vitro-Test bestimmt werden. Typischerweise werden T-Zellen für die Tests durch transformierte T-Zelllinien bereitgestellt, wie T-Zell-Hybridome oder T-Zellen, die aus einem Säuger wie einem Menschen oder einem Nagetier wie einer Maus isoliert werden. Geeignete T-Zell-Hybridome sind frei verfügbar oder können in an sich bekannter Weise hergestellt werden. T-Zellen können in an sich bekannter Weise aus einem Säuger isoliert werden; vgl. z.B. Shimonkevitz, R. et al., 1983, J. Exp. Med. 158:303.

Ein geeigneter Test zur Bestimmung, ob eine Zusammensetzung zur Modulation der Aktivität von T-Zellen fähig ist, erfolgt wie folgt durch die nachstehenden Schritte 1-4. T-Zellen exprimieren in geeigneter Weise einen Marker, der getestet werden kann und der T-Zell-Aktivierung oder Modulation der T-Zell-Aktivität nach Aktivierung anzeigt. So kann das Maus-T-Zell-Hybridom DO11.10, das Interleukin-2 (IL-2) bei einer Aktivierung exprimiert, verwendet werden. IL-2-Konzentrationen können gemessen werden, um zu bestimmen, ob eine Zusammensetzung zur Modulation der Aktivität dieses T-Zell-Hybridoms fähig ist. Ein derartiger geeigneter Test erfolgt durch die nachstehenden Schritte:
1. T-Zellen werden z.B. aus einem interessierenden T-Zell-Hybridom oder durch Isolierung aus einem Säuger erhalten.
2. Die T-Zellen werden unter Bedingungen kultiviert, die eine Vermehrung erlauben.
3. Die wachsenden T-Zellen werden mit Antigen-präsentierenden Zellen in Kontakt gebracht, die ihrerseits mit einem Antigen oder einer dafür kodierenden Nukleinsäure in Kontakt gebracht wurden.
4. Die T-Zellen werden auf einen Marker getestet, z.B. wird die IL-2-Produktion gemessen.

Die in den Tests verwendeten T-Zellen werden unter für eine Vermehrung geeigneten Bedingungen inkubiert. Zum Beispiel wird ein DO11.10-T-Zell-Hybridom geeigneterweise bei etwa 37°C und 5% CO₂ im Vollmedium (RPMI 1640, supplementiert mit 10% FBS, Penicillin/Streptomycin, L-Glutamin und 5 x 10⁻⁵ M 2-Mercaptoethanol) inkubiert. T-Zell-Aktivierungssignale werden durch Antigen-präsentierende Zellen bereitgestellt, die mit dem geeigneten antigenen Peptid beladen worden waren.

Alternativ zu der Messung eines exprimierten Proteins wie IL-2 kann die Modulation der T-Zell-Aktivierung geeigneter Weise durch Veränderungen der Vermehrung von Antigenabhängigen T-Zellen, wie gemessen durch bekannte Radiomarkierungsverfahren, bestimmt werden. Zum Beispiel kann ein markiertes (wie tritiert) Nukleotid in ein Testkulturmedium eingebracht werden. Das Einbringen eines derartigen markierten Nukleotids in die DNA dient als Messgröße für die T-Zell-Vermehrung. Dieser Test ist nicht für T-Zellen geeignet, die keiner Antigen-Präsentation für das Wachstum bedürfen, wie T-Zell-Hybridome. Der Test ist für die Messung der Modulation von T-Zell-Aktivierung im Fall von nicht-transformierten T-Zellen, die aus Säugern isoliert wurden, geeignet.

Die Fähigkeit, eine Immunreaktion zu induzieren, einschließlich eine Vakzinierung gegen eine Zielerkrankung zu ermöglichen, kann einfach durch einen in vivo-Test bestimmt werden. Zum Beispiel kann eine Zusammensetzung an einen Säuger wie eine Maus verabreicht werden und Blutproben aus dem Säuger zum Zeitpunkt der ersten Verabreichung und mehrfach in periodischen Zeiträumen danach (wie 1, 2, 5 und 8 Wochen nach Verabreichung) entnommen werden. Serum wird aus den Blutproben gewonnen und auf das Auftreten von durch die Immunisierung entstandenen Antikörpern getestet. Antikörper-Konzentrationen können bestimmt werden. Daneben können aus dem Blut bzw. aus lymphatischen Organen T-Lymphozyten isoliert werden und funktionell auf Reaktivitität gegen das Antigen bzw. von dem Antigen abgeleitete Epitope getestet werden. Alle dem Fachmann bekannten "Readout"-Systeme u.a. Proliferationsassay, Zytokinsekretion, zytotoxische Aktivität, Tetrameranalyse können hierbei verwendet werden.

Ein Nukleinsäuremolekül oder eine Nukleinsäuresequenz betrifft erfindungsgemäß eine Nukleinsäure, die vorzugsweise Desoxyribonukleinsäure (DNA) oder Ribonukleinsäure (RNA) ist. Nukleinsäuren umfassen erfindungsgemäß genomische DNA, cDNA, mRNA, rekombinant hergestellte und chemisch synthetisierte Moleküle. Eine Nukleinsäure kann erfindungsgemäß als einzelsträngiges oder doppelsträngiges und lineares oder kovalent kreisförmig geschlossenes Molekül vorliegen.

Der Begriff "RNA" betrifft ein Molekül, das mindestens einen Ribonukleotidrest umfasst. "Ribonukleotid" betrifft ein Nukleotid mit einer Hydroxylgruppe an der 2'-Position einer beta-D-Ribofuranosegruppe. Der Begriff umfasst doppelsträngige RNA, einzelsträngige RNA, isolierte RNA, wie teilweise oder vollständig aufgereinigte RNA, im wesentlichen reine RNA, synthetische RNA, rekombinant hergestellte RNA, sowie veränderte RNA, die sich von natürlich auftretender RNA durch die Addition, Deletion, Substitution und/oder Veränderung eines oder mehrerer Nukleotide unterscheidet. Solche Veränderungen können die Addition von nicht-Nukleotid-Material, wie an dem (den) Ende(n) einer RNA oder im inneren davon, beispielsweise an einem oder mehreren Nukleotiden der RNA umfassen. Nukleotide in RNA-Molekülen können auch nicht-Standard-Nukleotide wie nicht-natürlicherweise auftretende Nukleotide oder chemisch synthetisierte Nukleotide oder Desoxynukleotide umfassen. Diese veränderten RNAs können als Analoga oder Analoga von natürlicherweise auftretender RNA bezeichnet werden.

"mRNA" bedeutet "messenger-RNA" und betrifft ein "Transkript", das unter Verwendung von DNA als Matrize produziert wird und selbst für ein Peptid oder Protein kodiert. Eine mRNA umfasst typischerweise eine 5'-nicht-translatierte Region, eine Protein-kodierende Region und eine 3'-nicht-translatierte Region. mRNA hat eine begrenzte Halbwertszeit in Zellen und in vitro. Erfindungsgemäß kann mRNA durch in vitro-Transkription von einer DNA-Matrize hergestellt werden.

Erfindungsgemäß kann RNA mit Modifikationen versehen werden, die beispielsweise die Stabilität der RNA und/oder Effizienz mit der die RNA translatiert wird, erhöhen. So kann die RNA beispielsweise mit einer Poly(A)-Sequenz, insbesondere einer offen endenden Poly(A)-Sequenz, versehen werden. Es konnte gezeigt werden, dass RNA mit einer offen endenden Poly(A)-Sequenz effizienter translatiert wird als RNA mit einer verdeckt endenden Poly(A)-Sequenz. Ferner wurde festgestellt, dass eine lange Poly(A)-Sequenz, insbesondere von etwa 120 bp, zu einer optimalen Transkriptstabilität und Translationseffizienz von RNA führt. Auch konnte gezeigt werden, dass eine doppelte 3'-nicht translatierte Region (UTR), insbesondere des humanen Beta-Globin-Gens, in einem RNA-Molekül zu einer Verbesserung der Translationseffizienz führt, die über den Summationseffekt, der mit zwei einzelnen UTR zu erwarten ist, deutlich hinaus geht. Eine Kombination der vorstehend beschriebenen Modifikationen kann einen synergistischen Einfluss auf die Stabilisierung der RNA und die Erhöhung der Translation haben. Derartige Modifikationen sind in der PCT/EP2006/009448 beschrieben, die hierin durch Bezugnahme eingeschlossen ist, und erfindungsgemäß vorgesehen.

Vorzugsweise wird erfindungsgemäß eine Modifizierung und dadurch Stabilisierung und/oder Steigerung der Translationseffizienz von RNA dadurch erreicht, dass Expressionsvektoren gentechnisch modifiziert werden, die vorzugsweise als Matrize für die in vitro-Transkription von RNA dienen.

Solche Vektoren sollen insbesondere die Transkription von RNA mit einer Poly(A)-Sequenz erlauben, wobei die Poly(A)-Sequenz vorzugsweise in der RNA ein offenes Ende aufweist, d.h. keine von A-Nukleotiden verschiedene Nukleotide die Poly(A)-Sequenz an ihrem 3'-Ende flankieren. Eine offen endende Poly(A)-Sequenz in der RNA kann dadurch erreicht werden, dass in einen Expressionsvektor, welcher die Transkription von RNA unter der Kontrolle eines 5' gelegenen RNA-Polymerase-Promotors erlaubt und eine Polyadenylkassette (Poly(A)-Sequenz) enthält, eine Restriktionsschnittstelle vom Typ IIs eingebracht wird, wobei die Erkennungssequenz 3' von der Poly(A)-Sequenz liegt, während die Schnittstelle stromaufwärts und damit innerhalb der Poly(A)-Sequenz liegt. Durch Restriktionsspaltung an der Restriktionsschnittstelle vom Typ IIs wird bei einem Plasmid eine Linearisierung des Plasmids innerhalb der Poly(A)-Sequenz möglich. Das linearisierte Plasmid kann dann als Matrize für eine in vitro-Transkription verwendet werden, wobei das resultierende Transkript mit einer unverdeckten Poly(A)-Sequenz endet.

Weiterhin oder alternativ kann erfindungsgemäß eine Modifizierung und dadurch Stabilisierung und/oder Steigerung der Translationseffizienz von RNA dadurch erreicht werden, dass Expressionsvektoren gentechnisch derart modifiziert werden, dass sie die Transkription von RNA mit zwei oder mehreren 3'-nicht translatierten Regionen an ihrem 3'-Ende und vorzugsweise zwischen der für ein Peptid oder Protein kodierenden Sequenz (offenes Leseraster) und der Poly(A)-Sequenz erlauben.

In einer bevorzugten Ausführungsform wird erfindungsgemäß RNA durch in vitro-Transkription einer geeigneten DNA-Matrize erhalten. Der Promotor für eine Steuerung der Transkription kann ein jeglicher Promotor für eine RNA-Polymerase sein. Spezifische Beispiele für RNA-Polymerasen sind die T7-, T3- und SP6-RNA-Polymerasen. Vorzugsweise wird die in vitro-Transkription erfindungsgemäß durch einen T7- oder SP6-Promotor gesteuert.

Eine DNA-Matrize für eine in vitro-Transkription kann durch Klonieren einer Nukleinsäure, insbesondere cDNA, und Einbringen der Nukleinsäure in einen für eine in vitro-Transkription geeigneten Vektor hergestellt werden.

Erfindungsgemäß bedeutet der Begriff, "RNA, die kodiert" im Hinblick auf ein Antigen, dass die RNA, falls sie in einer geeigneten Umgebung, vorzugsweise in einer Zelle, vorliegt, exprimiert werden kann, um das Antigen zu produzieren. Vorzugsweise ist die RNA fähig, mit der zellulären Translationsmaschinerie wechselzuwirken, um das Antigen bereitzustellen, das sie kodiert.

Wenn erfindungsgemäß darauf Bezug genommen wird, dass RNA mehr als ein Antigen exprimiert, kann die RNA verschiedene RNA-Moleküle umfassen, die verschiedene dieser mehreren Antigene exprimieren. Jedoch umfasst die Erfindung auch Fälle, bei denen ein RNA-Molekül verschiedene Antigene exprimiert, die gegebenenfalls miteinander verbunden sind.

Erfindungsgemäß kann eine jegliche Technik, die zum Transfer von RNA in Zellen geeignet ist, verwendet werden, um RNA in Zellen einzubringen. Vorzugsweise wird RNA in Zellen durch Standardtechniken transfiziert. Solche Techniken umfassen Elektroporation, Lipofektion und Mikroinjektion. Vorzugsweise verursacht ein Einbringen von RNA, die ein Antigen kodiert, in eine Zelle eine Expression von Antigen in der Zelle.

Weiterhin umfasst der Begriff "Nukleinsäure" auch Derivate von Nukleinsäuren oder Nukleinsäuresequenzen wie eine chemische Derivatisierung einer Nukleinsäure an einer Nukleotidbase, am Zucker oder am Phosphat und Nukleinsäuren, die nicht in der Natur vorkommende Nukleotide und Nukleotidanaloga enthalten.

"3'-Ende einer Nukleinsäure" betrifft erfindungsgemäß dasjenige Ende, an dem sich eine freie Hydroxy-Gruppe befindet. In schematischer Darstellung von doppelsträngigen Nukleinsäuren, insbesondere DNA, befindet sich das 3'-Ende immer rechts. "5'-Ende einer Nukleinsäure" betrifft erfindungsgemäß dasjenige Ende, an dem sich eine freie Phosphat-Gruppe befindet. In schematischer Darstellung von doppelsträngigen Nukleinsäuren, insbesondere DNA, befindet sich das 5'-Ende immer links.

| | | |
|---|---|---|
| 5'-Ende | 5'--P-NNNNNNN-OH-3' | 3'-Ende |
| | 3'-HO-NNNNNNN-P--5' | |

"Funktionelle Verbindung" oder "funktionell verbunden" betrifft erfindungsgemäß eine Verbindung in einer funktionellen Beziehung. Eine Nukleinsäure ist "funktionell verbunden", wenn sie in eine funktionelle Beziehung mit einer anderen Nukleinsäuresequenz gesetzt wird. Zum Beispiel ist ein Promotor mit einer kodierenden Sequenz funktionell verbunden, falls er die Transkription der kodierenden Sequenz beeinflusst. Funktionell verbundene Nukleinsäuren sind typischerweise benachbart zueinander, gegebenenfalls getrennt durch weitere Nukleinsäuresequenzen.

Die erfindungsgemäß beschriebenen Nukleinsäuren sind vorzugsweise isoliert. Der Begriff "isolierte Nukleinsäure" bedeutet erfindungsgemäß, dass die Nukleinsäure (i) in vitro amplifiziert wurde, zum Beispiel durch Polymerase-Kettenreaktion (PCR), (ii) rekombinant durch Klonierung produziert wurde, (iii) gereinigt wurde, zum Beispiel durch Spaltung und gelelektrophoretische Auftrennung, oder (iv) synthetisiert wurde, zum Beispiel durch chemische Synthese. Eine isolierte Nukleinsäure ist eine Nukleinsäure, die für eine Manipulierung durch rekombinante DNA-Techniken zur Verfügung steht.

Erfindungsgemäß betrifft eine "Nukleinsäuresequenz, die von einer Nukleinsäuresequenz abgeleitet ist" eine Nukleinsäure, in der im Vergleich zu der Nukleinsäure, von der sie abgeleitet ist, einzelne oder multiple Nukleotidsubstitutionen, -deletionen und/oder -additionen vorliegen, wobei zwischen den Nukleinsäuren ein gewisser Grad an Homologie vorhanden ist, d.h. die Nukleinsäuren weisen in der Abfolge ihrer Nukleotide signifikante direkte oder komplementäre Übereinstimmungen auf. Eine von einer Nukleinsäure abgeleitete Nukleinsäure weist erfindungsgemäß eine funktionelle Eigenschaft der Nukleinsäure auf, von der sie abgeleitet ist. Solche Eigenschaften sind insbesondere durch die Eigenschaften der Expressionsprodukte der Nukleinsäuren definiert. Im Fall von Flt3-Ligand betrifft dies insbesondere die Eigenschaften, an Flt3-Rezeptor zu binden und vorzugsweise die biologische Aktivität aufzuweisen, ein stimulatorisches Signal an die Zelle über den gebundenen Flt3-Rezeptor zu transduzieren, und/oder bei gemeinsamer Verabreichung mit einer Vakzine-RNA die durch die RNA ausgelöste Immunreaktion verstärken zu können. Im Fall von Antigenen betrifft dies die Eigenschaft, eine Immunreaktion mit vergleichbarer Spezifität und/oder Reaktivität auslösen zu können. Ein Beispiel für eine "Nukleinsäuresequenz, die von einer Nukleinsäuresequenz abgeleitet ist" ist eine Nukleinsäure, in der im Vergleich zu der Nukleinsäure, von der sie abgeleitet ist, Codon-Optimierungen für beispielsweise eine bessere Expression in einem bestimmten Wirtsorganismus oder einer bestimmten Wirtszelle vorliegen.

Eine von einer Nukleinsäuresequenz abgeleitete Sequenz oder der Begriff "von einer Nukleinsäuresequenz abgeleitete Sequenz" betrifft vorzugsweise homologe Sequenzen.

Vorzugsweise beträgt der Grad an Identität zwischen homologen Nukleinsäuren erfindungsgemäß mindestens 70%, insbesondere mindestens 75%, mindestens 80%, mindestens 85%, mindestens 90%, mindestens 95%, mindestens 96%, mindestens 97%, mindestens 98%, und vorzugsweise mindestens 99%. Der Grad an Identität ist vorzugsweise für einen Bereich von mindestens etwa 30, mindestens etwa 50, mindestens etwa 70, mindestens etwa 90, mindestens etwa 100, mindestens etwa 150, mindestens etwa 200, mindestens etwa 300, mindestens etwa 400, mindestens etwa 500, oder mindestens etwa 1000 konsekutiven Nukleotiden angegeben. In bevorzugten Ausführungsformen, ist der Grad an Identität für die gesamte Länge der Referenznukleinsäure wie der im Sequenzprotokoll angegebenen Nukleinsäuresequenzen angegeben.

Der Begriff "% Identität" soll einen Prozentwert an Nukleotiden bezeichnen, die zwischen zwei zu vergleichenden Sequenzen bei einem optimalen Alignment identisch sind, wobei dieser Prozentwert rein statistisch ist, die Unterschiede zwischen den zwei Sequenzen zufällig und über die gesamte Sequenzlänge verteilt sein können und die zu vergleichende Sequenz Additionen oder Deletionen im Vergleich zu der Referenzsequenz umfassen kann, um ein optimales Alignment zwischen zwei Sequenzen zu erreichen. Sequenzvergleiche zwischen zwei Sequenzen werden im Allgemeinen durch Vergleich dieser Sequenzen nach einem optimalen Alignment in Bezug auf ein Segment oder "Vergleichsfenster" durchgeführt, um lokale Bereiche einer Sequenzübereinstimmung zu identifizieren. Das optimale Alignment für einen Vergleich kann manuell oder mit Hilfe des lokalen Homologiealgorithmus von Smith and Waterman, 1981, Ads App. Math. 2, 482, mit Hilfe des lokalen Homologiealgorithmus von Neddleman and Wunsch, 1970, J. Mol. Biol. 48, 443, und mit Hilfe des Ähnlichkeitssuchalgorithmus von Pearson and Lipman, 1988, Proc. Natl Acad. Sci. USA 85, 2444, oder mit Hilfe von Computerprogrammen, die diese Algorithmen verwenden, (GAP, BESTFIT, FASTA, BLAST P, BLAST N and TFASTA in Wisconsin Genetics Software Package, Genetics Computer Group, 575 Science Drive, Madison, Wis.) durchgeführt werden.

Die prozentuale Identität wird durch Bestimmung der Anzahl an identischen Positionen, in denen die zu vergleichenden Sequenzen übereinstimmen, Teilung dieser Anzahl durch die verglichenen Positionen und Multiplikation dieses Ergebnisses mit 100 erhalten.

Beispielsweise kann das Programm BLAST "BLAST 2 sequences", das von der Website http://www.ncbi.nlm.nih.gov/blast/bl2seq/wblast2.cgi erhältlich ist, verwendet werden.

Eine Nukleinsäure ist insbesondere dann zu einer anderen Nukleinsäure "homolog", wenn die beiden Sequenzen der komplementären Stränge miteinander hybridisieren und ein stabiles Duplex eingehen können, wobei die Hybridisierung vorzugsweise unter Bedingungen erfolgt, die eine spezifische Hybridisierung zwischen Polynukleotiden erlauben (stringente Bedingungen). Stringente Bedingungen sind beispielsweise in Molecular Cloning: A Laboratory Manual, J. Sambrook et al., Hrsg., 2. Auflage, Cold Spring Harbor Laboratory Press, Cold Spring Harbor, New York, 1989 oder Current Protocols in Molecular Biology, F.M. Ausubel et al., Hrsg., John Wiley & Sons, Inc., New York, beschrieben und betreffen beispielsweise die Hybridisierung bei 65°C in Hybridisierungspuffer (3,5 x SSC, 0,02% Ficoll, 0,02% Polyvinylpyrrolidon, 0,02% Rinderserumalbumin, 2,5mM NaH₂PO₄ (pH 7), 0,5% SDS, 2mM EDTA). SSC ist 0,15 M Natriumchlorid / 0,15 M Natriumcitrat, pH 7. Nach der Hybridisierung wird die Membran, auf die die DNA übertragen wurde beispielsweise in 2 x SSC bei Raumtemperatur und sodann in 0,1 - 0,5 x SSC/ 0,1 x SDS bei Temperaturen bis 68°C gewaschen.

Prozentuale Komplementarität gibt den prozentualen Wert an konsekutiven Nukleotiden in einer Nukleinsäure an, die mit einer zweiten Nukleinsäure Wasserstoffbrückenbindungen (z.B. durch Watson-Crick-Bsaenpaarung) ausbilden können. Komplementäre Nukleinsäuren weisen erfindungsgemäß vorzugsweise mindestens 40%, insbesondere mindestens 50%, mindestens 60%, mindestens 70%, mindestens 80%, mindestens 90% und vorzugsweise mindestens 95%, mindestens 98% oder mindestens 99% komplementäre Nukleotide auf. Vorzugsweise sind komplementäre Nukleinsäuren vollständig komplementär, was bedeutet, dass alle konsekutiven Nukleotide mit der gleichen Anzahl an konsekutiven Nukleotiden in einer zweiten Nukleinsäure Wasserstoffbrückenbindungen eingehen werden.

"Sequenzähnlichkeit" gibt den prozentualen Wert an Aminosäuren an, die entweder identisch sind oder konservative Aminosäuresubstitutionen darstellen. "Sequenzidentität" zwischen zwei Polypeptiden oder Nukleinsäuren gibt den prozentualen Wert an Aminosäuren oder Nukleotiden an, die zwischen den Sequenzen identisch sind.

Mit "Derivat" einer Nukleinsäure ist erfindungsgemäß gemeint, dass einzelne oder multiple Nukleotidsubstitutionen, -deletionen und/oder -additionen in der Nukleinsäure vorliegen. Weiterhin umfasst der Begriff "Derivat" auch eine chemische Derivatisierung einer Nukleinsäure an einer Base, einem Zucker oder Phosphat eines Nukleotids. Der Begriff "Derivat" umfasst auch Nukleinsäuren, die nicht in der Natur vorkommende Nukleotide und Nukleotidanaloga enthalten.

Derivate einer bestimmten Nukleinsäure betreffen insbesondere Varianten der Nukleinsäure, insbesondere Splicevarianten, Isoformen und Species-Homologe der Nukleinsäure, insbesondere solche, die natürlicherweise exprimiert werden.

Nukleinsäuren können bezüglich Varianten wie Splicevarianten erfindungsgemäß in an sich bekannter Weise analysiert werden. Techniken zur Analyse von Splicevarianten umfassen Reverse Transkription-Polymerase-Kettenreaktion (RT-PCR), Northern Blotting und in situ-Hybridisierung.

Eine "RNAse-Protektion" genannte Technik kann auch verwendet werden, um alternativ gesplicte mRNAs zu identifizieren. RNAse-Protektion umfasst die Transkription einer Gensequenz in synthetische RNA, die an RNA, die beispielsweise von anderen Zellen abgeleitet ist, hybridisiert wird. Die hybridisierte RNA wird sodann mit Enzymen inkubiert, die RNA:RNA-Hybrid-Fehlpaarungen erkennen. Fragmente, die kleiner als erwartet sind, zeigen das Vorliegen alternativ gesplicter mRNAs an. Die putativen alternativ gesplicten mRNAs können in an sich bekannter Weise kloniert und sequenziert werden.

RT-PCR kann auch verwendet werden, um alternativ gesplicte mRNAs zu identifizieren. Bei der RT-PCR wird mRNA in cDNA durch das Enzym reverse Transkriptase in an sich bekannter Weise konvertiert. Die gesamte kodierende Sequenz der cDNA wird sodann mittels PCR unter Verwendung eines Vorwärts-Primers, der in der 3'-nicht-translatierten Region liegt, und eines reversen Primers, der in der 5'-nicht-translatierten Region liegt, amplifiziert. Die Amplifikationsprodukte können bezüglich alternativer Spliceformen beispielsweise durch Vergleich der Größe der amplifizierten Produkte mit der Größe des erwarteten Produkts aus normal gespliceter mRNA beispielsweise mittels Agarose-Gel-Elektrophorese analysiert werden. Jegliche Veränderungen hinsichtlich der Größe der Amplifikationsprodukte können ein alternatives Splicen anzeigen.

Von mutierten Genen abgeleitete mRNA aus kann auch einfach mit Hilfe der vorstehend beschriebenen Techniken zur Identifizierung alternativer Spliceformen identifiziert werden. So können beispielsweise allelische Formen von Genen und die dadurch produzierte mRNA, die erfindungsgemäß als "Mutanten" betrachtet werden, identifiziert werden.

Nukleinsäuren können erfindungsgemäß alleine oder in Kombination mit anderen Nukleinsäuren, die homo- oder heterolog sein können, vorliegen. In bestimmten Ausführungsformen liegt eine Nukleinsäure erfindungsgemäß funktionell in Verbindung mit Expressionskontrollsequenzen vor, die in Bezug zu der Nukleinsäure homolog oder heterolog sein können. Der Begriff "homolog" bezeichnet, dass eine Nukleinsäure auch natürlicherweise mit der Nukleinsäure, mit der sie in Kombination vorliegt, funktionell verbunden ist und der Begriff "heterolog" bezeichnet, dass eine Nukleinsäure nicht natürlicherweise mit der Nukleinsäure, mit der sie in Kombination vorliegt, funktionell verbunden ist.

Eine transkribierbare Nukleinsäure, insbesondere eine für ein Peptid oder Protein kodierende Nukleinsäure, und eine Expressionskontrollsequenz sind dann "funktionell" miteinander verbunden, falls sie derart kovalent miteinander verknüpft sind, dass die Transkription oder Expression der transkribierbaren und insbesondere kodierenden Nukleinsäure unter der Kontrolle oder unter dem Einfluss der Expressionskontrollsequenz steht. Falls die Nukleinsäure in ein funktionelles Peptid oder Protein translatiert werden soll, führt bei einer funktionellen Verbindung einer Expressionskontrollsequenz mit der kodierenden Sequenz eine Induktion der Expressionskontrollsequenz zu einer Transkription der kodierenden Sequenz, ohne dass es zu einer Leserasterverschiebung in der kodierenden Sequenz oder zu einem Unvermögen der kodierenden Sequenz kommt, in das gewünschte Peptid oder Protein translatiert zu werden.

Der Begriff "Expressionskontrollsequenz" umfasst erfindungsgemäß Promotoren, ribosombindende Sequenzen und andere Kontrollelemente, welche die Transkription eines Gens oder die Translation der abgeleiteten RNA steuern. In bestimmten erfindungsgemäßen Ausführungsformen sind die Expressionskontrollsequenzen regulierbar. Die genaue Struktur von Expressionskontrollsequenzen kann speziesabhängig oder zelltypusabhängig variieren, umfasst jedoch im allgemeinen 5'-nicht-transkribierte und 5'- und 3'-nicht-translatierte Sequenzen, die an der Initiation der Transkription bzw. Translation beteiligt sind wie TATA-Box, Capping-Sequenz, CAAT-Sequenz und ähnliches. Insbesondere umfassen 5'-nicht-transkribierte-Expressionskontrollsequenzen eine Promotorregion, die eine Promotorsequenz für eine transkriptionelle Kontrolle des funktionell verbundenen Gens einschließt. Expressionskontrollsequenzen können auch Enhancer-Sequenzen oder stromaufwärts gelegene Aktivatorsequenzen umfassen.

Der Begriff "Promotor" oder "Promotorregion" betrifft eine DNA-Sequenz, die stromaufwärts (5') zu der kodierenden Sequenz eines Gens liegt und die Expression der kodierenden Sequenz durch Bereitstellen einer Erkennungs- und Bindungsstelle für RNA-Polymerase steuert. Die Promotorregion kann weitere Erkennungs- oder Bindungsstellen für weitere Faktoren beinhalten, die an der Regulation der Transkription des Gens beteiligt sind. Ein Promotor kann die Transkription eines prokaryontischen oder eukaryontischen Gens steuern. Ein Promotor kann "induzierbar" sein und die Transkription in Reaktion auf ein Induktionsmittel initiieren oder er kann "konstitutiv" sein, falls die Transkription nicht durch ein Induktionsmittel gesteuert wird. Ein induzierbarer Promotor wird nicht exprimiert oder nur in einem sehr geringen Maß exprimiert, wenn ein Induktionsmittel fehlt. In Gegenwart des Induktionsmittels wird das Gen "angeschalten" oder das Transkriptionsniveau erhöht. Dies wird herkömmlicherweise durch die Bindung eines spezifischen Transkriptionsfaktors vermittelt.

Erfindungsgemäß bevorzugte Promotoren sind beispielsweise Promotoren für SP6-, T3- oder T7-Polymerase.

Der Begriff "Expression" wird erfindungsgemäß in seiner allgemeinsten Bedeutung verwendet und umfasst die Produktion von RNA, oder von RNA und Protein. Er umfasst auch eine teilweise Expression von Nukleinsäuren. In Bezug auf RNA betrifft der Begriff "Expression" oder "Translation" insbesondere die Produktion von Peptiden oder Proteinen. Expression kann transient oder stabil erfolgen.

Eine Nukleinsäure, die für ein Protein oder Peptid kodiert, kann erfindungsgemäß in Verbindung mit einer anderen Nukleinsäure vorliegen, die für eine Peptidsequenz kodiert, die beispielsweise eine Sekretion des durch die Nukleinsäure kodierten Proteins oder Peptids aus einer Wirtszelle steuert oder die Immunogenizität des durch die Nukleinsäure kodierten Proteins oder Peptids erhöht. Auch kann eine Nukleinsäure erfindungsgemäß in Verbindung mit einer anderen Nukleinsäure vorliegen, die für eine Peptidsequenz kodiert, die eine Verankerung des kodierten Proteins oder Peptids auf der Zellmembran einer Wirtszelle oder seine Kompartimentalisierung in bestimmte Organellen dieser Zelle herbeiführt. Gleichermaßen kann eine Verbindung mit einer Nukleinsäure erfolgen, die ein Reportergen oder einen beliebigen "Tag" darstellt.

Der Begriff "Transkription" betrifft erfindungsgemäß einen Vorgang, bei dem der genetische Code in einer DNA-Sequenz in RNA transkribiert wird. Die RNA kann darauffolgend in Protein translatiert werden. Erfindungsgemäß umfasst der Begriff "Transkription" "in vitro-Transkription", wobei der Begriff "in vitro-Transkription" ein Verfahren betrifft, bei dem RNA, insbesondere mRNA, zellfrei, d.h. vorzugsweise unter Verwendung entsprechend aufbereiteter Zellextrakte, in vitro synthetisiert wird. Vorzugsweise werden für die Herstellung von Transkripten Klonierungsvektoren eingesetzt, die im Allgemeinen als Transkriptionsvektoren bezeichnet werden und erfindungsgemäß von dem Begriff "Vektor" umfasst sind.

Der Begriff "Translation" betrifft erfindungsgemäß einen Vorgang an den Ribosomen, durch den ein Strang an mRNA den Zusammenbau einer Sequenz von Aminosäuren steuert, um ein Protein oder Peptid herzustellen.

Die 3'-nicht-translatierte Region betrifft eine Region, die am 3'-Ende eines Gens stromabwärts zu dem Terminations-Codon einer Protein-kodierenden Region liegt, transkribiert wird, jedoch nicht in eine Aminosäuresequenz translatiert wird.

Erfindungsgemäß wird eine erste Polynukleotid-Region als zu einer zweiten Polynukleotid-Region stromabwärts gelegen betrachtet, wenn das 5'-Ende der ersten Polynukleotid-Region der zu dem 3'-Ende der zweiten Polynukleotid-Region nächstgelegene Teil der ersten Polynukleotid-Region ist.

Die 3'-nicht-translatierte Region erstreckt sich typischerweise von dem Terminations-Codon für ein Translationsprodukt bis zu der Poly(A)-Sequenz, die herkömmlicherweise nach dem Transkriptionsprozess angefügt wird. Die 3'-nicht-translatierten Regionen von Säuger-mRNA weisen typischerweise einen Homologiebereich auf, der als die AAUAAA-Hexanukleotidsequenz bekannt ist. Diese Sequenz ist vermutlich das Poly(A)-Anfügungssignal. Häufig liegt es 10 bis 30 Basen vor der Poly(A)-Anfügungsstelle.

3'-nicht-translatierte Regionen können eine oder mehrere invertierte Wiederholungen enthalten, die sich in Stamm-Schleife-Strukturen falten können, die als Barriere für Exoribonukleasen fungieren oder mit Proteinen interagieren, von denen bekannt ist, dass sie die RNA-Stabilität erhöhen (z.B. RNA-bindende Proteine).

5'- und/oder 3'-nicht-translatierte Regionen können erfindungsgemäß mit einer transkribierbaren und insbesondere kodierenden Nukleinsäure funktionell verbunden sein, so dass diese Regionen derart mit der Nukleinsäure in Beziehung steht, dass sie die Stabilität und/oder Translationseffizienz der von der transkribierbaren Nukleinsäure transkribierten RNA erhöht.

Die 3'-nicht-translatierten Regionen von Immunglobulin-mRNAs sind relativ kurz (weniger als etwa 300 Nukleotide), während die 3'-nicht-translatierten Regionen anderer Gene relativ lang sind. Zum Beispiel ist die 3'-nicht-translatierte Region von tPA etwa 800 Nukleotide lang, die von Faktor VIII ist etwa 1800 Nukleotide lang und die von Erythropoietin ist etwa 560 Nukleotide lang.

Erfindungsgemäß kann ermittelt werden, ob eine 3'-nicht-translatierte Region oder eine davon abgeleitete Nukleinsäuresequenz die Stabilität und/oder Translationseffzienz von RNA erhöht, indem die 3'-nicht-translatierte Region oder die davon abgeleitete Nukleinsäuresequenz in die 3'-nicht-translatierte Region eines Gens eingebaut wird und gemessen wird, ob dieser Einbau die Menge des synthetisierten Proteins erhöht.

Vorstehendes gilt sinngemäß für den Fall, dass erfindungsgemäß eine Nukleinsäure 2 oder mehrere 3'-nicht-translatierte Regionen umfasst, die vorzugsweise sequentiell mit oder ohne einen dazwischen liegenden Linker, vorzugsweise in einer "Kopf-an-Schwanz-Beziehung" (d.h. die 3'-nicht-translatierten Regionen weisen die gleiche Orientierung, vorzugsweise die in einer Nukleinsäure natürlich auftretende Orientierung auf) gekoppelt vorliegen.

Der Begriff "Gen" betrifft erfindungsgemäß eine bestimmte Nukleinsäuresequenz, die für die Produktion eines oder mehrerer zellulärer Produkte und/oder für das Erreichen einer oder mehrerer interzellulärer oder intrazellulärer Funktionen verantwortlich ist. Insbesondere betrifft der Begriff einen DNA-Abschnitt, welcher eine Nukleinsäure umfasst, die für ein spezifisches Protein oder ein funktionelles bzw. strukturelles RNA-Molekül kodiert.

Die Begriffe "Polyadenylkassette" oder "Poly(A)-Sequenz" betreffen eine Sequenz von Adenyl-Resten, die typischerweise am 3'-Ende eines RNA-Moleküls liegt. Erfindungsgemäß ist vorgesehen, dass eine solche Sequenz durch eine DNA-Matrize anhand von sich wiederholenden Thymidyl-Resten in dem zu dem kodierenden Strang komplementären Strang bei einer Transkription von RNA angefügt wird, wohingegen normalerweise sie nicht in der DNA kodiert ist, sondern durch eine matrizenunabhängige RNA-Polymerase nach der Transkription im Zellkern an das freie 3'-Ende der RNA angeheftet wird. Erfindungsgemäß ist eine Nukleotidsequenz von mindestens 20, vorzugsweise mindestens 40, vorzugsweise mindestens 80, vorzugsweise mindestens 100 und vorzugsweise bis zu 500, vorzugsweise bis zu 400, vorzugsweise bis zu 300, vorzugsweise bis zu 200 und insbesondere bis zu 150 aufeinander folgenden A-Nukleotiden, und insbesondere etwa 120 aufeinander folgenden A-Nukleotiden als eine derartige Poly(A)-Sequenz zu verstehen, wobei der Begriff "A-Nukleotide" Adenyl-Reste bezeichnet.

"Restriktions-Endonuklease" oder "Restriktionsenzym" bezeichnet eine Klasse von Enzymen, die in beiden Strängen eines DNA-Moleküls innerhalb spezifischer Basensequenzen Phosphodiester-Bindungen spalten. Sie erkennen auf einem doppelsträngigen DNA-Molekül spezifische Bindungsstellen, die als Erkennungssequenzen bezeichnet werden. Die Stellen, an denen die Phosphodiester-Bindungen in der DNA durch die Enzyme gespalten werden, werden als Spaltstellen bezeichnet. Bei Typ IIs-Enzymen, liegt die Schnittstelle in einem definierten Abstand von der DNA-Bindungsstelle entfernt. Der Begriff "Restriktions-Endonuklease" umfasst erfindungsgemäß beispielsweise die Enzyme SapI, EciI, BpiI, AarI, AloI, BaeI, BbvCI, PpiI und PsrI, BsrDI, BtsI, EarI, BmrI, BsaI, BsmBI, FauI, BbsI, BciVI, BfuAI, BspMI, BseRI, EciI, BtgZI, BpuEI, BsgI, MmeI, CspCI, BaeI, BsaMI, Mva1269I, PctI, Bse3DI, BseMI, Bst6I, Eam1104I, Ksp632I, BfiI, Bso31I, BspTNI, Echo31I, Esp3I, BfuI, Acc36I, AarI, Eco57I, Eco57MI, GsuI, AloI, Hin4I, PpiI, und PsrI.

"Halbwertszeit" betrifft die Zeitspanne, die für eine Beseitigung der Hälfte der Aktivität, Menge oder Anzahl an Molekülen benötigt wird.

In einer bevorzugten Ausführungsform ist ein Nukleinsäuremolekül erfindungsgemäß ein Vektor. Der Begriff "Vektor" wird dabei in seiner allgemeinsten Bedeutung verwendet und umfasst jegliche intermediären Vehikel für eine Nukleinsäure, die es z.B. ermöglichen, die Nukleinsäure in prokaryontische und/oder in eukaryontische Wirtszellen einzubringen und gegebenenfalls in ein Genom zu integrieren. Solche Vektoren werden vorzugsweise in der Zelle repliziert und/oder exprimiert. Vektoren umfassen Plasmide, Phagemide oder Virusgenome. Der Begriff "Plasmid", wie hierin verwendet, betrifft allgemein ein Konstrukt von extrachromosomalem genetischen Material, gewöhnlich ein zirkuläres DNA-Duplex, das unabhängig von chromosomaler DNA replizieren kann.

Der Begriff "Wirtszelle" betrifft erfindungsgemäß jede Zelle, die mit einer exogenen Nukleinsäure, vorzugsweise DNA oder RNA, transformierbar oder transfizierbar ist. Der Begriff "Wirtszelle" umfasst erfindungsgemäß prokaryontische (z.B. E. coli) oder eukaryontische Zellen (z.B. Säugerzellen, insbesondere Zellen aus Mensch, Hefezellen und Insektenzellen). Besonders bevorzugt sind Säugerzellen wie Zellen aus Mensch, Maus, Hamster, Schwein, Ziege und Primaten. Die Zellen können aus einer Vielzahl von Gewebetypen abgeleitet sein und umfassen primäre Zellen und Zelllinien. Spezifische Beispiele umfassen Keratinocyten, periphere Blutleukocyten und Stammzellen des Knochenmarks. In weiteren Ausführungsformen ist die Wirtszelle eine Antigen-präsentierende Zelle, wobei der Begriff "Antigen-präsentierende Zelle" erfindungsgemäß Dendritische Zellen, Monocyten und Makrophagen umfasst. Eine Nukleinsäure kann in der Wirtszelle in einer einzigen oder in mehreren Kopien vorliegen und wird in einer Ausführungsform in der Wirtszelle exprimiert.

Der Begriff "Peptid" betrifft Substanzen, die zwei oder mehr, vorzugsweise 3 oder mehr, vorzugsweise 4 oder mehr, vorzugsweise 6 oder mehr, vorzugsweise 8 oder mehr, vorzugsweise 10 oder mehr, vorzugsweise 13 oder mehr, vorzugsweise 16 oder mehr, vorzugsweise 20 oder mehr and bis zu vorzugsweise 50, vorzugsweise 100 oder vorzugsweise 150 aufeinander folgende Aminosäuren umfassen, die durch Peptidbindungen miteinander verbunden sind. Der Begriff "Protein" oder "Polypeptid" betrifft große Peptide, vorzugsweise Peptide mit mindestens 151 Aminosäuren, jedoch werden die Begriffe "Peptid", "Polypeptid" und "Protein" hierin im Allgemeinen als Synonyme verwendet. Die Begriffe "Peptid", "Polypeptid" und "Protein" umfassen erfindungsgemäß Substanzen, die nicht nur Aminosäure-Bestandteile, sondern auch nicht-Aminosäure-Bestandteile wie Zucker und Phosphatstrukturen enthalten und umfassen auch Substanzen die Bindungen wie Ester-, Thioether- oder Disulfidbindungen enthalten.

Eine von einer Aminosäuresequenz abgeleitete Sequenz oder der Begriff "von einer Aminosäuresequenz abgeleitete Sequenz" betrifft erfindungsgemäß homologe Sequenzen und Derivate der ersteren Sequenz.

Eine von einer Aminosäuresequenz abgeleitete Sequenz weist erfindungsgemäß eine funktionelle Eigenschaft der Aminosäuresequenz auf, von der sie abgeleitet ist. Im Fall von Flt3-Ligand betrifft dies insbesondere die Eigenschaften, an Flt3-Rezeptor zu binden und vorzugsweise die biologische Aktivität aufzuweisen, ein stimulatorisches Signal an die Zelle über den gebundenen Flt3-Rezeptor zu transduzieren, und/oder bei gemeinsamer Verabreichung mit einer Vakzine-RNA die durch die RNA ausgelöste Immunreaktion verstärken zu können. Im Fall von Antigenen betrifft dies die Eigenschaft, eine Immunreaktion mit vergleichbarer Spezifität und/oder Reaktivität auslösen zu können.

"Homologe" oder "Derivate" eines Proteins oder Polypeptids oder einer Aminosäuresequenz im Sinne dieser Erfindung umfassen Aminosäure-Insertionsvarianten, Aminosäure-Deletionsvarianten und/oder Aminosäure-Substitutionsvarianten.

Aminosäure-Insertionsvarianten umfassen amino- und/oder carboxyterminale Fusionen, sowie Insertionen von einzelnen oder mehreren Aminosäuren in einer bestimmten Aminosäuresequenz. Bei Aminosäure-Sequenzvarianten mit einer Insertion werden ein oder mehrere Aminosäurereste in eine vorbestimmte Stelle in einer Aminosäuresequenz eingebracht, obwohl eine zufällige Insertion mit geeignetem Screening des resultierenden Produkts auch möglich ist. Aminosäure-Deletionsvarianten sind durch das Entfernen von einer oder mehreren Aminosäuren aus der Sequenz charakterisiert. Aminosäure-Substitutionsvarianten zeichnen sich dadurch aus, dass wenigstens ein Rest in der Sequenz entfernt und ein anderer Rest an dessen Stelle eingefügt wird. Vorzugsweise befinden sich die Modifikationen an Positionen in der Aminosäuresequenz, die zwischen homologen Proteinen oder Polypeptiden nicht konserviert sind. Vorzugsweise werden Aminosäuren durch andere mit ähnlichen Eigenschaften, wie Hydrophobizität, Hydrophilizität, Elektronegativität, Volumen der Seitenkette und ähnliches, ersetzt (konservative Substitution). Konservative Substitutionen betreffen beispielsweise den Austausch einer Aminosäure durch eine andere, wobei beide Aminosäuren in derselben nachstehenden Gruppe aufgeführt sind:
1. kleine aliphatische, nicht-polare oder leicht-polare Reste: Ala, Ser, Thr (Pro, Gly)
2. negativ geladene Reste und ihre Amide: Asn, Asp, Glu, Gln
3. positiv geladene Reste: His, Arg, Lys
4. große aliphatische, nicht-polare Reste: Met, Leu, Ile, Val (Cys)
5. große aromatische Reste: Phe, Tyr, Trp.

Drei Reste sind aufgrund ihrer besonderen Rolle für die Proteinarchitektur in Klammern gesetzt. Gly ist der einzige Rest ohne eine Seitenkette und verleiht der Kette somit Flexibilität. Pro besitzt eine ungewöhnliche Geometrie, die die Kette stark einschränkt. Cys kann eine Disulfidbrücke bilden.

Die vorstehend beschriebenen Aminosäure-Varianten können leicht mit Hilfe von bekannten Peptidsynthesetechniken wie z.B. durch "Solid Phase Synthesis" (Merrifield, 1964) und ähnliche Verfahren oder durch rekombinante DNA-Manipulation hergestellt werden. Techniken, um Substitutionsmutationen an vorbestimmten Stellen in DNA einzubringen, die eine bekannte oder teilweise bekannte Sequenz besitzt, sind gut bekannt und umfassen z.B. M13-Mutagenese. Die Manipulation von DNA-Sequenzen zur Herstellung von Proteinen mit Substitutionen, Insertionen oder Deletionen und die allgemeinen rekombinanten Verfahren zur Expression von Proteinen z.B. in einem biologischen System (wie Säuger-, Insekten-, Pflanzen- und viralen Systeme) sind z.B. in Sambrook et. al. (1989) ausführlich beschrieben.

"Derivate" von Proteinen oder Polypeptiden umfassen erfindungsgemäß auch einzelne oder multiple Substitutionen, Deletionen und/oder Additionen jeglicher Moleküle, die mit dem Protein oder Polypeptid assoziiert sind, wie Kohlenhydrate, Lipide und/oder Proteine oder Polypeptide.

In einer Ausführungsform umfassen "Derivate" von Proteinen oder Polypeptiden diejenigen modifizierten Analoga, die durch Glykosylierung, Acetylierung, Phosphorylierung, Amidierung, Palmitoylierung, Myristoylierung, Isoprenylierung, Lipidierung, Alkylierung, Derivatisierung, Einbringen von Schutz-/Blockierungsgruppen, proteolytische Spaltung oder Bindung an einen Antikörper oder an einen anderen zellulären Liganden entstehen. Derivate von Proteinen oder Polypeptiden können auch durch andere Verfahren wie beispielsweise durch chemische Spaltung mit Bromcyan, Trypsin, Chymotrypsin, Papain, V8-Protease, NaBH₂, Acetylierung, Formylierung, Oxidation, Reduktion oder durch metabolische Synthese in Gegenwart von Tunicamycin hergestellt werden.

Ferner erstreckt sich der Begriff "Derivat" auch auf alle funktionellen chemischen Äquivalente der Proteine oder Polypeptide.

Derivate eines bestimmten Proteins oder Peptids betreffen auch posttranslationell modifizierte Varianten, Isoformen und Species-Homologe des Proteins oder Peptids, insbesondere solche, die natürlicherweise exprimiert werden.

Die erfindungsgemäß beschriebenen Proteine und Peptide sind vorzugsweise isoliert. Die Begriffe "isoliertes Protein" oder "isoliertes Peptid" bedeuten, dass das Protein oder Peptid aus seiner natürlichen Umgebung isoliert wurde. Ein isoliertes Protein oder Peptid kann in einem im Wesentlichen aufgereinigten Zustand vorliegen. Der Begriff "im Wesentlichen aufgereinigt" bedeutet, dass das Protein oder Peptid im Wesentlichen frei von anderen Substanzen ist, mit denen es in der Natur oder in vivo assoziiert ist.

Erfindungsgemäß beschriebene Proteine und Peptide können von biologischen Proben wie Gewebs- oder Zellhomogenisaten isoliert werden oder können in einer Vielzahl von eukaryontischen und prokaryontischen Expressionssystemen exprimiert werden.

Vorzugsweise beträgt der Grad an Ähnlichkeit, vorzugsweise Identität zwischen einer Aminosäuresequenz, die hierin beschrieben ist, und einer Aminosäuresequenz, die von dieser Aminosäuresequenz abgeleitet ist, mindestens 70%, vorzugsweise mindestens 80%, noch mehr bevorzugt mindestens 90% oder am meisten bevorzugt mindestens 95%, 96%, 97%, 98% or 99%. Der Grad an Ähnlichkeit oder Identität ist vorzugsweise für einen Bereich von mindestens etwa 10, mindestens etwa 20, mindestens etwa 40, mindestens etwa 60, mindestens etwa 80, mindestens etwa 100, mindestens etwa 150, mindestens etwa 200, mindestens etwa 250, oder mindestens etwa 300 konsekutive Aminosäuren angegeben. In bevorzugten Ausführungsformen, ist der Grad an Identität für die gesamte Länge der Referenzaminosäuresequenz angegeben.

Bezüglich einer Identität von Aminosäuresequenzen gilt das vorstehend für die Identität von Nukleinsäuresequenzen Gesagte sinngemäß.

Ein Teil, d.h. Fragment, oder Derivat eines Proteins oder Peptids weist erfindungsgemäß vorzugsweise eine funktionelle Eigenschaft des Proteins oder Peptids auf, aus dem er/es abgeleitet ist. Solche funktionellen Eigenschaften sind vorstehend für Flt3-Ligand und Antigene erläutert und umfassen beispielsweise Immunreaktivität, insbesondere die Interaktion mit Antikörpern oder die Interaktion mit anderen Peptiden oder Proteinen. Eine bedeutende Eigenschaft ist die Fähigkeit, einen Komplex mit MHC-Molekülen bzw. Flt3-Rezeptoren einzugehen und gegebenenfalls eine Immunreaktion beispielsweise durch Stimulation oder Hemmung von cytotoxischen oder Helfer T-Zellen zu erzeugen oder zu verhindern bzw. eine zelluläre Reaktion auszulösen. Ein Teil eines Proteins oder Peptids umfasst vorzugsweise eine Sequenz von mindestens 6, mindestens 8, mindestens 10, mindestens 12, mindestens 15, mindestens 20, mindestens 30 und vorzugsweise bis zu 8, bis zu 10, bis zu 12, bis zu 15, bis zu 20, bis zu 30 oder bis zu 50 aufeinander folgenden Aminosäuren aus dem Protein oder Peptid. In einer Ausführungsform betrifft ein Teil eines Proteins oder Peptids erfindungsgemäß ein oder mehrere Epitope aus dem vollständigen Peptid oder Protein, wobei die mehreren Epitope in ihrer natürlichen Verbindung vorliegen können oder eine künstliche, d.h. nicht natürlich vorkommende Verbindung aufweisen können, d.h. die Epitope können beispielsweise durch einen künstlichen Linker voneinander getrennt sein. Vorzugsweise betrifft ein Teil eines Proteins oder Peptids erfindungsgemäß eine solche Sequenz, die ein Ziel, insbesondere ein Epitop, für eine Immunreaktion in einem Patienten ist. In bevorzugten Ausführungsformen ist die Sequenz Ziel für eine Antikörper- und/oder T-Zell-vermittelte Immunreaktion. Ein erfindungsgemäß verwendetes Peptid, Protein oder Derivat kann auch mehrere solcher Sequenzen umfassen, die Epitope für Antikörper oder T-Zellen darstellen.

Ein Teil, d.h. Fragment, einer Nukleinsäure, die für ein Protein oder Peptid kodiert, betrifft erfindungsgemäß vorzugsweise den Teil der Nukleinsäure, der zumindest für das Protein oder Peptid und/oder für einen Teil des Proteins oder Peptids wie vorstehend definiert kodiert. Ein Teil einer Nukleinsäure, die für ein Protein oder Peptid kodiert, betrifft vorzugsweise den Teil der Nukleinsäure, der dem offenen Leserahmen entspricht.

Die erfindungsgemäß beschriebenen pharmazeutischen Präparate und Zusammensetzungen können therapeutisch für die Behandlung einer bereits bestehenden Erkrankung oder präventiv/prophylaktisch als Vakzinen für die Immunisierung eingesetzt werden, um die hier beschriebenen Erkrankungen zu verhindern.

Tiermodelle können für ein Testen einer immunisierenden Wirkung z.B. gegenüber Krebs bei Verwendung eines Tumor-assoziierten Antigens als Antigen eingesetzt werden. Dabei können beispielsweise menschliche Krebszellen in eine Maus für die Schaffung eines Tumors eingebracht werden und ein erfindungsgemäßes Präparat oder eine erfindungsgemäße Zusammensetzung, umfassend eine für ein Tumor-assoziiertes Antigen kodierende RNA, kann verabreicht werden. Die Wirkung auf die Krebszellen (beispielsweise Verringerung der Tumorgröße) kann als Maß für die Wirksamkeit einer Immunisierung gemessen werden.

Als Teil der Zusammensetzung für eine Immunisierung können ein oder mehrere Vakzine-RNAs mit einem oder mehreren Adjuvanzien für eine Induktion einer Immunreaktion oder eine Erhöhung einer Immunreaktion verabreicht werden.

Andere Stoffe, die eine Immunreaktion des Patienten stimulieren, können auch verabreicht werden. Zum Beispiel sind Zytokine bei einer Vakzinierung aufgrund ihrer regulatorischen Eigenschaften auf Lymphozyten verwendbar. Solche Zytokine umfassen z.B. Interleukin-12 (IL-12), von dem gezeigt wurde, dass es die schützenden Wirkungen von Vakzinen verstärkt (vgl. Science 268:1432-1434, 1995), GM-CSF und IL-18.

Das erfindungsgemäße Vorgehen zur Induktion einer Immunreaktion in einem Säuger umfasst im Allgemeinen die Verabreichung einer Menge einer Vakzine-RNA, die zusammen mit der Verabreichung von Flt3-Ligand eine Immunreaktion, die vorzugsweise prophylaktisch und/oder therapeutisch ist, auslöst.

Der Begriff "Transfektion" betrifft erfindungsgemäß das Einbringen einer oder mehrerer Nukleinsäuren in einen Organismus oder in eine Wirtszelle. Verschiedene Verfahren können eingesetzt werden, um erfindungsgemäß Nukleinsäuren in Zellen in vitro oder in vivo einzubringen. Solche Verfahren umfassen die Transfektion von Nukleinsäure-CaPO₄-Präzipitaten, die Transfektion von Nukleinsäuren, die mit DEAE assoziiert sind, die Transfektion oder Infektion mit Viren, die die interessierenden Nukleinsäuren tragen, die Liposomen-vermittelte Transfektion und ähnliches. In bestimmten Ausführungsformen ist eine Steuerung der Nukleinsäure an bestimmte Zellen bevorzugt. In solchen Ausführungsformen kann ein Träger, der für die Verabreichung einer Nukleinsäure an eine Zelle (z.B. ein Retrovirus oder ein Liposom) eingesetzt wird, ein gebundenes Zielsteuerungsmolekül aufweisen. Zum Beispiel kann ein Molekül wie ein Antikörper, der für ein Oberflächenmembran-Protein auf der Zielzelle spezifisch ist, oder ein Ligand für einen Rezeptor auf der Zielzelle in den Nukleinsäureträger eingebaut oder daran gebunden werden. Falls eine Verabreichung einer Nukleinsäure durch Liposomen erwünscht ist, können Proteine, die an ein Oberflächenmembran-Protein binden, das mit der Endozytose assoziiert ist, in die Liposomenformulierung eingebaut werden, um eine Zielsteuerung und/oder Aufnahme zu ermöglichen. Solche Proteine umfassen Kapsid-Proteine oder Fragmente davon, die für einen bestimmten Zelltyp spezifisch sind, Antikörper gegen Proteine, die intemalisiert werden, Proteine, die eine intrazelluläre Stelle ansteuern, und ähnliches.

Erfindungsgemäß kann eine Verabreichung von Nukleinsäuren entweder als nackte Nukleinsäure oder in Verbindung mit einem Verabreichungsreagens erfolgen. Beispielsweise ist erfindungsgemäß auch eine Verabreichung von Nukleinsäuren in vivo mittels Zielgesteuerter Liposomen vorgesehen.

Für eine Verabreichung von Nukleinsäuren können Vektoren, die von Adenovirus (AV), Adeno-assoziiertem Virus (AAV), Retroviren (wie Lentiviren (LV), Rhabdoviren, murinem Leukämievirus), oder Herpesvirus abgeleitet sind, und dergleichen eingesetzt werden. Der Tropismus der viralen Vektoren kann durch Pseudotypisierung der Vektoren mit Hüllproteinen oder anderen Oberflächen-Antigenen von anderen Viren oder durch Substitution verschiedenen viraler Kapsidproteine wie geeignet modifiziert werden.

Liposomen können die Zufuhr der Nukleinsäure an ein bestimmtes Gewebe unterstützen und können auch die Halbwertszeit der Nukleinsäure erhöhen. Liposomen, die erfindungsgemäß geeignet sind, werden aus Standard-Vesikel-bildenden Lipiden, die im Allgemeinen neutrale oder negativ geladene Phospholipide einschließen, und einem Sterin wie Cholesterin gebildet. Die Auswahl von Lipiden wird im Allgemeinen durch Faktoren wie die gewünschte Liposomengröße und die Halbwertszeit der Liposomen bestimmt. Eine Vielzahl von Verfahren zur Herstellung von Liposomen ist bekannt; vgl. z.B. Szoka et al. (1980), Ann. Rev. Biophys. Bioeng. 9: 467; und US-PSen 4,235,871, 4,501,728, 4,837,028 und 5,019,369.

In bestimmten Ausführungsformen ist eine Steuerung der Nukleinsäure an bestimmte Zellen bevorzugt. In solchen Ausführungsformen kann ein Träger, der für die Verabreichung einer Nukleinsäure an eine Zelle (z.B. ein Retrovirus oder ein Liposom) eingesetzt wird, ein gebundenes Zielsteuerungsmolekül aufweisen. Zum Beispiel kann ein Molekül wie ein Antikörper, der für ein Oberflächenmembran-Protein auf der Zielzelle spezifisch ist, oder ein Ligand für einen Rezeptor auf der Zielzelle in den Nukleinsäureträger eingebaut oder daran gebunden werden. Falls eine Verabreichung einer Nukleinsäure durch Liposomen erwünscht ist, können Proteine, die an ein Oberflächenmembran-Protein binden, das mit der Endocytose assoziiert ist, in die Liposomenformulierung eingebaut werden, um eine Zielsteuerung und/oder Aufnahme zu ermöglichen. Solche Proteine umfassen Kapsid-Proteine oder Fragmente davon, die für einen bestimmten Zelltyp spezifisch sind, Antikörper gegen Proteine, die internalisiert werden, Proteine, die eine intrazelluläre Stelle ansteuern, und ähnliches.

Vorzugsweise wird RNA zusammen mit stabilisierenden Substanzen wie RNase-Inhibitoren verabreicht.

Eine Verabreichung von Polypeptiden und Peptiden kann in an sich bekannter Weise erfolgen.

Der Begriff "Patient", "Individuum" oder "Lebewesen" betrifft Säuger. Beispielsweise sind Säuger, die erfindungsgemäß vorgesehen sind, Menschen, Primaten, Haustiere wie Hunde, Katzen usw., domestizierte Tiere wie Schafe, Rinder, Ziegen, Schweine, Pferde und dergleichen, Labortiere wie Mäuse, Ratten, Kaninchen, Meerschweinchen usw., sowie in Gefangenschaft gehaltene Tiere wie Zootiere. Der Begriff "Tier" umfasst wie hierin verwendet den Menschen.

Begriffe wie "erhöhten", "steigern" oder "verstärken" betreffen vorzugsweise eine Erhöhung, Steigerung oder Verstärkung um mindestens 10%, insbesondere mindestens 20%, mindestens 50% oder mindestens 100% bzw. von einem nicht vorhandenen und/oder nicht nachweisbaren Zustand in einen vorhandenen und/oder nachweisbaren Zustand.

Die Begriffe "T-Zelle" und "T-Lymphocyt" werden hier austauschbar verwendet und umfassen T-Helferzellen und cytolytische T-Zellen wie cytotoxische T-Zellen.

"Verringern" oder "hemmen" betrifft hier die Fähigkeit, eine Abnahme zu bewirken, wie eine Abnahme um 20% oder mehr, mehr bevorzugt von 50% oder mehr, und am meisten bevorzugt von 75% oder mehr.

Immunisierungsprotokolle unter Verwendung von Flt3-Ligand betreffen die Verabreichung von Flt3-Ligand und RNA, entweder im Gemisch oder getrennt voneinander, gegebenenfalls in Kombination mit einem oder mehreren Hilfsstoffen sowie anderen begleitenden Molekülen und/oder Formulierungen (wie Verdünnungsmittel, Träger, Exzipienzien und dergleichen) an ein Lebewesen für die Vermeidung und/oder Behandlung einer Erkrankung oder Infektion. Der Flt3-Ligand und die RNA und jegliche andere Bestandteile, die hierin beschrieben sind, können in jeglicher Dosis, Reihenfolge, Häufigkeit und temporären Anordnungen verabreicht werden. Der Fachmann wird erkennen, dass eine Veränderung dieser Parameter für eine Optimierung einer Behandlung durch den Fachmann routinemäßig erfolgen kann.

Die erfindungsgemäßen pharmazeutischen Zusammensetzungen, die Vakzine-RNA, Flt3-Ligand oder beides enthalten, werden vorzugsweise in pharmazeutisch verträglichen Zubereitungen verabreicht. Solche Zubereitungen können gewöhnlich pharmazeutisch verträgliche Konzentrationen von Salzen, Pufferstoffen, Konservierungsstoffen, Trägern, ergänzenden immunitätssteigernden Stoffen wie Adjuvanzien (z.B. CpG-Oligonukleotide) und Zytokine und gegebenenfalls therapeutische Wirkstoffe enthalten.

Die erfindungsgemäßen pharmazeutischen Zusammensetzungen können auf jedem herkömmlichen Weg verabreicht werden, einschließlich durch Injektion oder durch Infusion. Die Verabreichung kann beispielsweise oral, intravenös, intraperitoneal, intramuskulär, subkutan, intrakutan, transdermal, intralymphatisch, vorzugsweise durch Injektion in Lymphknoten, insbesondere Leistenlymphknoten, Lymphgefäße und/oder in die Milz, erfolgen.

Die Verabreichung von RNA und Flt3-Ligand kann getrennt voneinander, d.h. in verschiedenen Zusammensetzungen, oder in einer gemeinsamen Zusammensetzung erfolgen. Falls die Verabreichung getrennt voneinander erfolgt, kann die Verabreichung von RNA und Flt3-Ligand zeitgleich oder zu verschiedenen Zeitpunkten erfolgen, wobei die Verabreichung von RNA und/oder Flt3-Ligand mehrfach erfolgen kann. Erfolgt die Verabreichung von RNA und Flt3-Ligand zu verschiedenen Zeitpunkten, kann das zeitliche Intervall zwischen den Verabreichungen bzw. bei mehrfacher Verabreichung zwischen der letzten Verabreichung von RNA bzw. Flt3-Ligand und der ersten Verabreichung des jeweilig verbleibenden Bestandteils 6 Stunden oder mehr, 12 Stunden oder mehr, 24 Stunden oder mehr, 2 Tage oder mehr, 3 Tage oder mehr, 5 Tage oder mehr, 7 Tage oder mehr oder 9 Tage oder mehr betragen. Vorzugsweise beträgt das zeitliche Intervall zwischen den Verabreichungen nicht mehr als 24 Stunden, nicht mehr als 2 Tage, nicht mehr als 4 Tage, nicht mehr als 8 Tage oder nicht mehr als 10 Tage. Vorzugsweise wird der Flt3-Ligand vor einer Verabreichung von RNA verabreicht. Falls die Verabreichung von RNA und Flt3-Ligand getrennt voneinander erfolgt, erfolgt die Verabreichung von RNA vorzugsweise intralymphatisch, mehr bevorzugt intranodal, und die Verabreichung von Flt3-Ligand erfolgt vorzugsweise intravenös, intraperitoneal, intramuskulär, subkutan, intrakutan oder transdermal, vorzugsweise intraperitoneal oder subkutan.

Die erfindungsgemäßen Zusammensetzungen werden in wirksamen Mengen verabreicht. Eine "wirksame Menge" betrifft die Menge, die alleine oder zusammen mit weiteren Dosen eine gewünschte Reaktion oder eine gewünschte Wirkung erzielt. Im Fall einer Behandlung einer bestimmten Erkrankung oder eines bestimmten Zustands betrifft die gewünschte Reaktion die Hemmung des Krankheitsverlaufs. Dies umfasst die Verlangsamung des Fortschreitens der Erkrankung und insbesondere eine Unterbrechung des Fortschreitens der Erkrankung. Die gewünschte Reaktion bei einer Behandlung einer Erkrankung oder eines Zustands kann auch die Verzögerung des Ausbruchs oder eine Verhinderung des Ausbruchs der Erkrankung oder des Zustands sein.

Eine wirksame Menge einer erfindungsgemäßen Zusammensetzung wird von dem zu behandelnden Zustand, der Schwere der Krankheit, den individuellen Parametern des Patienten, einschließlich Alter, physiologischer Zustand, Größe und Gewicht, der Dauer der Behandlung, der Art einer begleitenden Therapie (falls vorhanden), dem spezifischen Verabreichungsweg und ähnlichen Faktoren abhängen.

Die erfindungsgemäßen pharmazeutischen Zusammensetzungen sind vorzugsweise steril und enthalten eine wirksame Menge der wirksamen Substanz für die Erzeugung der gewünschten Reaktion oder der gewünschten Wirkung.

Die Dosen der erfindungsgemäßen Zusammensetzungen, die verabreicht werden, können von verschiedenen Parametern wie der Verabreichungsart, dem Zustand des Patienten, dem gewünschten Verabreichungszeitraum, usw. abhängen. Für den Fall, dass eine Reaktion bei einem Patienten bei einer anfänglichen Dosis unzureichend ist, können höhere Dosen (oder effektiv höhere Dosen, die durch einen anderen, stärker lokalisierten Verabreichungsweg erzielt werden) eingesetzt werden.

Im Allgemeinen werden für eine Behandlung oder für eine Erzeugung oder Erhöhung einer Immunreaktion vorzugsweise Dosen der RNA von 1 ng bis 700 µg, 1 ng bis 500 µg, 1 ng bis 300 µg, 1 ng bis 200 µg, oder 1 ng bis 100 µg formuliert und verabreicht.

Die erfindungsgemäßen pharmazeutischen Zusammensetzungen werden im Allgemeinen in pharmazeutisch verträglichen Mengen und in pharmazeutisch verträglichen Zusammensetzungen verabreicht. Solche Zusammensetzungen können gewöhnlich Salze, Pufferstoffe, Konservierungsstoffe, Träger und gegebenenfalls therapeutische Wirkstoffe enthalten. Bei einer Verwendung in der Medizin sollten die Salze pharmazeutisch verträglich sein. Nicht-pharmazeutisch verträgliche Salze können jedoch für die Herstellung pharmazeutisch verträglicher Salze davon verwendet werden und sind erfindungsgemäß umfasst. Solche pharmakologisch und pharmazeutisch verträglichen Salze umfassen in nicht begrenzender Weise diejenigen, die aus den nachstehenden Säuren hergestellt werden: Chlorwasserstoff-, Bromwasserstoff-, Schwefel-, Salpeter-, Phosphor-, Malein-, Essig-, Salicyl-, Citronen-, Ameisen-, Malon-, Bernsteinsäure und ähnliches. Pharmazeutisch verträgliche Salze können auch als Alkalimetall- oder Erdalkalimetallsalze wie Natrium-, Kalium- oder Calciumsalze hergestellt werden.

Eine erfindungsgemäße pharmazeutische Zusammensetzung kann einen pharmazeutisch verträglichen Träger umfassen. Der Begriff "pharmazeutisch verträglicher Träger" betrifft erfindungsgemäß einen oder mehrere kompatible feste oder flüssige Füllstoffe, Verdünnungsmittel oder Kapselsubstanzen, die für eine Verabreichung an einen Menschen geeignet sind. Der Begriff "Träger" betrifft einen organischen oder anorganischen Bestandteil, natürlicher oder synthetischer Natur, in dem der aktive Bestandteil kombiniert wird, um eine Anwendung zu erleichtern. Die Bestandteile der erfindungsgemäßen pharmazeutischen Zusammensetzung sind gewöhnlich derart, dass keine Interaktion auftritt, die die gewünschte pharmazeutische Wirksamkeit wesentlich beeinträchtigt.

Vorzugsweise sind die Trägerstoffe sterile Flüssigkeiten wie Wasser oder Öle, einschließlich derjenigen, die sich von Erdöl, Tieren oder Pflanzen ableiten oder synthetischen Ursprungs sind, wie z.B. Erdnussöl, Sojabohnenöl, Mineralöl, Sesamöl, Sonnenblumenöl und dergleichen. Salzlösungen und wässrige Dextrose- und Glycerinlösungen können auch als wässrige Trägerstoffe verwendet werden.

Beispiele für Hilfs- und Trägerstoffe sind Acryl- und Methacrylderivate, Alginsäure, Sorbinsäurederivate wie α-Octadecyl-ω-hydroxypoly(oxyethylen)-5-sorbinsäure, Aminosäuren und deren Derivate, insbesondere Aminverbindungen wie Cholin, Lecithin und Phosphatidylcholin, Gummi arabicum, Aromastoffe, Ascorbinsäure, Carbonate wie beispielsweise Natrium-, Kalium-, Magnesium- und Calciumcarbonat und -hydrogencarbonat, Hydrogenphosphate und Phosphate von Natrium, Kalium, Calcium und Magnesium, Carmellosenatrium, Dimeticon, Farbstoffe, Geschmacksstoffe, Puffersubstanzen, Konservierungsmittel, Verdickungsmittel, Weichmacher, Gelatine, Glucosesirupe, Malz, hochdisperses Siliziumdioxid, Hydromellose, Benzoate, insbesondere Natrium- und Kaliumbenzoat, Macrogol, Magermilchpulver, Magnesiumoxid, Fettsäuren und deren Derivate und Salze wie Stearinsäure und Stearate, insbesondere Magnesium- und Calciumstearat, Fettsäureester sowie Mono- und Diglyceride von Speisefettsäuren, natürliche und künstliche Wachse wie Bienenwachs, gelbes Wachs und Montanglycolwachs, Chloride, insbesondere Natriumchlorid, Polyvidon, Polyethylenglykole, Polyvinylpyrrolidon, Povidon, Öle wie Rizinusöl, Sojaöl, Kokosnussöl, Palmkernöl, Zucker und Zuckerderivate, insbesondere Mono- und Disaccharide wie Glucose, Fructose, Mannose, Galactose, Lactose, Maltose, Xylose, Saccharose, Dextrose und Cellulose und deren Derivate, Schellack, Stärke und Stärkederivate, insbesondere Maisstärke, Talg, Talkum, Titandioxid, Weinsäure, Zuckeralkohole wie Glycerin, Mannit, Sorbit und Xylit und deren Derivate, Glykol, Ethanol und Gemische derselben.

Vorzugsweise können die pharmazeutischen Zusammensetzungen zusätzlich auch Benetzungsmittel, Emulgatoren und/oder pH-puffernde Mittel enthalten.

In einer weiteren Ausführungsform können die pharmazeutischen Zusammensetzungen einen Resorptionsverstärker enthalten. Diese Resorptionsverstärker können, falls gewünscht, eine äquimolare Menge des Trägerstoffs in der Zusammensetzung ersetzen. Beispiele für solche Resorptionsverstärker umfassen in nicht begrenzender Weise Eucalyptol, N,N-Diethyl-m-toluamid, Polyoxyalkylenalkohole (wie Propylenglykol und Polyethylenglykol), N-Methyl-2-pyrrolidon, Isopropylmyristat, Dimethylformamid (DMF), Dimethylsulfoxid (DMSO), Dimethylacetamid (DMA), Harnstoff, Diethanolamin, Triethanolamin und dergleichen (siehe z.B. Percutaneous Penetration Enhancers, Hrsg. Smith et al. (CRC Press, 1995)). Die Menge an Resorptionsverstärker in der Zusammensetzung kann von den gewünschten zu erreichenden Wirkungen abhängen.

Ein Protease-Inhibitor kann in die erfindungsgemäße Zusammensetzung, insbesondere die Flt3-Ligand enthaltende Zusammensetzung, eingebaut werden, um einen Abbau eines Peptid-oder Proteinwirkstoffs zu vermeiden und dadurch die Bioverfügbarkeit zu erhöhen. Beispiele für Protease-Inhibitoren umfassen in nicht-begrenzender Weise Aprotinin, Leupepsin, Pepstatin, α2-Makroglobulin und Trypsin-Inhibitor. Diese Inhibitoren können alleine oder in Kombination verwendet werden.

Die erfindungsgemäßen pharmazeutischen Zusammensetzungen können mit einer oder mehreren Beschichtungen versehen sein. Vorzugsweise sind die festen oralen Darreichungsformen mit einer magensaftresistenten Beschichtung versehen oder liegen in Form einer magensaftresistenten, gehärteten Weichgelatinekapsel vor.

Die erfindungsgemäßen pharmazeutischen Zusammensetzungen können geeignete Pufferstoffe wie Essigsäure in einem Salz, Citronensäure in einem Salz, Borsäure in einem Salz und Phosphorsäure in einem Salz enthalten.

Die pharmazeutischen Zusammensetzungen können auch gegebenenfalls geeignete Konservierungsstoffe wie Benzalkoniumchlorid, Chlorbutanol, Parabene und Thimerosal enthalten.

Die pharmazeutischen Zusammensetzungen werden gewöhnlich in einer einheitlichen Dosisform dargeboten und können in an sich bekannter Weise hergestellt werden. Erfindungsgemäße pharmazeutische Zusammensetzungen können beispielsweise in Form von Kapseln, Tabletten, Lutschpastillen, Lösungen, Suspensionen, Sirupen, Elixieren oder als Emulsion vorliegen.

Zusammensetzungen, die für eine parenterale Verabreichung geeignet sind, umfassen gewöhnlich eine sterile wässrige oder nicht-wässrige Zubereitung, die vorzugsweise mit dem Blut des Empfängers isotonisch ist. Verträgliche Träger und Lösungsmittel sind beispielsweise Ringer-Lösung und isotonische Natriumchloridlösung. Zusätzlich werden gewöhnlich sterile, fixierte Öle als Lösungs- oder Suspensionsmedium eingesetzt.

Die vorliegende Erfindung wird durch die nachstehenden Beispiele und Figuren ausführlich beschrieben, die ausschließlich der Erläuterung dienen und nicht begrenzend zu verstehen sind. Dem Fachmann sind aufgrund der Beschreibung und der Beispiele weitere Ausführungsformen zugänglich, die nicht über den Rahmen der Erfindung und den Umfang der anhängenden Ansprüche hinausgehen.

### Kurze Beschreibung der Zeichnungen:

### Abb. 1:

C57B1/6 Mäusen (n = 3 - 9) wurde 10 µg Flt3L intraperitoneal zu verschiedenen Zeitpunkten (d1 bis d3 oder d1, d1, d3 oder d0, d3) appliziert. Am Tag 10 wurden die Lymphknoten (LK) sowie die Milz präpariert und die Zellzahl bestimmt. Die dargestellten Daten repräsentieren die mittlere Zellzahl +SEM der Lymphknoten. *:p < 0,05 in Tukey's multiple comparison test.

### Abb. 2:

C57B1/6 Mäusen (n = 3) wurde 10 µg Flt3L zweimal (d0, d3) intraperitoneal appliziert. Am Tag 10 wurden die inguinalen Lymphknoten präpariert, die Zellen mit entsprechenden Antikörpern gefärbt und die Subpopulationen der Dendritischen Zellen durchflußzytometrisch quantifiziert. Die dargestellten Daten repräsentieren die mittlere Zellzahl der Subpopulation +SEM. *:p < 0,05 und **:p< 0,001 im zweisetigen ungepaarten t-Test.

### Abb. 3:

Anästhesierten C57B1/6 Mäusen (n = 5) wurden zweimal (d0, d3) jeweils 20 µg SIINFEKL kodierende RNA in den inguinalen Lymphknoten appliziert. Den Mäusen wurden verschiedene Adjuvantien appliziert (MPLA d0 + d3, 20 µg s.c.; Poly I:C d0 + d3, 20 µg s.c.; Aldara Creme d0 + d3, 5 µg transcutan; GM-CSF -d2, -d1, d1, d2, 5 µg s.c; IL-2 (Proleukin) d1 - d6, 80000 IU s.c.; Flt3-L d-7 + d-4, 10 µg i.p.). Am Tag 8 wurde den Mäusen Blut entnommen und die Frequenz der Epitop-spezifischen T-Lymphozyten wurde nach Färbung mit einem SIINFEKL-Tetramer und anti-CD8-Antikörper durchflußzytometrisch quantifiziert. Die dargestellten Daten repräsentieren die mittlere Frequenz Tetramer-positiver CD8⁺ T-Lymphozyten +SEM aus 2 Experimenten. *:p < 0,05 und **:p < 0,001 in Tukey's multiple comparison test.

### Abb. 4:

C57B1/6 Mäusen (n = 4) wurde 10 µg Flt3L oder humanes IgG4 zweimal (d0, d3) intraperitoneal appliziert. Am Tag 7 und 10 wurden den anästhesierten Mäusen jeweils 20 µg SIINFEKL-kodierende RNA in den inguinalen Lymphknoten appliziert. Am Tag 15 wurde die Milz und der inguinale Lymphknoten präpariert.
(a) Die Frequenz der epitopspezifischen T-Lymphozyten wurde nach Färbung mit einem SIINFEKL-Tetramer und anti-CD8-Antikörper durchflußzytometrisch quantifiziert. Die dargestellten Daten repräsentieren die mittlere Anzahl und mittlere Frequenz Tetramer-positiver CD8⁺ T-Lymphozyten +SEM.
(b) Zur Messung der IFNγ-produzierenden SIINFEKL-spezifischen T-Lymphozyten wurden Milzzellen mit SIINFEKL-Peptid oder Kontrollpeptid für 6 h inkubiert. Nach 1,5 h wurde Brefeldin A zugegeben [10 µg/ml]. Die Proben wurden nach Fixierung und Permeabilisierung mit anti-CD8- und anti-IFNγ-Antikörpern gefärbt. Die gezeigten Daten repräsentieren die Frequenz SIINFEKL-spezifisch IFNγ-sekretierender CD8⁺ T-Lymphozyten nach Substraktion des unspezifischen Hintergrundes + SEM.*: p < 0,05 im zweisetigen ungepaarten t-Test.
(c) Repräsentative Dot-Plots. Die angegeben Prozente geben die jeweilige Frequenz Tetramer-positiver CD8⁺ T-Lymphozyten an.

### Abb. 5:

(a) Balb/c Mäusen (n = 5) wurde entweder 10 µg Cy3-Fluorophor markierte RNA (rot) oder reines Cy3-Ribonukleotid (control) intranodal injiziert. Nach 5 und 30 min. wurden jeweils Lymphknoten präpariert und mit Paraformaldehyd fixiert und geschnitten. Während Kontroll-Lymphknoten einen minimalen Hintergrund zeigen, ist ansonsten ein zelluläres RNA-Signal zu erkennen, welches von 5 Minuten zu 30 Minuten an Klarheit gewinnt. Dies ist auf die Zerstörung von interzellulär vorliegender RNA zurückzuführen.
(b) Humane immature DCs (iDCs) wurden in vitro mit Cy3-Fluorophor markierter RNA (5 µg, Rot) und FITC-Dextran (1 µg/µl, Grün) für 10 min. koinkubiert, mit Paraformaldehyd fixiert und gegengefärbt (Hoechst 33342, Blau). Die Zeitkinetik zeigt wie bei maximaler Kolokalisation mit FITC-Dextran die RNA zu Beginn in der Peripherie der Zelle lokalisiert ist, die Vesikel dann im gesamten Zytoplasma zu sehen sind und schließlich in größeren Strukturen konfluieren.

### Abb. 6:

(a) Humane iDCs (n = 3) wurden in vitro bei verschiedenen Temperaturen mit Luciferase-RNA (20 µg) für 15 min. koinkubiert. Nach 24 h wurde das Luziferase-Signal über einen Standard-Lumineszenztest quantifiziert. Das Ergebnis weist auf einen aktiven energieverbrauchenden Prozeß hin.
(b-c) Humane iDCs wurden mit verschieden Inhibitoren (Dimethylamilorid, Cytochalasin D, LY294002, Rottlerin) vorbehandelt und dann für 15 min. mit Luciferase-RNA oder Cy3-RNA koinkubiert. Nach 24 h wurde das Luziferase-Signal über einen Standard-Lumineszenztest quantifiziert. Es zeigte sich, dass mit dem hochspezifischen Makropinozytoseinhibitor Rottlerin eine Inhibition der RNA Aufnahme bis über 90 % auftritt.
(d) Die inguinalen Lymphknoten von C57B1/6 Mäusen wurden in vivo mit Rottlerin (10 µl [10 µM]) vorbehandelt und dann Luciferase-RNA (10 µg) intranodal injiziert. Nach in vivo Inhibition der Makropinozytose ist die RNA-Aufnahme im Lymphknoten drastisch vermindert.
(e) C57B1/6 Mäuse (n = 3) wurden am d0 und d3 mit SIINFEKL-kodierender RNA (20 µg) intranodal immunisiert. An beiden Tagen wurden die Lymphknoten wie oben beschrieben mit Rottlerin vorbehandelt. Am Tag 8 wurde der Erfolg der Immunisierung über eine Tetramermessung im peripheren Blut quantifiziert. Der Erfolg einer intranodalen RNA-Immunisierung korreliert direkt mit der Fähigkeit von Zellen, RNA über Makropinozytose aufzunehmen.
*, P<0.05; **, P<0.01; ***, P<0.001; (ANOVA mit Tukey's multiple comparison test).

### Abb. 7:

(a-d) Humane (a, c) und murine (b, d) DCs wurden mit verschiedenen Agentien (Poly I:C (50 µg/ml), CD40L (1.0 µg/ml), LPS (20 ng/ml), Mat. Mix (TNFalpha (10 ng/ml), IL1b (10 ng/ml), PGE (1 µg/ml), IL6 (1000 U/ml)) für 40 Stunden maturiert. Danach wurden die Zellen für 15 min. mit Luciferase-RNA oder Cy3-RNA koinkubiert. Nach 24 h wurde das Luziferase-Signal über einen Standard-Lumineszenztest quantifiziert. Für die Quantifizierung der Aufnahme von Cy3-RNA wurden die Zellen eine halbe Stunde nach Inkubation mit der RNA gewaschen und fixiert. Nachfolgend konnte die Cy3-vermittelte Fluoreszenz am Immunfluoreszenzmikroskop quantifiziert werden (Till Vision Software 4.0, Till Photonics). Nach Maturation der iDCs ist die RNA-Aufnahme um mehr als 90 % reduziert.
(e) Effekt von Poly I:C auf die RNA-Aufnahme. C57B1/6 Mäusen (n = 4) wurde PBS oder Poly I:C (20 µg) s.c. injiziert und nach 2 oder 24 h Luciferase-RNA intranodal appliziert. Nach 24 h wurde das Luziferase-Signal über einen Standard Biolumineszenztest quantifiziert. In Abhängigkeit vom Zeitintervall kommt es nach Adjuvanzgabe zu einer starken Reduktion der RNA Aufnahme.
   *, P<0.05; **, P<0.01; ***, P<0.001; (ANOVA mit Tukey's multiple comparison test).
(f) Effekt von Flt3-L auf die RNA-Aufnahme. C57BL/6 Mäuse (n = 8) wurden am Tag 0 und 3 mit 10 µg Flt3-L i.p. behandelt oder in der Kontrollgruppe nicht behandelt. Am Tag 10 wurde den Mäusen 20 µg Luziferase-RNA intranodal injiziert. 24 h später erfolgte die Messung des Luziferasesignals per in vivo Biolumineszenz. Flt3-L-Gabe wirkt sich nicht hemmend auf die RNA Aufnahme im Lymphknoten aus.

### Abb. 8:

C57BL/6 Mäusen (n = 5) wurde am Tag 0 Flt3L-IgG4, Flt3L (Humanzyme), Flt3L (Peprotech) oder humanes IgG4 in einer Menge von 0,4 mol intraperitoneal injiziert. Am Tag 10 wurden die Lymphknoten der Mäuse präpariert und durchflußzytometrisch charakterisiert. Dendritische Zellen (DCs (Marker: CD1 1c⁺/NK1.1⁻)), CD4⁺ Helfer T-Zellen (Marker: CD3⁺/CD4⁺/CD8⁻/NK1.1⁻), CD8⁺ T-Zellen (Marker: CD3⁺/CD8⁺/CD4⁻/NK1.1⁻), CD19⁺ B-Zellen (Marker: CD19⁺/CD3⁻/NK1.1⁻).

### Abb. 9:

Naiven C57BL/6 Mäusen (n = 7) wurde am Tag 0, +3 Flt3L (Flt3L-IgG4, Flt3L (Humanzyme), Flt3L (Peprotech)) oder humanes IgG4 in einer Menge von 0,4 mol intraperitoneal injiziert. Diese Mäuse wurden am Tag +7, +10 mit 20 µg SIINFEKL kodierender RNA intralymphatisch immunisiert. Die Kontrollgruppe wurde nicht behandelt (n = 2). Am Tag +15 wurde aus dem peripheren Blut mittels MHC-Multimermessung die Frequenz der antigenspezifischen CD8⁺ T-Lymphozyten gemessen.

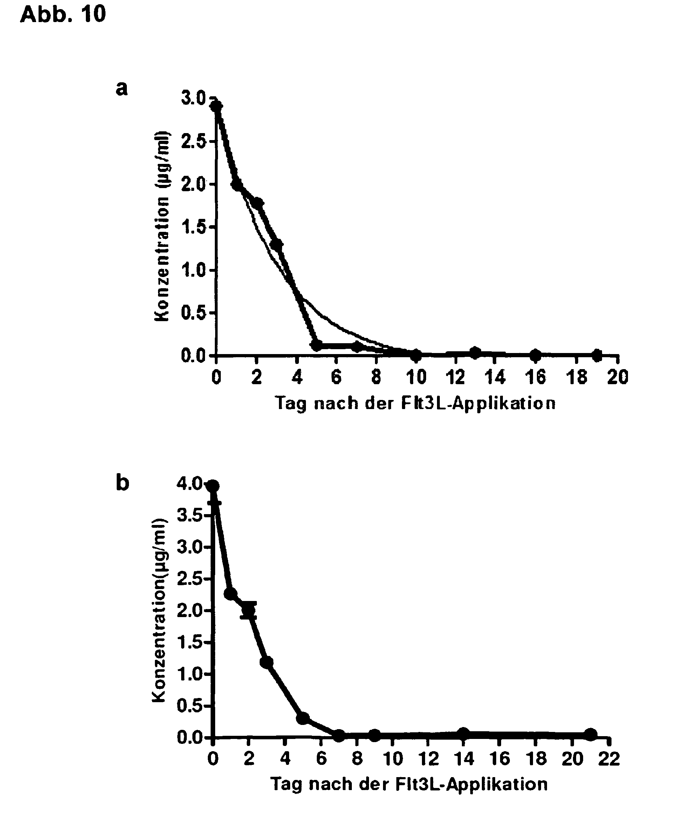

### Abb. 10: Zeitkinetik von Flt3L-IgG4 im Serum von Mäusen.

(a) Balb/c Mäusen (n = 3) wurde 20 µg Flt3L-IgG4 i.p. verabreicht. Zu definierten Zeitpunkten (vor Gabe; 3 h, 24 h, 48 h, 3 d, 5 d, 7 d, 9 d, 14 d, 21 d) wurden Serumproben der Mäuse asserviert. Diese Proben wurden in einem ELISA-Test zur Quantifizierung von humanem IgG eingesetzt. Die Halbwertszeit beträgt 2,14 Tage (= 51 Std.).
(b) Balb/c Mäusen (n = 3) wurde 50 µg Flt3L-IgG4 i.p. verabreicht. Zu definierten Zeitpunkten (vor Gabe; 3h, 24h, 48h, 3d, 5d, 7d, 9d, 14d, 21d) wurden Serumproben der Mäuse asserviert. Diese Proben wurden in einem ELISA-Test zur Quantifizierung von humanem IgG eingesetzt. Die Halbwertszeit beträgt 1,667 Tage (= 40 Std.).

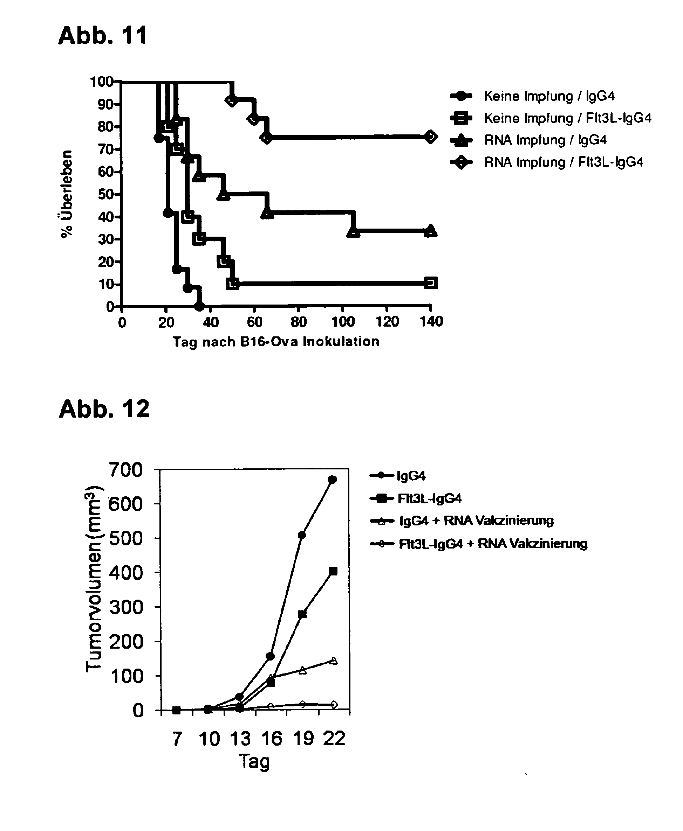

### Abb. 11: Therapeutische Vakzinierung gegen B16 Ova Tumoren.

Um die Synergie der Kombination von Flt3L-Gabe mit RNA Vakzination zu untersuchen wurde ein therapeutisches Tumorexperiment durchgeführt. Hierzu wurden 4 Gruppen (n = 10) mit C57BL/6 Mäusen gebildet. Alle Mäuse erhielt am Tag 0 eine s.c. Injektion von B16-Ova Zellen (2x10⁵). Hiervon wurde eine Kontrollgruppe nur mit IgG4 Injektion behandelt (10 µg; d3, d7, d14, d17). Eine zweite Kontrollgruppe erhielt nur Flt3L-IgG4 Injektionen (15 µg; d3, d7, d14, d17). Die erste Therapiegruppe wurde mit intranodaler Injektion SIINFEKL kodierender RNA (20 µg; d11, d14, d17, d24) in Kombination mit IgG4 Gabe therapiert und die zweite Therapiegruppe erhielt zur RNA Immunisierung Flt3L-IgG4 wie oben angegeben. Dargestellt ist der Kaplan Meier Plot der Überlebensrate der Mäuse. Die Mäuse wurden bei einem Tumordurchmesser von > 1,5 cm in einer Achse abgetötet.

### Abb. 12: Therapeutische Vakzinierung gegen B16 Ova Tumoren.

Untersuchung zum Tumorwachstum. Hierzu wurden 4 Gruppen (n = 10) mit C57BL/6 Mäusen gebildet. Alle Mäuse erhielten am Tag 0 eine s.c. Injektion von B16-Ova Tumor-Zellen (2x10⁵). Hiervon wurde eine Kontrollgruppe nur mit IgG4 Injektion behandelt (15 µg; d3, d7, d14, d18). Eine zweite Kontrollgruppe erhielt nur Flt3L-IgG4 Injektionen (15 µg; d3, d7, d14, d18). Die erste Therapiegruppe wurde mit intranodaler Injektion SIINFEKL kodierender RNA (20 µg; d10, d14, d18, d21) in Kombination mit IgG4 Gabe therapiert und die zweite Therapiegruppe erhielt zur RNA Immunisierung Flt3L-IgG4 wie oben angegeben. Das Tumorvolumen wurde nach Tumorinokulation regelmäßig bestimmt (d7, d10, d13, d16, d19, d22). Dargestellt ist das mittlere Tumorvolumen [mm³] an den Tagen nach Tumorinokulation [Tag].

### Beispiele:

### Beispiel 1:

Der in diesem Beispiel und in den nachstehenden Beispielen verwendete rekombinante humane Flt3-Ligand wurde als Fusionsprotein mit IgG4 bereitgestellt und wies die in SEQ ID NO: 6 gezeigte Sequenz auf. Dazu wurde die für das Flt3L-IgG4 Fusionsprotein kodierende Nukleinsäuresequenz in einen Expressionsvektor kloniert. Das resultierende Plasmid wurde mittels Lipofektion in HEK293 Zellen (ATCC Nr. CRL-1573) transfiziert. Der Überstand wurde gesammelt und über eine Protein A Säule (GE HiTrap MabSelect SuRe, GE Healthcare) gemäß den Herstellerangaben aufgereinigt. Das Produkt wurde gegen PBS dialysiert, aliquotiert und bis zur Verwendung eingefroren.

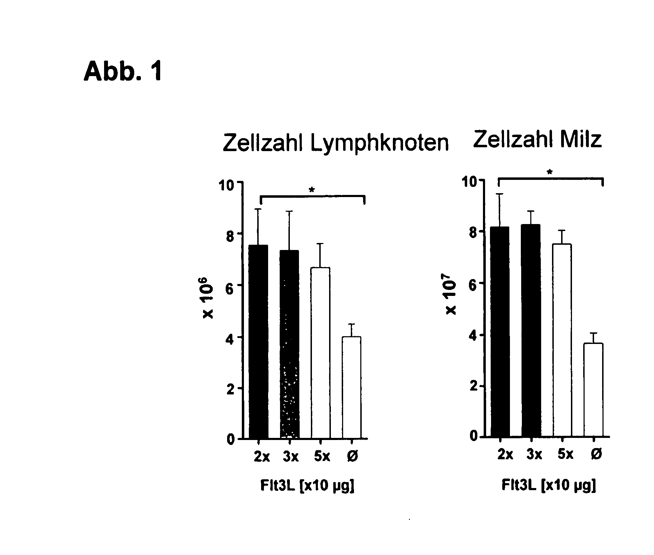

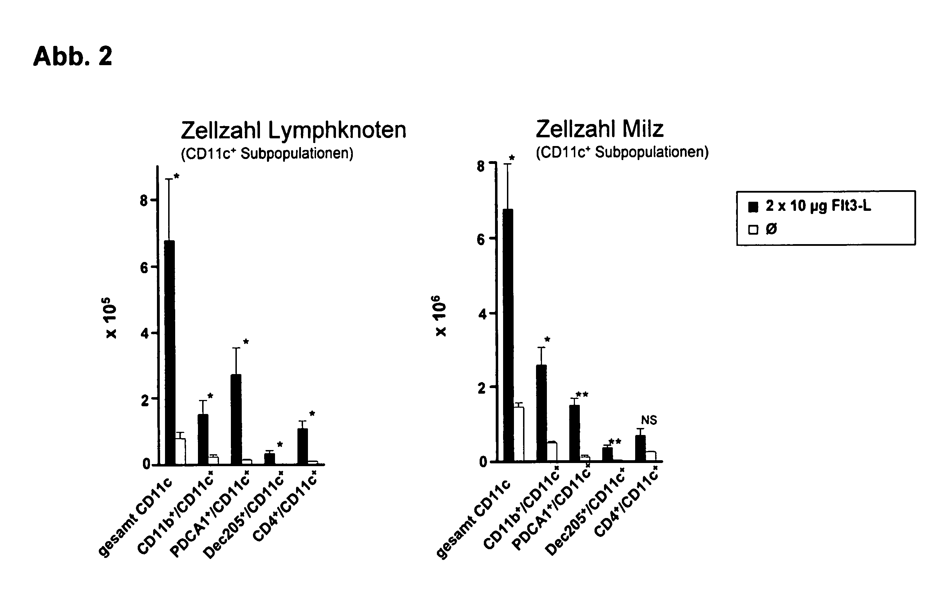

Um die Auswirkungen der Applikation von humanem Flt3-Ligand auf die Effizienz von RNA basierten Immunisierungen zu testen, wurden zunächst im Mausmodell die Veränderungen der zellulären Zusammensetzung von Lymphknoten und Milz untersucht. Hierbei wurden verschiedene Applikationsschemata (2x, 3x, 5x 10 µg) von rekombinantem Flt3-L intraperitoneal appliziert und die Zellularität 10 - 12 Tage nach Erstinjektion bestimmt. Wie in der Literatur für das Maussystem beschrieben konnten wir zeigen (Abb. 1), dass es zu einem Anstieg der Zellularität in Milz und Lymphknoten kommt (Lyman, S.D. et al. (1994) Blood 83:2795-2801, Hannum, C. et al. (1994) Nature 368:643-648, Maraskovsky, E. et al. (1996) Journal of Experimental Medicine 184:1953-1962). Weiterhin zeigte sich (Abb. 2) in Einklang mit publizierten Daten für die Maus und den Menschen, dass die Applikation von Flt3-L in unterschiedlicher Dosis zu einer Vermehrung Dendritischer Zellen führt (Maraskovsky, E. et al. (1996) Journal of Experimental Medicine 184:1953-1962, McNeel, D.G. et al. (2003) Journal of Clinical Immunology 23:62-72, Freedman, R.S. et al. (2003) Clinical Cancer Research 9:5228-5237, Maraskovsky, E. et al. (2000) Blood 96:878-884). Diese Vermehrung konnte für alle relevanten Subpopulationen der Dendritischen Zellen in Milz und Lymphknoten gezeigt werden (Abb. 2).

### Beispiel 2:

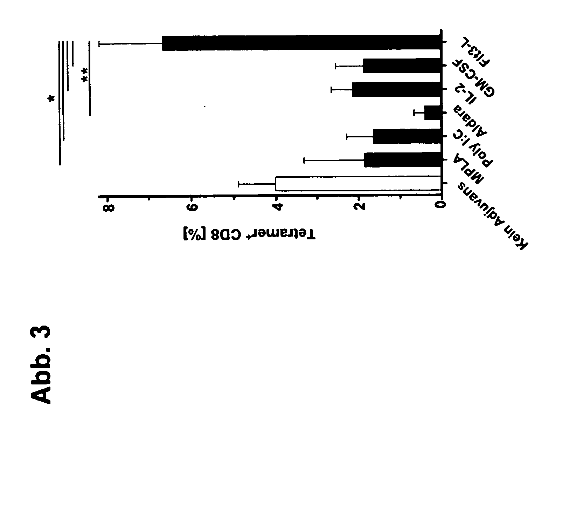

Im Folgenden untersuchten wir, wie sich verschiedene bekannte Adjuvantien (Aldara, Monophosphoryl Lipid A, GM-CSF, Poly I:C, IL2) und Flt3-L auf das Priming naiver T-Zellen und deren Frequenz im peripheren Blut nach intranodaler RNA-Immunisierung auswirken. Zu diesem Zweck wurden die Adjuvantien s.c. oder i.p. appliziert (s. Legende von Abb. 3 für Details) und die Mäuse im Abstand von 3 Tagen zweimal mit einer für das H-2K^{b} restringierte SIINFEKL-Epitop kodierenden RNA immunisiert. Fünf Tage nach der zweiten Immunisierung wurde die Frequenz der epitopspezifischen CD8⁺ T-Zellen durch Tetramermessung im Blut quantifiziert. Zu unserer Überraschung zeigte sich in der Analyse, dass alle Adjuvantien bis auf Flt3-L zu einer Reduktion der Effizienz des T-Zell-Primings führten (Abb. 3). Für die Verwendung von Adjuvantien im Setting der Applikation nackter IVT-RNA ist bisher nur die oben erwähnte Arbeit publiziert in welcher gezeigt wurde, dass nur die Gabe von GM-CSF nach intradermaler RNA-Injektion im Gegensatz zur Gabe vorher einen Vorteil gegenüber der alleinigen RNA-Injektion bringt (Carralot, J.P. et al. (2004) Cell Mol. Life Sci. 61:2418-2424). Diese Daten stehen im Einklang mit unseren Experimenten, bei denen GM-CSF vor der RNA Immunisierung (-48 h, -24 h) appliziert wurde. Darüber hinaus zeigen unsere Daten zum ersten mal, dass etablierte Adjuvantien tendenziell zu einer Verschlechterung der Effizienz des T-Zell-Primings führen, während Flt3-L eine signifikante Steigerung induziert (Abb. 3). Weitere Tetrameranalysen aus peripherem Blut 7 Tage nach der letzten Immunisierung ergaben analoge Ergebnisse (Daten nicht gezeigt).

### Beispiel 3:

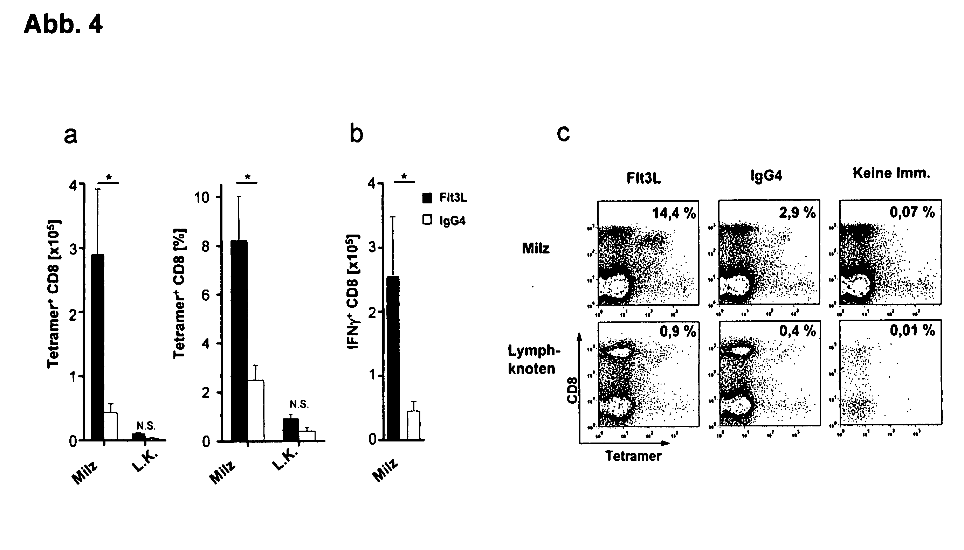

In weiteren Experimenten konnten wir zeigen, dass die Gabe von humanem Flt3-L zu einer signifikanten Steigerung der Frequenz antigenspezifischer funktioneller T-Zellen nach intranodaler RNA-Immunisierung auch in anderen Organen (Milz) führt. Hierzu wurden Mäusen am Tag 0 und Tag 3 jeweils 10 µg Flt3-L oder humanes IgG4 intraperitoneal gespritzt. Am Tag 7 und 10 erfolgte dann die intranodale Immunisierung mit SIINFEKL-kodierender RNA. Am Tag 15 wurde die Frequenz der epitopspezifischen CD8⁺ T-Zellen durch Tetramermessung und intrazelluläre Zytokinbestimmung quantifiziert (Abb. 4). Hierbei zeigte sich in der Tetramerquantifizierung eine signifikant erhöhte Frequenz epitopspezifischer CD8⁺ T-Zellen in der Gruppe, die mit Flt3-L vorbehandelt wurde (Milz: 8,2% vs 2,5%). Auf funktioneller Ebene konnte gezeigt werden, dass diese Zellen auch in der Lage sind, IFNγ zu sezernieren (Abb. 4). Weitere Untersuchungen zeigten, dass Dosiserhöhungen des Flt3-L über die Dosis von 2 x 10 µg hinaus nicht mit einer weiteren Verstärkung der Immunantwort gekoppelt sind (Daten nicht gezeigt).

### Beispiel 4:

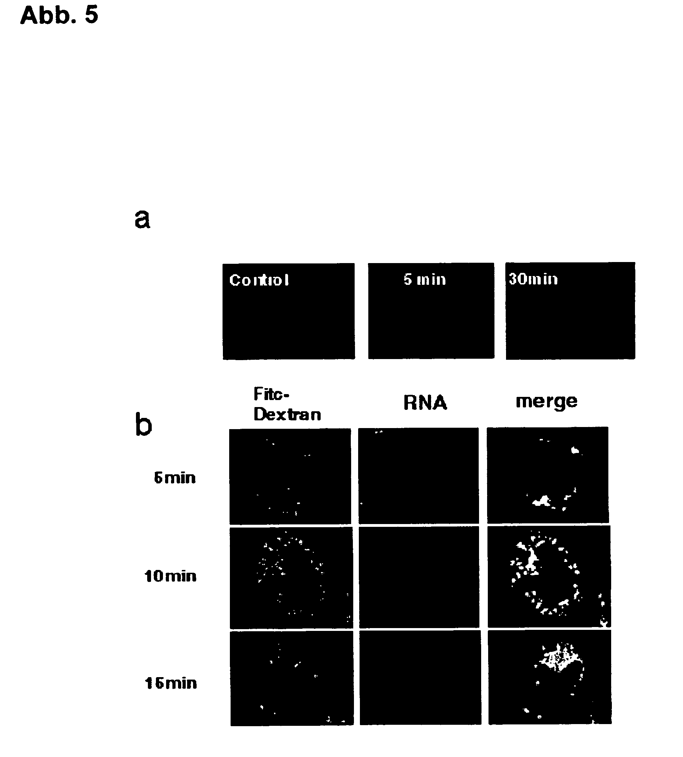

Balb/c Mäusen (n = 5) wurde entweder 10 µg Cy3-Fluorophor-markierte RNA oder reines Cy3-Ribonukleotid (control) intranodal injiziert. Nach 5 oder 30 Minuten wurden Lymphknoten präpariert und Kryostatschnitte nach Paraformaldehydfixierung immunfluoreszenz-mikroskopisch ausgewertet. Die in Abb. 5 gezeigten repräsentativen Schnitte zeigen einen minimalen Hintergrund in den Kontroll-Lymphknoten und ein zelluläres RNA-Signal (Rot) welches von 5 Minuten zu 30 Minuten an Klarheit gewinnt. Dies ist auf die Zerstörung von interzellulär vorliegender RNA zurückzuführen.

Des weiteren wurden humane immature DCs in vitro mit Cy3-Fluorophor markierter RNA (5 µg, Rot) und FITC-Dextran (1 µg/µl, Grün) für 10 min. koinkubiert, mit Paraformaldehyd fixiert und gegengefärbt (Hoechst 33342, Blau). Die Zeitkinetik zeigt wie bei maximaler Kolokalisation mit FITC-Dextran die RNA zu Beginn in der Peripherie der Zelle lokalisiert ist, die Vesikel dann im gesamten Zytoplasma zu sehen sind und schließlich in größeren Strukturen konfluieren.

Somit konnte gezeigt werden, dass nackte RNA sowohl in vitro als auch in vivo von Zellen aufgenommen wird.

### Beispiel 5:

Wir haben das Phänomen der fehlenden Adjuvanswirkung mancher bekannter Adjuvantien näher untersucht.

Wir haben festegestellt, dass nackte RNA (d.h. gelöst in Flüssigkeit z.B. PBS) nach Injektion z.B. in Lymphknoten fast ausschließlich durch Dendritische Zellen aufgenommen wird. Die Aufnahme ist hierbei ungemein effizient. Die aufgenommene RNA wird anschließend translatiert.

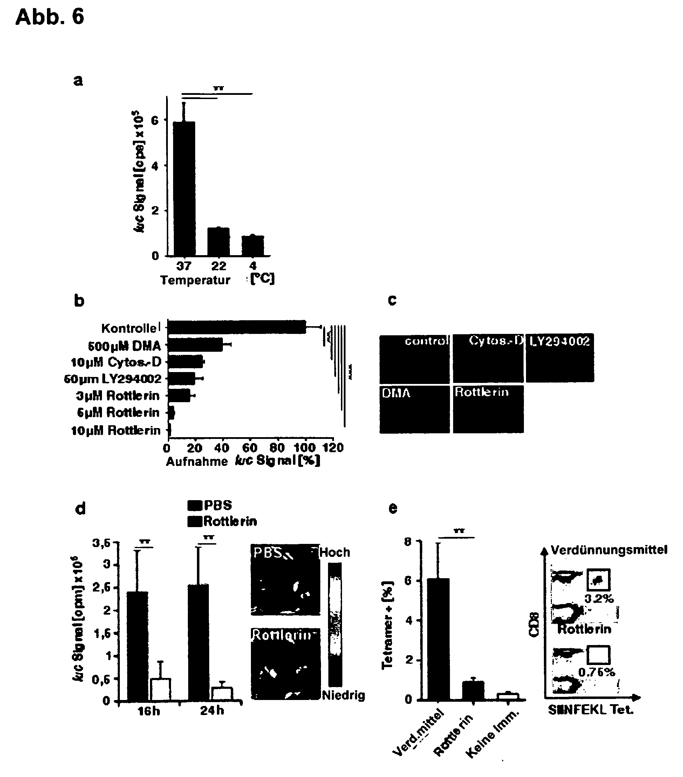

Um den Aufnahmeprozeß für nackte RNA näher zu charakterisieren, wurden in vitro humane iDCs (n = 3) bei verschiedenen Temperaturen mit Luciferase-RNA (20 µg) für 15 min. koinkubiert. Nach weiteren 22 h Kultur bei 37°C wurde die RNA-Aufnahme in einem Luciferasetest quantifiziert. Dargestellt ist der Mean + SEM. Das in Abb. 6(a) gezeigte Ergebnis weist auf einen aktiven energieverbrauchenden Prozeß hin.

Um zu überprüfen, ob die in iDCs konstitutiv aktive Makropinozytose relevant für die Aufnahme nackter RNA ist, wurden humane iDCs mit verschieden Inhibitoren (Dimethylamilorid, Cytochalasin D, LY294002, Rottlerin) vorbehandelt und dann für 15 min. mit Luciferase-RNA oder Cy3-RNA koinkubiert. Nach weiteren 22 h Kultur wurde RNA-Aufnahme in einem Luciferasetest quantifiziert. Dargestellt ist der Mean + SEM. Die mit Cy3-RNA (Rot) koinkubierten iDCs wurden mit Paraformaldehyd fixiert und gegengefärbt (Hoechst 33342, Blau). Es zeigte sich, dass mit dem hochspezifischen Makropinozytoseinhibitor Rottlerin eine Inhibition der RNA-Aufnahme bis über 90 % auftritt; vgl. Abb. 6(b-c).

Um zu klären, ob Makropinozytose auch der in vivo relevante Aufnahmemechanismus für RNA im Lymphknoten ist, wurden die inguinalen Lymphknoten von C57/B16 Mäusen mit Rottlerin (n = 4, 10 µM) vorbehandelt und dann Luciferase-RNA (10 µg) intranodal injiziert. Nach 24 h wurde das in vivo Biolumineszenzsignal gemessen. In Abb. 6(d) ist der Mean + SEM dargestellt. Wir konnten zeigen, dass nach in vivo Inhibition der Makropinozytose die RNA Aufnahme im Lymphknoten drastisch vermindert ist.

Um zu überprüfen, ob die in vivo Inhibition der Makropinozytose sich auf die Effizienz des T-Zell-Primings nach intranodaler RNA-Immunisierung auswirkt, wurden C57B1/6 Mäuse (n = 3) am d0 und d3 mit SIINFEKL-kodierender RNA (20 µg) intranodal immunisiert. An beiden Tagen wurden die Lymphknoten wie oben beschrieben mit Rottlerin vorbehandelt. Dargestellt ist der Mean + SEM der Frequenz CD8⁺ Antigen-spezifischer T-Lymphozyten. Wir konnten nachweisen, dass der Erfolg einer intranodalen RNA Immunisierung direkt mit der Fähigkeit von Zellen, RNA über Makropinozytose aufzunehmen, korreliert; vgl. Abb. 6(e).

Der Hauptaufnahmemechanismus der RNA-Aufnahme ist Makropinozytose. Hemmung der Makropinozytose z.B. durch Chemikalien, welche die Makropinozytose inhibieren (z.B. Rottlerin), führt zu einem fast kompletten Verlust der Impfstoffwirkung.

### Beispiel 6:

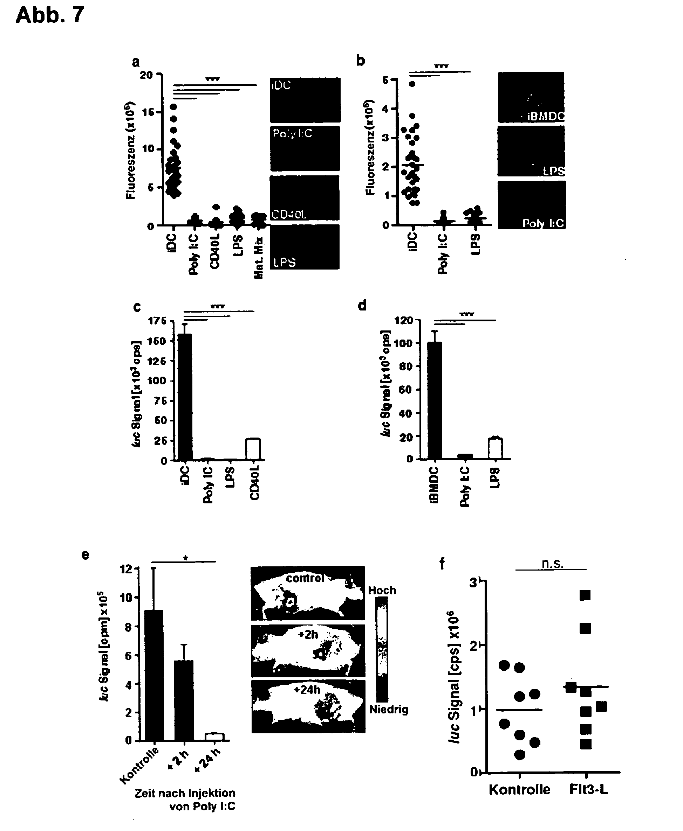

Im Folgenden untersuchten wir inwieweit die Maturation von iDCs, die mit einer Herunterregulation der Makropinozytose verbunden ist, zu einer Reduktion der RNA-Aufnahme führt. Die Ergebnisse sind in Abb. 7 gezeigt.

Wir maturierten humane (Abb. 7(a,c)) und murine (Abb. 7(b,d)) DCs mit verschiedenen Agentien (Poly I:C (50 µg/ml), CD40L (1.0 µg/ml), LPS (20 ng/ml), Mat. Mix (TNFalpha (10 ng/ml), IL1b (10 ng/ml), PGE (1 µg/ml), IL6 (1000 U/ml)) für 40 Stunden. Danach wurden die Zellen für 15 min. mit Luciferase-RNA oder Cy3-RNA koinkubiert. Nach weiteren 22 h Kultur wurde RNA-Aufnahme in einem Luciferasetest quantifiziert. Dargestellt ist der Mean + SEM. Die mit Cy3-RNA (Rot) koinkubierten iDCs wurden mit Paraformaldehyd fixiert und gegengefärbt (Hoechst 33342, Blau). Es zeigte sich sowohl in der Quantifizierung der Cy3-Fluoreszenz als auch dem Luziferasetest, dass nach Maturation der iDCs die RNA-Aufnahme um mehr als 90 % reduziert wurde. Diese Daten sind im Einklang mit publizierten Daten, die zeigen, dass die Maturation von DCs zur Herunterregulierung der Makropinozytose führt. Um zu überprüfen, inwieweit maturierende Adjuvantien ebenfalls in vivo zu einer Reduktion der RNA-Aufnahme führen können, testeten wir den Effekt von Poly I:C auf die RNA Aufnahme; vgl. Abb. 7(e). Hierzu wurden C57B1/6 Mäusen (n = 4) PBS oder Poly I:C (20 µg) s.c. injiziert und nach 2 oder 24 h Luciferase-RNA intranodal appliziert. Die in vivo Biolumineszenz wurde nach weiteren 24 h gemessen. Dargestellt ist der Mean + SEM. Es konnte gezeigt werden, dass es in Abhängigkeit vom Zeitintervall nach Adjuvanzgabe zu einer starken Reduktion der RNA-Aufnahme kommt. Diese Daten stehen im Einklang mit der Beobachtung, dass eine vollständige Maturation von DCs ca. 24 h benötigt.

Im Gegensatz dazu wirkt sich eine Flt3-L-Gabe nicht hemmend auf die RNA-Aufnahme im Lymphknoten aus. C57BL/6 Mäuse (n = 8) wurden am Tag 0 und 3 mit 10 µg Flt3-L i.p. behandelt oder in der Kontrollgruppe nicht behandelt. Am Tag 10 wurde den Mäusen 20 µg Luziferase-RNA intranodal injiziert. 24 h später erfolgte die Messung des Luziferasesignals durch in vivo Biolumineszenz. Die Grafik in Abb. 7(f) zeigt die Meßergebnise für jede einzelne Maus. Der Balken gibt den Mittelwert aller gemessenen Werte für eine Gruppe an. Das Experiment ist representativ für 3 unabhängige Experimente. Statistik: student's t-test.

Des Weiteren wurde C57BL/6 Mäusen (n = 3-7) zweimal Flt3L (Tag 0 und 3, je 10 µg) verabreicht. Am Tag 10 wurden die Lymphknoten präpariert und per Durchflußzytometrie der Aktivierungsstatus (CD86, CD80, MHC-II, CD40) der Dendritischen Zellen bestimmt. Wir konnten zeigen, dass die Gabe von Flt3L nicht zu einer Maturation der Dendritischen Zellen im Lymphknoten führt.

### Beispiel 7: Auswirkungen von verschiedenen Flt3L auf die zelluläre Zusammensetzung im Lymphknoten

In diesem Experiment wurde FLt3-Ligand (Flt3-IgG4) mit kommerziell erhältlichen Flt3 Präparaten hinsichtlich der Auswirkung auf verschiedene Zellpopulationen des Lymphknotens der Maus untersucht. Als kommerziell erhältliche Flt3-Präparate wurden ein in Bakterien rekombinant exprimiertes Produkt (Peprotech Flt3L; Peprotech, Hamburg, Germany) und ein in humanen HEK293 Zellen exprimiertes Produkt (Humanzyme Flt3L, Humanzyme; Chicago IL, U.S.A) verwendet. Als Kontrolle diente humanes IgG4 (Sigma-Aldrich, Deisenhofen, Germany).

C57BL/6 Mäusen (n = 5) wurde am Tag 0 Flt3L-IgG4, Flt3L (Humanzyme), Flt3L (Peprotech) oder humanes IgG4 (Sigma-Aldrich) in einer Menge von 0,4 mol intraperitoneal injiziert. Am Tag 10 wurden beide inguinalen Lymphknoten der Mäuse präpariert, die Zellzahlen mittels Neubauerkammer bestimmt und die Zellpopulationen durchflußzytometrisch charakterisiert.

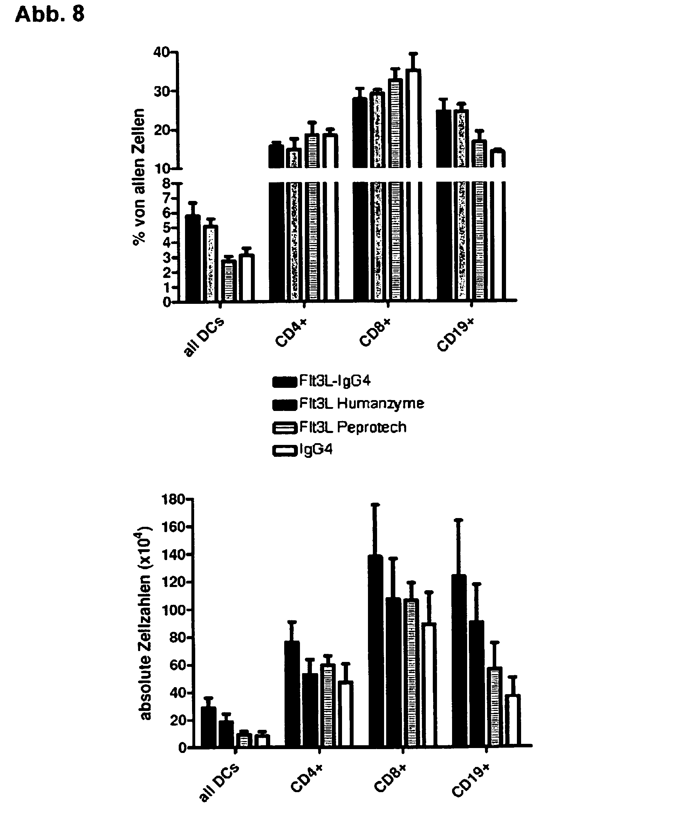

Die verschiedenen Zellpopulationen wurden über folgende Markerkombinationen definiert: Dendritische Zellen (DCs (Marker: CD 11 c⁺/NK1.1⁻), CD4⁺ Helfer T-Zellen (Marker: CD3⁺/CD4⁺/CD8⁻/NK1.1⁻), CD8⁺ T-Zellen (Marker: CD3⁺/CD8⁺/CD4⁻/NK1.1⁻), CD19⁺ B-Zellen (Marker: CD19⁺/CD3⁻/NK1.1⁻). Die Antikörper zur Detektion der Oberflächenmarker wurden von der Firma Beckton Dickinson bezogen. In Abbildung 8 ist die Häufigkeit von Dendritischen Zellen (all DCs), CD4 positiven, CD8 positiven und CD 19 positiven Zellen in Relation zur Gesamtzahl der präparierten Zellen des Lymphknotens gezeigt.

Es zeigte sich, dass die durch Flt3L-IgG4 ausgelösten Effekte ähnlich zu denen waren, die durch die kommerziell erhältlichen Flt3L Produkte induziert wurden. Bezüglich der Expansion dendritischer Zellen wiesen Flt3L-IgG4 und Flt3L der Firma Humanzyme große Ähnlichkeiten auf während der Peprotech Flt3L nur eine geringe Potenz in dieser Hinsicht zeigte. Bezogen auf die Expansion der Lymphzytenpopulationen waren die Effekte von Flt3L-IgG4 tendenziell am stärksten ausgeprägt.

### Beispiel 8: Auswirkungen von verschiedenen Flt3L auf die Stimulation naiver T-Zellen

In diesem Experiment wurde untersucht, inwieweit die Adjuvanzfunktion von Flt3L-IgG4 äquivalent ist zu der von kommerziell erhältlichen Flt3L Produkten. Ausgesucht wurde hierzu ein in Bakterien rekombinant exprimiertes Produkt (Peprotech Flt3L) und ein in humanen HEK293 Zellen exprimiertes Produkt (Humanzyme Flt3L); vgl. Beispiel 7. Als Kontrolle diente humanes IgG4.

Naiven C57BL/6 Mäusen (n = 7) wurde am Tag 0, +3 Flt3L (Flt3L-IgG4 oder Flt3L (Humanzyme) oder Flt3L (Peprotech)) oder humanes IgG4 (Sigma) in einer Menge von 0,4 mol intraperitoneal injiziert. Diese Mäuse wurden am Tag +7, +10 mit 20 µg SIINFEKL kodierender RNA intralymphatisch immunisiert. Die Kontrollgruppe wurde nicht behandelt (n = 2). Am Tag +15 wurde aus dem peripheren Blut mittels MHC-Multimermessung (Beckman Coulter) durchflußzytometrisch die Frequenz der antigenspezifischen CD8⁺ T-Lymphozyten gemessen.

Flt3L wurde den Mäusen in äquimolaren Mengen intraperitoneal verabreicht (Tag 0, +3). Zusätzlich wurden die Mäuse zweimal mit SIINFEKL kodierender RNA intranodal immunisiert (+7, +10). Am Tag +15 wurde mittels einer Tetramerfärbung der Erfolg der Immunisierung durchflußzytometrisch quantifiziert.

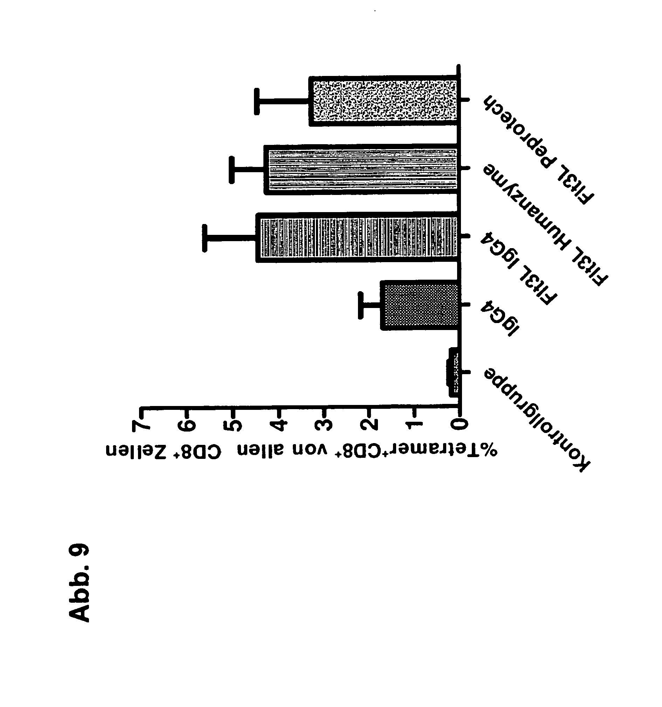

Es zeigte sich, dass sowohl Flt3L-IgG4 als auch die kommerziell erhältlichen Flt3L Produkte einen deutlichen Adjuvanzeffekt aufwiesen. Die nicht immunisierte Kontrollgruppe zeigte keine relevante Frequenz Tetramerpositiver T-Zellen. Gegenüber den Mäusen, welche ohne die Applikation von Flt3L immunisiert wurden, war die Frequenz antigenspezifischer CD8⁺ T-Lymphozyten um einen Faktor von 2-3 gesteigert. Tendenziell konnte der stärkste Effekt bei Verwendung von Flt3L-IgG4 beobachtet werden (Abb. 9).

### Beispiel 9: Bestimmung der Serumhalbwertszeit von Flt3-IgG4

Um die Serumhalbwertszeit des Flt3L-IgG4 zu bestimmen, wurden zwei Gruppen von Balb/c Mäusen (n = 3) 20 µg bzw. 50 µg Flt3L-IgG4 i.p. verabreicht. Zu definierten Zeitpunkten (vor Gabe; 3 h, 24 h, 48 h, 3 d, 5 d, 7 d, 9 d, 14 d, 21 d) wurden Serumproben der Mäuse asserviert. Diese Proben wurden in einem ELISA-Test zur Quantifizierung von humanem IgG eingesetzt. Aufgrund der Fusion von humanem IgG4 an den Flt3L in unserem Konstrukt kann über die Quantifizierung von humanem IgG in Mäuseserum die Flt3L Konzentration ermittelt werden. Die Daten zeigen, dass nach einem initialen Maximum Flt3L bis 5 Tage nach Injektion im Mausserum nachweisbar ist. Die errechnete Halbwertszeit für 50 µg Flt3L-IgG4 liegt bei 40 Stunden, die Halbwertszeit (HWZ) für 20 µg liegt bei 51 Stunden.

Diese Werte zeigen im Vergleich zu dem für die Halbwertszeit von Flt3L publizierten Wert von 5 h (Robinson et al., 2003, BMT, 31:361-369) eine gesteigerte Stabilität von Flt3L-IgG4 gegenüber Flt3L ohne IgG4-Fusionsanteil (Abb. 10).

### Beispiel 10: Therapeutische Vakzinierung gegen B16 Ova Tumoren

Um die Synergie der Kombination von Flt3L-Gabe mit RNA Vakzinierung zu untersuchen, wurde ein therapeutisches Tumorexperiment durchgeführt. Hierzu wurden 4 Gruppen (n = 10) mit C57BL/6 Mäusen gebildet. Alle Mäuse erhielten am Tag 0 eine s.c. Injektion von 2x10⁵ B16-Ova Tumor-Zellen (Bellone et al., J. Immunol., 2000, 165:2651-2656). Hiervon wurde eine Kontrollgruppe nur mit IgG4 Injektion behandelt (10 µg; d3, d7, d 14, d17). Eine zweite Kontrollgruppe erhielt nur Flt3L-IgG4 Injektionen (15 µg; d3, d7, d14, dg17). Die erste Therapiegruppe wurde mit intranodaler Injektion SIINFEKL kodierender RNA (20 µg; d11, d14, d17, d24) in Kombination mit IgG4 Gabe therapiert, und die zweite Therapiegruppe erhielt zur RNA Immunisierung Flt3L-IgG4 wie oben angegeben.

Es zeigte sich, dass die Kombination von Flt3L-IgG4 mit der intranodalen RNA Vakzinierung synergistisch wirkt. Während bei RNA Vakzinierung ohne Flt3L-IgG4 nur 1/3 der Mäuse langfristig überlebt, kann durch die Kombination mit Flt3L-IgG4 der Anteil der langfristig überlebenden Mäuse auf ca. 80 % angehoben werden. Flt3L-IgG4 ohne RNA Vakzinierung zeigt einen minimalen therapeutischen Effekt auf das Tumorwachstum, welcher aber nur zu einem Langzeitüberleben von 10 % der Tiere führt (Abb. 11).

### Beispiel 11: Therapeutische Vakzinierung gegen B16 Ova Tumoren

Zur Bestätigung des synergistischen Effektes einer kombinierten Gabe von Flt3L und einer RNA-Vakzine wurde ein weiteres therapeutisches Tumorexperiment durchgeführt. Hierzu wurden 4 Gruppen (n = 10) mit C57BL/6 Mäusen gebildet. Alle Mäuse erhielten am Tag 0 eine s.c. Injektion von 2x10⁵ B16-Ova Tumor-Zellen (Bellone et al., J. Immunol., 2000, 165:2651-2656). Hiervon wurde eine Kontrollgruppe nur mit IgG4 Injektion behandelt (15 µg; d3, d7, d14, d18). Eine zweite Kontrollgruppe erhielt nur Flt3L-IgG4 Injektionen (15 µg; d3, d7, d14, d18). Die erste Therapiegruppe wurde mit intranodaler Injektion SIINFEKL kodierender RNA (20 µg; d 10, d14, d18 und d21) in Kombination mit IgG4 Gabe therapiert und die zweite Therapiegruppe erhielt zur RNA Immunisierung Flt3L-IgG4 (Flt3L) wie oben angegeben. Das Tumorvolumen wurde an folgenden Tagen nach nach Tumorinokulation bestimmt: d7, d10, d13, d16, d19 und d22 (d=Tag).

Es zeigte sich, dass die Kombination von Flt3L-IgG4 mit der intranodalen RNA Vakzination synergistisch wirkt. Bei alleiniger Flt3L Gabe war nur eine geringfügige Verzögerung des Tumorwachstums zu beobachten und bei alleiniger RNA Vakzination war ebenfalls ein, zwar verlangsamtes, aber doch progredientes Tumorwachstum vorhanden. Erst in der Kombination von Flt3L-Gabe und RNA Vakzinierung konnte ein komplettes Sistieren des Tumorwachstums beobachtet werden (Abb. 12).

## Patentansprüche

1. Immunogenes Präparat, das RNA, die für mindestens ein Antigen kodiert, und Flt3-Ligand umfasst.

2. Immunogenes Präparat nach Anspruch 1, wobei die RNA mRNA ist.

3. Immunogenes Präparat nach Anspruch 1 oder 2, wobei die RNA durch in vitro-Transkription erhalten wurde.

4. Immunogenes Präparat nach einem der Ansprüche 1 bis 3, das ferner mindestens einen RNA-stabilisierenden Faktor umfasst.

5. Pharmazeutische Zusammensetzung, die ein immunogenes Präparat nach einem der Ansprüche 1 bis 4 umfasst.

6. Pharmazeutische Zusammensetzung nach Anspruch 5 in Form einer Formulierung als Vakzine.

7. Flt3-Ligand zur Verwendung in einem Verfahren zum Erzeugen oder Verstärken einer Immunreaktion bei einem Individuum, das eine Verabreichung von RNA, die für mindestens ein Antigen kodiert, gegen das die Immunreaktion gerichtet sein soll, und eine Verabreichung von Flt3-Ligand umfasst.

8. Flt3-Ligand nach Anspruch 7, wobei die Immunreaktion eine Antigen-spezifische T-Zell-Immunreaktion umfasst.

9. Flt3-Ligand zur Verwendung in einem Verfahren zur Erhöhung der Menge von Antigen-spezifischen Effektorzellen in einem Individuum, das eine Verabreichung von RNA, die für das Antigen kodiert, und eine Verabreichung von Flt-3-Ligand umfasst.

10. Flt3-Ligand nach Anspruch 9, wobei die Antigen-spezifischen Effektorzellen CD8+ cytotoxische T-Zellen und/oder CD4+ Helfer-T-Zellen sind.

11. Flt3-Ligand zur Verwendung in einem Verfahren zur Vermeidung und/oder Behandlung von Krebs in einem Individuum, das eine Verabreichung von RNA, die für ein Tumorantigen kodiert, gegen das die Immunreaktion gerichtet sein soll, und eine Verabreichung von Flt3-Ligand umfasst.

12. Flt3-Ligand zur Verwendung in einem Verfahren zur Vermeidung und/oder Behandlung einer viralen Infektion in einem Individuum, das eine Verabreichung von RNA, die für ein virales Antigen kodiert, gegen das die Immunreaktion gerichtet sein soll, und eine Verabreichung von Flt3-Ligand umfasst.

13. Flt3-Ligand zur Verwendung in einem Verfahren zur Vermeidung und/oder Behandlung einer bakteriellen Infektion in einem Individuum, das eine Verabreichung von RNA, die für ein bakterielles Antigen kodiert, gegen das die Immunreaktion gerichtet sein soll, und eine Verabreichung von Flt3-Ligand umfasst.

14. Flt3-Ligand zur Verwendung in einem Verfahren zur Vermeidung und/oder Behandlung einer Allergie in einem Individuum, das eine Verabreichung von RNA, die für ein bei der Allergie relevantes Allergen kodiert, und eine Verabreichung von Flt3-Ligand umfasst.

15. Flt3-Ligand nach einem der Ansprüche 7 bis 14, wobei das Individuum ein Mensch ist.

16. Verwendung von Flt3-Ligand zur Herstellung einer pharmazeutischen Zusammensetzung zur Erhöhung der Immunogenizität von Vakzine-RNA.

## Claims

1. An immunogenic preparation, which comprises RNA that codes for at least one antigen, and Flt3 ligand.

2. The immunogenic preparation as claimed in claim 1, **characterized in that** the RNA is mRNA.

3. The immunogenic preparation as claimed in claim 1 or 2, **characterized in that** the RNA was obtained by in vitro transcription.

4. The immunogenic preparation as claimed in any one of claims 1 to 3, which further comprises at least one RNA-stabilizing factor.

5. A pharmaceutical composition that comprises an immunogenic preparation as claimed in any one of claims 1 to 4.

6. The pharmaceutical composition as claimed in claim 5 in the form of a formulation as vaccine.

7. Flt3 ligand for use in a method of producing or intensifying an immune response in an individual, **characterized in that** it comprises the administration of RNA that codes for at least one antigen, against which the immune response is to be directed, and the administration of Flt3 ligand.

8. The Flt3 ligand as claimed in claim 7, **characterized in that** the immune response comprises an antigen-specific T cell immune response.

9. Flt3 ligand for use in a method of increasing the amount of antigen-specific effector cells in an individual, **characterized in that** it comprises the administration of RNA that codes for the antigen, and the administration of Flt-3 ligand.

10. The Flt3 ligand as claimed in claim 9, **characterized in that** the antigen-specific effector cells are CD8+ cytotoxic T cells and/or CD4+ helper T cells.

11. Flt3 ligand for use in a method for prevention and/or treatment of cancer in an individual, **characterized in that** it comprises the administration of RNA that codes for a tumor antigen, against which the immune response is to be directed, and the administration of Flt3 ligand.

12. Flt3 ligand for use in a method for prevention and/or treatment of a viral infection in an individual, **characterized in that** it comprises the administration of RNA that codes for a viral antigen, against which the immune response is to be directed, and the administration of Flt3 ligand.

13. Flt3 ligand for use in a method for prevention and/or treatment of a bacterial infection in an individual, **characterized in that** it comprises the administration of RNA that codes for a bacterial antigen, against which the immune response is to be directed, and the administration of Flt3 ligand.

14. Flt3 ligand for use in a method for prevention and/or treatment of an allergy in an individual, **characterized in that** it comprises the administration of RNA that codes for an allergen relevant to the allergy, and the administration of Flt3 ligand.

15. The Flt3 ligand as claimed in any one of claims 7 to 14, **characterized in that** the individual is a human.

16. A use of Flt3 ligand for preparing a pharmaceutical composition for increasing the immunogenicity of vaccine-RNA.

## Revendications

1. Préparation immunogène, qui comprend un ARN qui code pour au moins un antigène, et un ligand de Flt3.

2. Préparation immunogène selon la revendication 1, dans laquelle l'ARN est un ARNm.

3. Préparation immunogène selon la revendication 1 ou 2, dans laquelle l'ARN a été obtenu par transcription in vitro.

4. Préparation immunogène selon l'une des revendications 1 à 3, qui comprend en outre au moins un facteur de stabilisation d'ARN.

5. Composition pharmaceutique, qui comprend une préparation immunogène selon l'une des revendications 1 à 4.

6. Composition pharmaceutique selon la revendication 5, sous forme d'une formulation comme vaccin.

7. Ligand de Flt3 pour utilisation dans un procédé pour l'obtention ou le renforcement d'une réaction immunitaire chez un individu, qui comprend l'administration d'ARN qui code pour au moins un antigène contre lequel la réaction immunitaire doit être dirigée, et l'administration de ligand de Flt3.

8. Ligand de Flt3 selon la revendication 7, dans lequel la réaction immunitaire comprend une réaction immunitaire par cellules T spécifiques pour l'antigène.

9. Ligand de Flt3 pour utilisation dans un procédé pour augmenter la quantité de cellules effectrices spécifiques pour un antigène chez un individu, qui comprend une administration d'ARN qui code pour l'antigène et une administration de ligand de Flt3.

10. Ligand de Flt3 selon la revendication 9, dans lequel les cellules effectrices spécifiques pour un antigène sont des cellules T cytotoxiques CD8+ et/ou des cellules T auxiliaires CD4+.

11. Ligand de Flt3 pour utilisation dans un procédé pour empêcher et/ou traiter un cancer chez un individu, qui comprend l'administration d'ARN, qui code pour un antigène tumoral contre lequel la réaction immunitaire doit être dirigée, et l'administration de ligand de Flt3.

12. Ligand de Flt3 pour utilisation dans un procédé pour empêcher et/ou traiter une infection virale chez un individu, qui comprend l'administration d'ARN qui code pour un antigène viral contre lequel la réaction immunitaire doit être dirigée, et l'administration de ligand de Flt3.

13. Ligand de Flt3 pour utilisation dans un procédé pour empêcher et/ou traiter une infection bactérienne chez un individu, qui comprend l'administration d'ARN qui code pour un antigène bactérien contre lequel la réaction immunitaire doit être dirigée, et l'administration de ligand de Flt3.

14. Ligand de Flt3 pour utilisation dans un procédé pour empêcher et/ou traiter une allergie chez un individu, qui comprend l'administration d'ARN qui code pour un allergène concerné dans l'allergie, et l'administration de ligand de Flt3.

15. Ligand de Flt3 selon l'une des revendications 7 à 14, dans lequel l'individu est un humain.

16. Utilisation de ligand de Flt3 pour la préparation d'une composition pharmaceutique pour accroître l'immunogénicité d'ARN vaccinal.


### SEQUENCE LISTING

<110> BioNTech AG, Johannes Gutenberg-universität Mainz
<120> Verwendung von Flt3-Ligand zur Verstärkung von Immunreaktionen bei RNA-Immunisierung
<130> 410-9 PCT
<150> DE 10 2008 061 522.6
   <151> 2008-12-10
<160> 6
<170> PatentIn version 3.3
<210> 1
   <211> 235
   <212> PRT
   <213> Homo sapiens
<400> 1
   Met Thr Val Leu Ala Pro Ala Trp Ser Pro Thr Thr Tyr Leu Leu Leu
   1 5 10 15
   Leu Leu Leu Leu Ser Ser Gly Leu Ser Gly Thr Gln Asp Cys Ser Phe
   20 25 30
   Gln His Ser Pro Ile Ser Ser Asp Phe Ala Val Lys Ile Arg Glu Leu
   35 40 45
   Ser Asp Tyr Leu Leu Gln Asp Tyr Pro Val Thr Val Ala Ser Asn Leu
   50 55 60
   Gln Asp Glu Glu Leu Cys Gly Gly Leu Trp Arg Leu Val Leu Ala Gln
   65 70 75 80
   Arg Trp Met Glu Arg Leu Lys Thr Val Ala Gly Ser Lys Met Gln Gly
   85 90 95
   Leu Leu Glu Arg Val Asn Thr Glu Ile His Phe Val Thr Lys Cys Ala
   100 105 110
   Phe Gln Pro Pro Pro Ser Cys Leu Arg Phe Val Gln Thr Asn Ile Ser
   115 120 125
   Arg Leu Leu Gln Glu Thr Ser Glu Gln Leu Val Ala Leu Lys Pro Trp
   130 135 140
   Ile Thr Arg Gln Asn Phe Ser Arg Cys Leu Glu Leu Gln Cys Gln Pro
   145 150 155 160
   Asp Ser Ser Thr Leu Pro Pro Pro Trp Ser Pro Arg Pro Leu Glu Ala
   165 170 175
   Thr Ala Pro Thr Ala Pro Gln Pro Pro Leu Leu Leu Leu Leu Leu Leu
   180 185 190
   Pro Val Gly Leu Leu Leu Leu Ala Ala Ala Trp Cys Leu His Trp Gln
   195 200 205
   Arg Thr Arg Arg Arg Thr Pro Arg Pro Gly Glu Gln Val Pro Pro Val
   210 215 220
   Pro Ser Pro Gln Asp Leu Leu Leu Val Glu His
   225 230 235
<210> 2
   <211> 232
   <212> PRT
   <213> Mus musculus
<400> 2
   Met Thr Val Leu Ala Pro Ala Trp Ser Pro Asn Ser Ser Leu Leu Leu
   1               5                   10                  15
   Leu Leu Leu Leu Leu Ser Pro Cys Leu Arg Gly Thr Pro Asp Cys Tyr
               20                  25                  30
   Phe Ser His Ser Pro Ile Ser Ser Asn Phe Lys Val Lys Phe Arg Glu
           35                  40                  45
   Leu Thr Asp His Leu Leu Lys Asp Tyr Pro Val Thr Val Ala Val Asn
       50                  55                  60
   Leu Gln Asp Glu Lys His Cys Lys Ala Leu Trp Ser Leu Phe Leu Ala
   65                  70                  75                  80
   Gln Arg Trp Ile Glu Gln Leu Lys Thr Val Ala Gly Ser Lys Met Gln
                   85                  90                  95
   Thr Leu Leu Glu Asp Val Asn Thr Glu Ile His Phe Val Thr Ser Cys
               100                 105                 110
   Thr Phe Gln Pro Leu Pro Glu Cys Leu Arg Phe Val Gln Thr Asn Ile
           115                 120                 125
   Ser His Leu Leu Lys Asp Thr Cys Thr Gln Leu Leu Gly Leu Lys Pro
       130                 135                 140
   Cys Ile Gly Lys Ala Cys Gln Asn Phe Ser Arg Cys Leu Glu Val Gln
   145                 150                 155                 160
   Cys Gln Pro Asp Ser Ser Thr Leu Leu Pro Pro Arg Ser Pro Ile Ala
                   165                 170                 175
   Leu Glu Ala Thr Glu Leu Pro Glu Pro Arg Pro Arg Gln Leu Leu Leu
               180                 185                 190
   Leu Leu Leu Leu Leu Leu Pro Leu Thr Leu Val Leu Leu Ala Ala Ala
           195                 200                 205
   Trp Gly Leu Arg Trp Gln Arg Ala Arg Arg Arg Gly Glu Leu His Pro
       210                 215                 220
   Gly Val Pro Leu Pro Ser His Pro
   225                 230
<210> 3
   <211> 1056
   <212> DNA
   <213> Homo sapiens
<400> 3
   tctctggctg tcacccggct tggccccttc cacacccaac tggggcaagc ctgacccggc      60
   gacaggaggc atgaggggcc cccggccgaa atgacagtgc tggcgccagc ctggagccca     120
   acaacctatc tcctcctgct gctgctgctg agctcgggac tcagtgggac ccaggactgc     180
   tccttccaac acagccccat ctcctccgac ttcgctgtca aaatccgtga gctgtctgac     240
   tacctgcttc aagattaccc agtcaccgtg gcctccaacc tgcaggacga ggagctctgc     300
   gggggcctct ggcggctggt cctggcacag cgctggatgg agcggctcaa gactgtcgct     360
   gggtccaaga tgcaaggctt gctggagcgc gtgaacacgg agatacactt tgtcaccaaa     420
   tgtgcctttc agcccccccc cagctgtctt cgcttcgtcc agaccaacat ctcccgcctc     480
   ctgcaggaga cctccgagca gctggtggcg ctgaagccct ggatcactcg ccagaacttc     540
   tcccggtgcc tggagctgca gtgtcagccc gactcctcaa ccctgccacc cccatggagt     600
   ccccggcccc tggaggccac agccccgaca gccccgcagc cccctctgct cctcctactg     660
   ctgctgcccg tgggcctcct gctgctggcc gctgcctggt gcctgcactg gcagaggacg     720
   cggcggagga caccccgccc tggggagcag gtgccccccg tccccagtcc ccaggacctg     780
   ctgcttgtgg agcactgacc tggccaaggc ctcatcctgg ggaggatact gaggcacaca     840
   gaggggagtc accagccaga ggatgcatag cctggacaca gaggaagttg gctagaggcc     900
   ggtcccttcc ttgggcccct ctcattccct ccccagaatg gaggcaacgc cagaatccag     960
   caccggcccc atttacccaa ctctgtacaa agcccttgtc cccatgaaat tgtatataaa    1020
   tcatcctttt ctaccaaaaa aaaaaaaaaa aaaaaa                              1056
<210> 4
   <211> 1152
   <212> DNA
   <213> Mus musculus
<400> 4
   gaattcgcgg ccgcgtcgac attctgggga cgtcggtcgg ggttcttaga agaggagatg      60
   acttttcaca gtcactgagg ctcgtgcagg aagcctgggg gagcaggagg cggaaaccga     120
   cccacatcaa gggcggcagg gccgggcggc ggggtacagg ggttgggggg gaaggggctg     180
   cagggtatga gcccgagacc tgccctcctg tcacttccaa gaacctgtca caggcatgag     240
   gggtccccgg cagagatgac agtgctggcg ccagcctgga gcccaaattc ctccctgttg     300
   ctgctgttgc tgctgctgag tccttgcctg cgggggacac ctgactgtta cttcagccac     360
   agtcccatct cctccaactt caaagtgaag tttagagagt tgactgacca cctgcttaaa     420
   gattacccag tcactgtggc cgtcaatctt caggacgaga agcactgcaa ggccttgtgg     480
   agcctcttcc tagcccagcg ctggatagag caactgaaga ctgtggcagg gtctaagatg     540
   caaacgcttc tggaggacgt caacaccgag atacattttg tcacctcatg taccttccag     600
   cccctaccag aatgtctgcg attcgtccag accaacatct cccacctcct gaaggacacc     660
   tgcacacagc tgcttggtct gaagccctgt atcgggaagg cctgccagaa tttctctcgg     720
   tgcctggagg tgcagtgcca gccggactcc tccaccctgc tgcccccaag gagtcccata     780
   gccctagaag ccacggagct cccagagcct cggcccaggc agctgttgct cctgctgctg     840
   ctgctgctgc ctctcacact ggtgctgctg gcagccgcct ggggccttcg ctggcaaagg     900
   gcaagaagga ggggggagct ccaccctggg gtgcccctcc cctcccatcc ctaggatgcg     960
   agccttgtgc atcgttgact cagccagggt cttatctcga tgaggtctca atatgttgcc    1020
   caaactgact ttgaaaacct cgatgcacct tcctgcccca caaacttcca aacagctggg    1080
   cttacgggca tgctatatac aacaaggctt tcttttcttc tttcttggtg ctagagttgg    1140
   gaaccaaaac aa                                                        1152
<210> 5
   <211> 224
   <212> PRT
   <213> Homo sapiens
<400> 5
   Pro Pro Cys Pro Ser Cys Pro Ala Pro Glu Phe Leu Gly Gly Pro Ser
   1               5                   10                  15
   Val Phe Leu Phe Pro Pro Lys Pro Lys Asp Thr Leu Met Ile Ser Arg
               20                  25                  30
   Thr Pro Glu Val Thr Cys Val Val Val Asp Val Ser Gln Glu Asp Pro
           35                  40                  45
   Glu Val Gln Phe Asn Trp Tyr Val Asp Gly Val Glu Val His Asn Ala
       50                  55                  60
   Lys Thr Lys Pro Arg Glu Glu Gln Phe Asn Ser Thr Tyr Arg Val Val
   65                  70                  75                  80
   Ser Val Leu Thr Val Leu His Gln Asp Trp Leu Asn Gly Lys Glu Tyr
                   85                  90                  95
   Lys Cys Lys Val Ser Asn Lys Gly Leu Pro Ser Ser Ile Glu Lys Thr
   100 105 110
   Ile Ser Lys Ala Lys Gly Gln Pro Arg Glu Pro Gln Val Tyr Thr Leu
   115 120 125
   Pro Pro Ser Gln Glu Glu Met Thr Lys Asn Gln Val Ser Leu Thr Cys
   130 135 140
   Leu Val Lys Gly Phe Tyr Pro Ser Asp Ile Ala Val Glu Trp Glu Ser
   145 150 155 160
   Asn Gly Gln Pro Glu Asn Asn Tyr Lys Thr Thr Pro Pro Val Leu Asp
   165 170 175
   Ser Asp Gly Ser Phe Phe Leu Tyr Ser Arg Leu Thr Val Asp Lys Ser
   180 185 190
   Arg Trp Gln Glu Gly Asn Val Phe Ser Cys Ser Val Met His Glu Ala
   195 200 205
   Leu His Asn His Tyr Thr Gln Lys Ser Leu Ser Leu Ser Pro Gly Lys
   210 215 220
<210> 6
   <211> 411
   <212> PRT
   <213> Artificial
<220>
   <223> Fusionsprotein
<400> 6
   Met Thr Val Leu Ala Pro Ala Trp Ser Pro Thr Thr Tyr Leu Leu Leu
   1 5 10 15
   Leu Leu Leu Leu Ser Ser Gly Leu Ser Gly Thr Gln Asp Cys Ser Phe
   20 25 30
   Gln His Ser Pro Ile Ser Ser Asp Phe Ala Val Lys Ile Arg Glu Leu
   35 40 45
   Ser Asp Tyr Leu Leu Gln Asp Tyr Pro Val Thr Val Ala Ser Asn Leu
   50 55 60
   Gln Asp Glu Glu Leu Cys Gly Gly Leu Trp Arg Leu Val Leu Ala Gln
   65 70 75 80
   Arg Trp Met Glu Arg Leu Lys Thr Val Ala Gly Ser Lys Met Gln Gly
   85 90 95
   Leu Leu Glu Arg Val Asn Thr Glu Ile His Phe Val Thr Lys Cys Ala
   100 105 110
   Phe Gln Pro Pro Pro Ser Cys Leu Arg Phe Val Gln Thr Asn Ile Ser
   115 120 125
   Arg Leu Leu Gln Glu Thr Ser Glu Gln Leu Val Ala Leu Lys Pro Trp
   130 135 140
   Ile Thr Arg Gln Asn Phe Ser Arg Cys Leu Glu Leu Gln Cys Gln Pro
   145 150 155 160
   Asp Ser Ser Thr Leu Pro Pro Pro Trp Ser Pro Arg Pro Leu Glu Ala
   165 170 175
   Thr Ala Pro Thr Ala Pro Gln Pro Pro Arg Ser Pro Pro Cys Pro Ser
   180 185 190
   Cys Pro Ala Pro Glu Phe Leu Gly Gly Pro Ser Val Phe Leu Phe Pro
   195 200 205
   Pro Lys Pro Lys Asp Thr Leu Met Ile Ser Arg Thr Pro Glu Val Thr
   210 215 220
   Cys Val Val Val Asp Val Ser Gln Glu Asp Pro Glu Val Gln Phe Asn
   225 230 235 240
   Trp Tyr Val Asp Gly Val Glu Val His Asn Ala Lys Thr Lys Pro Arg
   245 250 255
   Glu Glu Gln Phe Asn Ser Thr Tyr Arg Val Val Ser Val Leu Thr Val
   260 265 270
   Leu His Gln Asp Trp Leu Asn Gly Lys Glu Tyr Lys Cys Lys Val Ser
   275 280 285
   Asn Lys Gly Leu Pro Ser Ser Ile Glu Lys Thr Ile Ser Lys Ala Lys
   290 295 300
   Gly Gln Pro Arg Glu Pro Gln Val Tyr Thr Leu Pro Pro Ser Gln Glu
   305 310 315 320
   Glu Met Thr Lys Asn Gln Val Ser Leu Thr Cys Leu Val Lys Gly Phe
   325 330 335
   Tyr Pro Ser Asp Ile Ala Val Glu Trp Glu Ser Asn Gly Gln Pro Glu
   340 345 350
   Asn Asn Tyr Lys Thr Thr Pro Pro Val Leu Asp Ser Asp Gly Ser Phe
   355 360 365
   Phe Leu Tyr Ser Arg Leu Thr Val Asp Lys Ser Arg Trp Gln Glu Gly
   370 375 380
   Asn Val Phe Ser Cys Ser Val Met His Glu Ala Leu His Asn His Tyr
   385                 390                 395                 400
   Thr Gln Lys Ser Leu Ser Leu Ser Pro Gly Lys
                   405                 410